(19) Europäisches Patentamt
European Patent Office
Office européen des brevets

(11) Veröffentlichungsnummer: **0 525 629 A2**

(12) # EUROPÄISCHE PATENTANMELDUNG

(21) Anmeldenummer: **92112568.8**

(22) Anmeldetag: **22.07.92**

(51) Int. Cl.[5]: **C07D 403/04**, C07D 257/04, C07D 233/54, C07D 249/08, C07D 277/30, C07D 263/32, C07D 271/06, C07D 285/12, C07D 233/24, C07D 307/54, C07D 401/14, //A61K31/395, A61K31/38,A61K31/335

(30) Priorität: **27.07.91 DE 4124942**

(43) Veröffentlichungstag der Anmeldung:
**03.02.93 Patentblatt 93/05**

(84) Benannte Vertragsstaaten:
**AT BE CH DE DK ES FR GB GR IT LI LU NL PT SE**

(71) Anmelder: **Dr. Karl Thomae GmbH**
**Postfach 1755**
**W-7950 Biberach 1(DE)**

(72) Erfinder: **Himmelsbach, Frank, Dr. Dipl.-Chem.**
**Ahornweg 16**
**W-7951 Mitelbiberach(DE)**
Erfinder: **Linz, Günter, Dr. Dipl.-Chem.**
**Drosselweg 14**
**W-7951 Mittelbiberach(DE)**
Erfinder: **Austel, Volkhard, Dr. Dipl.-Chem.**
**Kapellenweg 5**
**W-7950 Biberach 1(DE)**
Erfinder: **Pieper, Helmut, Dr.Dipl.-Chem.**
**Kapellenweg 5**
**W-7950 Biberach 1(DE)**
Erfinder: **Müller, Thomas, Dr. Dipl.-Chem.**
**Gymnasiumstrasse 16**
**W-7950 Biberach 1(DE)**
Erfinder: **Weisenberger, Johannes, Dr. Dipl.-Chem.**
**Haydnweg 5**
**W-7950 Biberach 1(DE)**
Erfinder: **Seewaldt-Becker, Elke, Dr. Dipl.-Biologin**
**Hühnerfeldstrasse 26**
**W-7950 Biberach 1(DE)**

(54) **5-gliedrige Heterocyclen, Verfahren zu ihrer Herstellung und diese Verbindungen enthaltende Arzneimittel.**

(57) Die Erfindung betrifft 5-gliedrige Heterocyclen der allgemeinen Formel

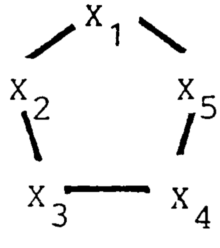

,(I)

in der
$X_1$ bis $X_5$ wie im Anspruch 1 definiert sind, deren Tautomere, deren Stereoisomere einschließlich deren

EP 0 525 629 A2

Gemische und deren Salze, insbesondere deren physiologisch verträgliche Salze mit anorganischen oder organischen Säuren oder Basen, welche wertvolle pharmakologische Eigenschaften aufweisen, vorzugsweise aggregationshemmende Wirkungen, die Verbindungen enthaltende Arzneimittel und Verfahren zu ihrer Herstellung.

Die Erfindung betrifft 5-gliedrige Heterocyclen der allgemeinen Formel

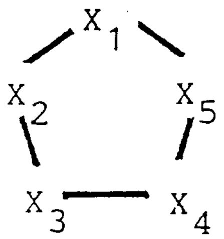

,(I)

deren Tautomere, deren Stereoisomere einschließlich deren Gemische und deren Salze, insbesondere deren physiologisch verträgliche Salze mit anorganischen oder organischen Säuren oder Basen, welche u.a. wertvolle pharmakologische Eigenschaften aufweisen, vorzugsweise aggregationshemmende Wirkungen, diese Verbindungen enthaltende Arzneimittel und Verfahren zu ihrer Herstellung.

In der obigen allgemeinen Formel I bedeutet mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält,
einer der Reste $X_1$ bis $X_5$ eine Gruppe der Formeln

A - B - C - N< ,
A - B - C - CH< oder

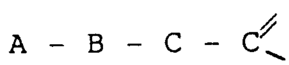

,

in denen

A eine Cyanogruppe, eine geradkettige oder verzweigte Cyanoalkylgruppe mit insgesamt 2 bis 4 Kohlenstoffatomen, eine nicht direkt an einen Phenylring der Gruppen B oder C gebundene Aminogruppe, eine geradkettige oder verzweigte Aminoalkylgruppe mit 1 bis 4 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 bis 6 Kohlenstoffatomen, durch eine Aralkoxycarbonyl-, Arylcarbonyl-, Aryloxycarbonyl-, Alkanoyloxymethoxycarbonyl-, Cycloalkanoyloxymethoxycarbonyl-, Aralkanoyloxymethoxycarbonyl-, Aroyloxymethoxycarbonyl-, Phosphono-, Dialkylphosphoryl- oder O-Alkyl-phosphonogruppe ersetzt sein kann, in denen jeweils die Alkanoylteile insgesamt 2 bis 7 Kohlenstoffatome und die Cycloalkanoylteile insgesamt 4 bis 8 Kohlenstoffatome enthalten sowie der Methoxyteil jeweils durch eine Cycloalkylgruppe mit 3 bis 6 Kohlenstoffatomen, durch eine Aralkyl-, Aryl- oder Alkylgruppe oder durch zwei Alkylgruppen, die zusammen mit dem Methylenkohlenstoffatom auch einen 5- oder 6-gliedrigen Ring bilden können, substituiert sein kann, oder, falls B oder B und C zusammen ein cyclisches Imin mit 4 bis 7 Ringgliedern darstellen, auch ein an den Iminstickstoff gebundenes Wasserstoffatom oder ein an den Iminstickstoff gebundener Alkylrest,
B eine Bindung,
eine Alkylen- oder Alkenylengruppe,
eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können und $R_1$ jeweils ein Wasserstoffatom, eine Alkylgruppe mit 1 bis 5 Kohlenstoffatomen, eine Aralkyl-, Aryl- oder Heteroarylgruppe bedeutet,
eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine -CO-$NR_1$-Gruppe ersetzt sein können und

dann eines der Stickstoffatome statt an den Rest $R_1$ auch an den Rest C gebunden sein kann,
eine gegebenenfalls durch eine Alkyl-, Aralkyl- oder Arylgruppe substituierte Cyclopropylengruppe, eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,
eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, und
C eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$N H-Gruppen mono- oder disubstituiert sein kann, wobei die Substituenten gleich oder verschieden sein können,
eine Indanylen- oder 1,2,3,4-Tetrahydronaphthylengruppe, in denen jeweils der gesättigte Ring an den Rest A oder B und der aromatische Ring an das im Ring befindliche Atom des jeweiligen Restes $X_1$ bis $X_5$ gebunden ist,
eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine -CO-$NR_1$-Gruppe ersetzt sein können und dann eines der Stickstoffatome statt an den Rest $R_1$ auch an den Rest B oder an das im Ring befindliche Atom des jeweiligen Restes $X_1$ bis $X_5$ gebunden sein kann, wobei jedoch C keine Pyrimidinylengruppe darstellen kann, wenn das heterocyclische Ringsystem ein Dithiolanring und gleichzeitig der Rest A eine Aminogruppe darstellt,
eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann, oder
eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarten Methylengruppen durch eine Carbonylgruppe ersetzt sein können, darstellen,
ein zweiter der Reste $X_1$ bis $X_5$ eine Gruppe der Formeln

F - E - D - N< ,
F - E - D - CH< oder

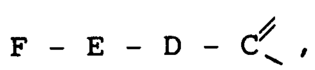

in denen

D eine gegebenenfalls durch eine Hydroxy-, Alkoxy-, Alkylsulfenyl-, $(R_1)_2N$-, (Alkylcarbonyl)$NR_1$-, (Aralkylcarbonyl)$NR_1$-, (Arylcarbonyl)$NR_1$-, (Heteroarylcarbonyl)$NR_1$-, (Alkoxycarbonyl)-$NR_1$-, (Aralkoxycarbonyl)$NR_1$-, (Aryloxycarbonyl)$NR_1$-, ($(R_1)_2NCO$)$NR_1$-, (Alkylsulfonyl)$NR_1$-, (Aralkylsulfonyl)$NR_1$-, (Arylsulfonyl)$NR_1$- oder $R_1OCO$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann,
eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$-, $(R_1)_2NSO_2$- oder $R_1OCO$-alkoxy-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,
eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine -CO-$NR_1$-Gruppe ersetzt sein können und dann eines der Stickstoffatome statt an den Rest $R_1$ auch an den Rest E, sofern dieser keine Bindung

darstellt und nicht über ein Sauerstoff- oder Schwefelatom an den Rest D gebunden ist, oder an das im Ring befindliche Atom des jeweiligen Restes $X_1$ bis $X_5$ gebunden sein kann,
eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,
eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, oder
eine über den Rest $W_1$ mit dem im Ring befindlichen Atom des jeweiligen Restes $X_1$ bis $X_5$ verknüpfte Alkylengruppe mit 1 bis 6 Kohlenstoffatomen, in der $W_1$ eine $NR_1$-Gruppe, ein Sauerstoff- oder Schwefelatom darstellt,
E eine Bindung,
eine geradkettige oder verzweigte Alkylengruppe mit 1 bis 5 Kohlenstoffatomen oder eine Alkenylengruppe mit 2 bis 5 Kohlenstoffatomen, die jeweils durch eine $R_1$OCO-alkyl-Gruppe substituiert sein kann, oder
eine über den Rest $W_2$ mit dem Rest D verknüpfte Alkylengruppe, in der $W_2$ ein Sauerstoff- oder Schwefelatom, eine Sulfinyl-, Sulfonyl-, -$NR_1$-, (Alkylcarbonyl)N-, -(Aralkylcarbonyl)N-, -(Arylcarbonyl)N-, (Heteroarylcarbonyl)N-, -(Alkylsulfonyl)N-, -(Arylsulfonyl)N-, -CON$R_1$- oder -$NR_1$CO-Gruppe darstellt,
F eine nicht an ein Heteroatom der Reste D oder E gebundene Carbonylgruppe, die durch eine Hydroxygruppe, durch eine Aminogruppe, durch eine Alkoxygruppe mit 1 bis 6 Kohlenstoffatomen, in der ein Alkoxyrest mit 1 bis 3 Kohlenstoffatomen in 1-, 2- oder 3-Stellung durch eine Cycloalkylgruppe mit 4 bis 8 Kohlenstoffatomen, durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, durch eine Cycloalkoxygruppe mit 4 bis 8 Kohlenstoffatomen, durch eine Alkanoyloxymethoxygruppe mit insgesamt 2 bis 7 Kohlenstoffatomen im Alkanoylteil, durch eine Cycloalkanoyloxymethoxygruppe mit insgesamt 4 bis 8 Kohlenstoffatomen im Cycloalkanoylteil, durch eine Alkoxycarbonyloxymethoxygruppe mit 1 bis 6 Kohlenstoffatomen im Alkylteil, durch eine Cycloalkoxycarbonyloxymethoxygruppe mit 3 bis 7 Kohlenstoffatomen im Cycloalkylteil, durch eine Aroyloxymethoxy-, Aralkanoyloxymethoxy-, Aryloxycarbonyloxymethoxy- oder Aralkoxycarbonyloxymethoxygruppe, in denen der Methoxyteil jeweils durch eine Alkylgruppe mit 1 bis 6 Kohlenstoffatomen, durch eine Cycloalkylgruppe mit 3 bis 7 Kohlenstoffatomen, durch eine Aralkyl- oder Arylgruppe substituiert sein kann, substituiert ist, eine Sulfo-, Phosphono-, O-Alkylphosphono- oder Tetrazol-5-ylgruppe darstellen, wobei, falls A eine Aminogruppe oder eine Cyanogruppe darstellt, der kürzeste Abstand zwischen dieser Gruppe und dem Rest F mindestens 10 Bindungen beträgt und generell A keine Cyanogruppe darstellen kann, wenn das heterocyclische Ringsystem ein Pyrazolinring und gleichzeitig die Reste C und D unsubstituierte Phenylengruppen und gleichzeitig die Reste B und E eine Bindung darstellen oder, wenn das heterocyclische Ringsystem ein Oxazol- oder Oxazolinring und gleichzeitig der Rest C eine unsubstituierte Phenylengruppe und B eine Bindung darstellen,
ein dritter der Reste $X_1$ bis $X_5$ ein Schwefelatom, eine Sulfinyl-, Sulfonyl-, $R_1$N<,

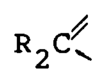

oder $(R_2)_2$C< Gruppe oder ein N-Atom, wobei $R_1$ jeweils wie eingangs definiert ist und
$R_2$ ein Wasserstoff-, Chlor- oder Bromatom, eine Alkylgruppe mit 1 bis 7 Kohlenstoffatomen, eine Arylalkyl-, Aryl-, Heteroaryl-, Alkoxy-, $(R_1)_2$N-, $R_1$OOC- oder $(R_1)_2$NCO-Gruppe bedeutet,
ein vierter der Reste $X_1$ bis $X_5$ ein Sauerstoff-, Schwefel- oder Stickstoffatom, eine Sulfonyl- oder

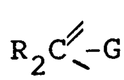 -Gruppe

oder auch eine Carbonylgruppe, wenn diese nicht zwischen zwei Stickstoffatomen steht,
ein fünfter der Reste $X_1$ bis $X_5$ ein Stickstoffatom, eine

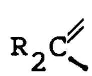

oder $(R_2)_2C<$ Gruppe oder auch zwei benachbarte Reste der Reste $X_1$ bis $X_5$ zusammen eine o-Phenylengruppe bedeuten,
wobei, soweit nichts anderes erwähnt wurde,
die vorstehend erwähnten Alkyl-, Alkylen-, Alkenylen- oder Alkoxyteile jeweils 1 bis 3 Kohlenstoffatome enthalten können, sowie
unter den vorstehend erwähnten Begriffen "eine Arylgruppe" oder "eine Aroylgruppe" eine Phenyl-, Naphthyl- oder Benzoylgruppe, die durch eine Trifluormethyl-, Carboxy-, $(R_1)_2NCO$-,Alkoxycarbonyl-, Alkylcarbonyl-, Alkylsulfenyl-, Alkylsulfinyl-, Alkylsulfonyl-, Nitro-, $(R_1)_2N$-, Alkylcarbonyl-$NR_1$-, Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono-, di- oder trisubstituiert sein können, und
unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist.

Unter die vorstehend erwähnte allgemeine Formel I fallen somit beispielsweise die entsprechend substituierten Furan-, Tetrahydrofuran-, 2,3-Dihydro-furan-, 2,5-Dihydro-furan-, Thiophen-, 2,3-Dihydro-thiophen-, 2,5-Dihydro-thiophen-, Tetrahydrothiophen-, S-Oxido-tetrahydrothiophen-, S,S-Dioxidotetrahydrothiophen-, 1,2-Dithiolan-, 1,3-Dithiolan-, 1,3-Dithiolan-S,S,S',S'-tetraoxid-, Pyrrol-, Indol-, Isoindol-, 2,3-Dihydro-indol-, 2,3-Dihydro-isoindol-, 2-Indolon-, Imidazol-, 4,5-Dihydro-imidazol-, Tetrahydroimidazol-, Benz-imidazolin-, Pyrazol-, 2H-Pyrazol-5-on-, 4,5-Dihydro-pyrazol-, 1,5-Dihydro-pyrazol-, Indazol-, 2,3-Dihydro-indazol-, Oxazol-, Isoxazol-, Oxazolin-, Oxazolidin-, Thiazol-, Isothiazol-, Thiazolin-, Thiazolidin-, 1,3,4-Oxadiazol-, 1,3,4-Thiadiazol-, 1,2,3-Triazol-, 1,2,4-Triazol- und Tetrazolderivate.

Bevorzugte Verbindungen der obigen allgemeinen Formel I sind diejenigen, in denen mit der Maßgabe, daß der 5-gliedrige heterocyclische Ring kein Pyrrolidin-, Pyrrolin-, Pyrrolinon- oder Pyrrolidinonring darstellt sowie mindestens ein Kohlenstoffatom enthält,
einer der Reste $X_1$ bis $X_5$ eine Gruppe der Formeln

A - B - C - N< ,
A - B - C - CH< oder

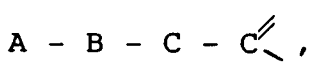

in denen

A eine Cyanogruppe, eine geradkettige oder verzweigte Cyanoalkylgruppe mit insgesamt 2 bis 4 Kohlenstoffatomen, eine nicht direkt an einen Phenylring der Gruppen B oder C gebundene Aminogruppe, eine geradkettige oder verzweigte Aminoalkylgruppe mit 1 bis 4 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 oder 5 Kohlenstoffatomen, durch eine Aralkoxycarbonyl-, Aryloxycarbonyloxy- oder Arylcarbonylgruppe oder durch eine Alkanoyloxymethoxycarbonylgruppe, in der der Alkanoylteil insgesamt 2 bis 7 Kohlenstoffatome enthalten und der Methoxyteil durch eine Alkylgruppe substituiert sein kann, oder durch eine Phosphono-, Dialkylphosphoryl- oder O-Alkyl-phosphonogruppe ersetzt sein kann, oder, falls B oder B und C zusammen ein cyclisches Imin mit 6 Ringgliedern darstellen, auch ein an den Iminstickstoff gebundenes Wasserstoffatom oder ein an den Iminstickstoff gebundener Alkylrest,
B eine Bindung,
eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-,

Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können und $R_1$ jeweils ein Wasserstoffatom, eine Alkylgruppe mit 1 bis 5 Kohlenstoffatomen, eine Aralkyl- oder Arylgruppe bedeutet,
eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH = N-Gruppen jeweils durch eine -CO-N$R_1$-Gruppe ersetzt sein können und dann eines der Stickstoffatome statt an den Rest $R_1$ auch an den Rest C gebunden sein kann,
eine Cycloalkylengruppe mit 3 bis 5 Kohlenstoffatomen, eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppe durch eine Carbonylgruppe ersetzt sein können, und
C eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2$N-, $(R_1)_2$NCO- oder $(R_1)_2$N$SO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$N H-Gruppen mono- oder disubstituiert sein kann, wobei die Substituenten gleich oder verschieden sein können,
eine Indanylen- oder 1,2,3,4-Tetrahydronaphthylengruppe, in denen jeweils der gesättigte Ring an den Rest A und der aromatische Ring an das im Ring befindliche Atom des jeweiligen Restes $X_1$ bis $X_5$ gebunden ist,
eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH = N-Gruppen jeweils durch eine -CO-N$R_1$-Gruppe ersetzt sein können und dann eines der Stickstoffatome statt an den Rest $R_1$ auch an den Rest B oder an das im Ring befindliche Atom des jeweiligen Restes $X_1$ bis $X_5$ gebunden sein kann, sofern dieses ein Kohlenstoffatom darstellt,
eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, darstellen,
ein zweiter der Reste $X_1$ bis $X_5$ eine Gruppe der Formeln

F - E - D - N< ,
F - E - D - CH< oder

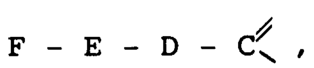

in denen

D eine gegebenenfalls durch eine Hydroxy-, Alkoxy-, Alkylsulfenyl-, $(R_1)_2$N-, (Alkylcarbonyl)N$R_1$-, (Aralkylcarbonyl)N$R_1$-, (Arylcarbonyl)N$R_1$-, (Heteroarylcarbonyl)N$R_1$-, (Alkoxycarbonyl)-N$R_1$-, (Aralkoxycarbonyl)N$R_1$-, ($(R_1)_2$NCO)N$R_1$-, (Alkylsulfonyl)-N$R_1$-, (Aralkylsulfonyl)N$R_1$-, (Arylsulfonyl)N$R_1$- oder $R_1$OCO-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann,
eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2$N-, $(R_1)_2$NCO-, $(R_1)_2$N$SO_2$- oder $R_1$OCO-alkoxy-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,
eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH = N-Gruppen jeweils durch eine -CO-N$R_1$-Gruppe ersetzt sein können und dann eines der Stickstoffatome statt an den Rest $R_1$ auch an den Rest E, sofern dieser keine Bindung darstellt und nicht über ein Heteroatom an den Rest D gebunden ist, oder an das im Ring befindliche Atom des jeweiligen Restes $X_1$ bis $X_5$, sofern dieses ein Kohlenstoffatom darstellt, gebunden sein kann,
eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppen

durch eine Carbonylgruppe ersetzt sein können, oder
eine über den Rest $W_1$ mit dem im Ring befindlichen Atom des jeweiligen Restes $X_1$ bis $X_5$, sofern dieser ein Kohlenstoffatom darstellt, verknüpfte Alkylengruppe mit 1 bis 3 Kohlenstoffatomen, in der $W_1$ eine $NR_1$-Gruppe, ein Sauerstoff- oder Schwefelatom darstellt,
E eine Bindung,
eine geradkettige oder verzweigte Alkylengruppe mit 1 bis 5 Kohlenstoffatomen oder eine Alkenylengruppe mit 2 bis 5 Kohlenstoffatomen, die jeweils durch eine oder zwei $R_1$OCO-alkylgruppen substituiert sein können, oder
eine über den Rest $W_2$ mit dem Rest D verknüpfte Alkylengruppe, in der $W_2$ ein Sauerstoff- oder Schwefelatom, eine Sulfinyl-, Sulfonyl-, $-NR_1-$, (Alkylcarbonyl)N-, -(Aralkylcarbonyl)N-, -(Arylcarbonyl)N-, -(Heteroarylcarbonyl)N-, -(Alkylsulfonyl)N-, -(Arylsulfonyl)N-, $-CONR_1-$ oder $-NR_1CO$-Gruppe darstellt und nicht an ein Heteroatom des Restes D gebunden ist,
F eine nicht an ein Heteroatom der Reste D oder E gebundene Carbonylgruppe, die durch eine Hydroxygruppe, durch eine Aminogruppe, durch eine Alkoxygruppe mit 1 bis 5 Kohlenstoffatomen substituiert ist, in der ein Alkoxyrest mit 1 bis 3 Kohlenstoffatomen in 1-, 2- oder 3-Stellung durch eine Cycloalkylgruppe mit 5 bis 7 Kohlenstoffatomen, durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, durch eine Cycloalkoxygruppe mit 4 bis 8 Kohlenstoffatomen, durch eine Alkanoyloxymethoxygruppe mit insgesamt 2 bis 7 Kohlenstoffatomen im Alkanoylteil, durch eine Aroyloxymethoxygruppe, durch eine Alkoxycarbonyloxymethoxygruppe mit 1 bis 4 Kohlenstoffatomen im Alkylteil oder durch eine Cycloalkoxycarbonyloxymethoxygruppe mit 5 bis 6 Kohlenstoffatomen im Cycloalkylteil, in denen der Methoxyteil jeweils durch eine Alkylgruppe substituiert sein kann, substituiert ist, eine Sulfo-, Phosphono-, O-Alkylphosphono- oder Tetrazol-5-ylgruppe darstellen, wobei, falls A eine Aminogruppe oder eine Cyanogruppe darstellt, der kürzeste Abstand zwischen dieser Gruppe und dem Rest F mindestens 10 Bindungen beträgt und generell A keine Cyanogruppe darstellen kann, wenn das heterocyclische Ringsystem ein Pyrazolinring und gleichzeitig die Reste C und D unsubstituierte Phenylengruppen und gleichzeitig die Reste B und E eine Bindung darstellen oder, wenn das heterocyclische Ringsystem ein Oxazol- oder Oxazolinring und gleichzeitig der Rest C eine unsubstituierte Phenylengruppe und B eine Bindung darstellen,
ein dritter der Reste $X_1$ bis $X_5$ eine $R_1N$<,

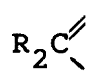

oder $(R_2)_2C$< Gruppe oder ein N-Atom, wobei $R_1$ jeweils wie eingangs definiert ist und
$R_2$ ein Wasserstoff-, Chlor- oder Bromatom, eine Alkylgruppe mit 1 bis 7 Kohlenstoffatomen, eine Arylalkyl-, Aryl-, Heteroaryl-, Alkoxy-, $(R_1)_2N$-, $R_1OOC$- oder $(R_1)_2NCO$-Gruppe bedeutet,
ein vierter der Reste $X_1$ bis $X_5$ ein Sauerstoff-, Schwefel- oder Stickstoffatom oder eine

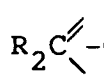 -Gruppe

oder auch eine Carbonylgruppe, wenn diese nicht zwischen zwei Stickstoffatomen steht,
ein fünfter der Reste $X_1$ bis $X_5$ ein Stickstoffatom, eine

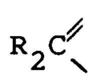

oder $(R_2)_2C$< Gruppe oder auch zwei benachbarte Reste der Reste $X_1$ bis $X_5$ zusammen eine o-Phenylengruppe bedeuten, darstellen,
deren Tautomere, deren Stereoisomere einschließlich deren Gemische und deren Salze, insbesondere deren physiologisch verträgliche Salze mit anorganischen oder organischen Säuren oder Basen,
wobei, soweit nichts anderes erwähnt wurde,
die vorstehend erwähnten Alkyl-, Alkylen-, Alkenylen- oder Alkoxyteile jeweils 1 bis 3 Kohlenstoffatome enthalten können, sowie
unter den vorstehend erwähnten Begriffen "eine Arylgruppe" oder "eine Aroylgruppe" eine Phenyl-, Naphthyl- oder Benzoylgruppe, die durch eine Trifluormethyl-, Carboxy-, $(R_1)_2NCO$-,Alkoxycarbonyl-,

Alkylcarbonyl-, Alkylsulfenyl-, Alkylsulfinyl-, Alkylsulfonyl-, Nitro-, $(R_1)_2N$-, Alkylcarbonyl-$NR_1$-, Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono- oder disubstituiert sein können, und

unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist.

Besonders bevorzugte Verbindungen der obigen allgemeinen Formel I sind diejenigen, in denen mit der Maßgabe, daß der 5-gliedrige heterocyclische Ring kein Pyrrolidin-, Pyrrolin-, Pyrrolinon- oder Pyrrolidinonring darstellt sowie mindestens ein Kohlenstoffatom enthält,

einer der Reste $X_1$ bis $X_5$ eine Gruppe der Formeln

A - B - C - N< ,
A - B - C - CH< oder

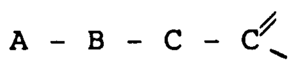 ,

in denen

A eine nicht direkt an einen Phenylring der Gruppen B oder C gebundene Aminogruppe, eine geradkettige oder verzweigte Aminoalkylgruppe mit 1 bis 3 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Allyloxycarbonylgruppe, durch eine Benzyloxycarbonylgruppe oder durch eine Alkanoyloxymethoxycarbonylgruppe, in der der Alkanoylteil insgesamt 2 bis 4 Kohlenstoffatome enthalten und der Methoxyteil durch eine Methylgruppe substituiert sein kann, oder durch eine Phosphono-, Dimethylphosphoryl- oder Diethylphosphorylgruppe ersetzt sein kann, oder, falls B oder B und C zusammen ein cyclisches Imin mit 6 Ringgliedern darstellt, auch ein an den Iminstickstoff gebundenes Wasserstoffatom oder eine an den Iminstickstoff gebundene Methylgruppe,

B eine Bindung,

eine Phenylengruppe, die durch ein Fluor-, Chlor- oder Bromatom, durch eine Methyl- oder Methoxygruppe substituiert sein kann,

eine Pyridinylen-, Pyrimidinylen-, Pyrazinylen- oder Pyridazinylengruppe,

eine Cycloalkylengruppe mit 3 bis 5 Kohlenstoffatomen,

eine Piperidinylen- oder 2-Oxo-piperidinylengruppe und

C eine Phenylengruppe, die durch ein Fluor-, Chlor- oder Bromatom, durch eine Methyl-, Methoxy-, Methylsulfenyl-, Methylsulfinyl- oder Methylsulfonylgruppe substituiert sein kann,

eine Indanylen- oder 1,2,3,4-Tetrahydronaphthylengruppe, in denen jeweils der gesättigte Ring an den Rest A und der aromatische Ring an das im Ring befindliche Atom des jeweiligen Restes $X_1$ bis $X_5$ gebunden ist,

eine gegebenenfalls durch eine Methylgruppe substituierte Pyridinylen-, Pyrimidinylen-, Pyrazinylen- oder Pyridazinylengruppe oder

eine Cyclohexylengruppe, in der eine oder zwei CH-Einheiten je durch ein Stickstoffatom ersetzt sein können, darstellen,

ein zweiter der Reste $X_1$ bis $X_5$ eine Gruppe der Formeln

F - E - D - N< ,
F - E - D - CH< oder

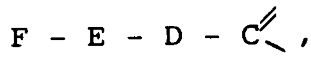

in denen

D eine gegebenenfalls durch eine Hydroxy-, Methoxy-, Amino-, Dimethylamino-, Dibenzylamino- oder Carboxygruppe, durch eine Alkoxycarbonylgruppe mit insgesamt 2 bis 4 Kohlenstoffatomen oder durch eine Alkoxycarbonylaminogruppe mit insgesamt 2 bis 5 Kohlenstoffatomen substituierte Alkylen- oder Alkenylengruppe, wobei die Alkylengruppe 1 bis 3 Kohlenstoffatomen und die Alkenylengruppe 2 oder 3 Kohlenstoffatome enthalten kann,
eine Phenylengruppe, die durch ein Fluor-, Chlor- oder Bromatom, durch eine Methyl-, Hydroxy-, Methoxy-, Methylsulfenyl-, Methylsulfinyl-, Methylsulfonyl-, Nitro-, Amino-, Acetamino-, Benzoylamino-, Methansulfonylamino-, Carboxymethoxy- oder Methoxycarbonylmethoxygruppe substituiert sein kann,
eine Pyridinylen-, Pyrimidinylen-, Pyrazinylen- oder Pyridazinylengruppe, in denen eine -CH=N-Gruppe durch eine -CO-NH-Gruppe ersetzt sein kann, wobei der Stickstoff statt an das Wasserstoffatom auch an den Rest E, sofern dieser keine Bindung darstellt und nicht über ein Heteroatom an den Rest D gebunden ist, oder an das im Ring befindliche Atom des jeweiligen Restes $X_1$ bis $X_5$, sofern dieser ein Kohlenstoffatom darstellt, gebunden sein kann,
eine Cyclohexylengruppe, in der eine CH-Einheit durch ein Stickstoffatom ersetzt sein kann, oder
eine über den Rest $W_1$ mit dem im Ring befindlichen Atom des jeweiligen Restes $X_1$ bis $X_5$, sofern dieses ein Kohlenstoffatom darstellt, verknüpfte Alkylengruppe mit 1 oder 2 Kohlenstoffatomen, in der $W_1$ eine Iminogruppe- oder ein Schwefelatom darstellt,
E eine Bindung,
eine geradkettige oder verzweigte Alkylengruppe mit 1 bis 4 Kohlenstoffatomen oder eine Alkenylengruppe mit 2 bis 4 Kohlenstoffatomen, die jeweils durch eine Carboxymethyl- oder Methoxycarbonylmethylgruppe substituiert sein können, oder
eine über den Rest $W_2$ mit dem Rest D verknüpfte Alkylengruppe mit 1 oder 2 Kohlenstoffatomen, in der $W_2$ ein Sauerstoff- oder Schwefelatom, eine Sulfinyl-, Sulfonyl-, Imino-, Methylimino- oder Acetyliminogruppe oder eine über die Carbonylgruppe an den Rest D gebundene Aminocarbonylgruppe darstellt, wobei E nicht an ein Heteroatom des Restes D gebunden sein kann, und
F eine nicht an ein Heteroatom des Restes D gebundene Carbonylgruppe, die durch eine Hydroxygruppe, durch eine Alkoxygruppe mit 1 bis 4 Kohlenstoffatomen, die in 1-, 2- oder 3-Stellung durch eine Cyclohexyl- oder Phenylgruppe substituiert sein kann, durch eine Cycloalkoxygruppe mit 5 bis 8 Kohlenstoffatomen, durch eine Alkanoyloxymethoxygruppe mit insgesamt 2 bis 6 Kohlenstoffatomen im Alkanoylteil, durch eine Benzoyloxymethoxygruppe, durch eine Alkoxycarbonyloxymethoxygruppe mit 1 bis 3 Kohlenstoffatomen im Alkoxyteil oder durch eine Cyclohexyloxycarbonyloxymethoxygruppe, in denen der Methoxyteil jeweils durch eine Methylgruppe substituiert sein kann, substituiert ist, eine Sulfo-, Phosphono-, O-Methyl-phosphono, O-Ethyl-phosphono- oder Tetrazol-5-yl-gruppe darstellen, wobei, falls A eine Aminogruppe darstellt, der kürzeste Abstand zwischen dieser Gruppe und dem Rest F mindestens 10 Bindungen beträgt,
ein dritter der Reste $X_1$ bis $X_5$ ein N-Atom, eine Imino-, Methylimino-, Ethylimino-, Phenylimino-, Benzylimino- oder 2-Phenylethylimino,

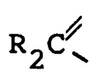

oder $(R_2)_2C<$ Gruppe, wobei $R_2$ ein Wasserstoffatom, eine Alkylgruppe mit 1 bis 7 Kohlenstoffatomen, eine Benzyl-, Phenylethyl-, Phenyl-, Pyridyl-, Carboxy-, Aminocarbonyl- oder Alkoxycarbonylgruppe mit insgesamt 2 bis 4 Kohlenstoffatomen bedeutet,
ein vierter der Reste $X_1$ bis $X_5$ ein Sauerstoff-, Schwefel- oder Stickstoffatom, eine

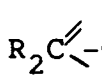-Gruppe

oder auch eine Carbonylgruppe, wenn diese nicht zwischen zwei Stickstoffatomen steht,
ein fünfter der Reste $X_1$ bis $X_5$ ein Stickstoffatom, eine

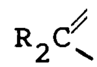

oder $(R_2)_2C<$ Gruppe, wobei $R_2$ wie vorstehend erwähnt definiert ist, oder auch 2 benachbarte Reste der Reste $X_1$ bis $X_5$ zusammen eine o-Phenylengruppe darstellen, bedeuten,
insbesondere jedoch diejenigen Verbindungen der allgemeinen Formel I, in denen mit der Maßgabe, daß der 5-gliedrige heterocyclische Ring kein Pyrrolidin-, Pyrrolin-, Pyrrolinon- oder Pyrrolidinonring darstellt sowie mindestens ein Kohlenstoffatom enthält,
einer der Reste $X_1$ bis $X_5$ eine Gruppe der Formeln

A - B - C - N< oder

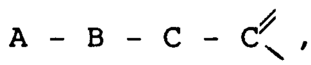

in denen

A eine gegebenenfalls an einem der Stickstoffatome durch eine Alkoxycarbonylgruppe mit insgesamt 2 oder 3 Kohlenstoffatomen substituierte Amidinogruppe,
B eine Bindung oder eine Phenylengruppe und
C eine Phenylen- oder Pyridazinylengruppe,
ein zweiter der Reste $X_1$ bis $X_5$ eine Gruppe der Formeln

F - E - D - N< oder

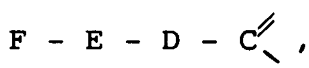

in denen

D eine gegebenenfalls durch eine Hydroxy-, Amino-, Dibenzylamino- oder tert.Butoxycarbonylaminogruppe substituierte Ethylengruppe, eine Phenylengruppe oder eine Methylenthiogruppe, in der das Schwefelatom an ein Kohlenstoffatom des Ringes gebunden ist,
E eine Bindung oder eine Ethylengruppe und
F eine Carbonylgruppe, die durch eine Hydroxy- oder Alkoxygruppe mit 1 oder 2 Kohlenstoffatomen substituiert ist, darstellen,
ein dritter der Reste $X_1$ bis $X_5$ ein N-Atom, eine Imino-, Methylimino- oder Methingruppe,
ein vierter der Reste $X_1$ bis $X_5$ ein Sauerstoff-, Schwefel- oder Stickstoffatom,
ein fünfter der Reste $X_1$ bis $X_5$ ein Stickstoffatom, eine Methin-, Carboxymethin-, Methoxycarbonylmethin- oder Ethoxycarbonylmethingruppe oder auch zwei benachbarte Reste der Reste $X_1$ bis $X_5$ zusammen eine o-Phenylengruppe darstellen, bedeuten,
deren Tautomere, deren Stereoisomere einschließlich deren Gemische und deren Salze, insbesondere deren physiologisch verträgliche Salze mit anorganischen oder organischen Säuren oder Basen.

Die neuen Verbindungen lassen sich beispielsweise nach folgenden Verfahren herstellen:

a) Zur Herstellung von Verbindungen der allgemeinen Formel I, in der F eine Carboxylgruppe darstellt:
Umwandlung einer Verbindung der allgemeinen Formel

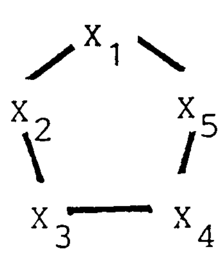

,(II)

in der

$X_1$ bis $X_5$ mit der Maßgabe wie eingangs definiert sind, daß einer der Reste $X_1$ bis $X_5$ eine Gruppe der Formel

F' - E - D - N< ,

F' - E - D - CH< oder

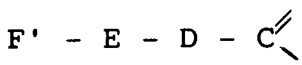 darstellt,

in denen

E und D wie eingangs definiert sind und

F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt.

Beispielsweise können funktionelle Derivate der Carboxylgruppe wie deren unsubstituierte oder substituierte Amide, Ester, Thioester, Trimethylsilylester, Orthoester, Iminoester, Amidine oder Anhydride, oder die Nitrilgruppe mittels Hydrolyse in eine Carboxylgruppe,

Ester mit tertiären Alkoholen, z.B. der tert. Butylester, mittels Behandlung mit einer Säure oder Thermolyse in eine Carboxylgruppe und

Ester mit Aralkanolen, z.B. der Benzylester, mittels Hydrogenolyse in eine Carboxylgruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure, in Gegenwart einer Base wie Lithiumhydroxid, Natriumhydroxid oder Kaliumhydroxid in einem geeigneten Lösungsmittel wie Wasser, Wasser/Methanol, Wasser/Äthanol, Wasser/Isopropanol, Methanol, Äthanol, oder Wasser/Dioxan bei Temperaturen zwischen -10 und 120 ° C, z.B. bei Temperaturen zwischen Raumtemperatur und der Siedetemperatur des Reaktionsgemisches, durchgeführt. Bei der Behandlung mit einer organischen Säure wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet F' in einer Verbindung der Formel II eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50 ° C in die Carboxylgruppe übergeführt werden.

Bedeutet F' in einer Verbindung der Formel II beispielsweise die tert. Butyloxycarbonylgruppe, so kann die tert. Butylgruppe auch durch Behandlung mit einer Säure wie Trifluoressigsäure, Ameisensäure, p-Toluolsulfonsäure, Schwefelsäure, Phosphorsäure oder Polyphosphorsäure gegebenenfalls in einem inerten Lösungsmittel wie Methylenchlorid, Chloroform, Benzol, Toluol, Tetrahydrofuran oder Dioxan vorzugsweise bei Temperaturen zwischen -10 und 120 ° C, z.B. bei Temperaturen zwischen 0 und 60 ° C, oder auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100 ° C, abgespalten werden.

Bedeutet F' in einer Verbindung der Formel II beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Ethanol, Ethanol/Wasser, Eisessig, Essigsäureäthylester, Dioxan oder Dimethylformamid vorzugsweise bei Temperaturen zwischen 0 und 50 ° C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, oder eine Benzyloxygruppe zur Hydroxygruppe mitreduziert werden.

b) Zur Herstellung von Verbindungen der allgemeinen Formel I, in der A eine gegebenenfalls durch eine Alkylgruppe substituierte $H_2N-C(=NH)$-Gruppe darstellt:

Umsetzung einer gegebenenfalls im Reaktionsgemisch gebildeten Verbindung der allgemeinen Formel

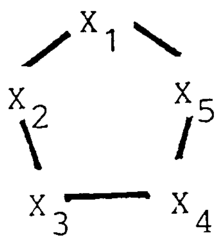 ,(III)

in der
$X_1$ bis $X_5$ mit der Maßgabe wie eingangs definiert sind, daß einer der Reste $X_1$ bis $X_5$ eine Gruppe der Formel

$Z_1$ - C(=NH) - B - C - N< ,
$Z_1$ - C(=NH) - B - C - CH< oder

$Z_1 - C(=NH) - B - C - C\lessdot$ darstellt,

in denen

B und C wie eingangs definiert sind und
$Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe oder eine Aminogruppe darstellt, mit einem Amin der allgemeinen Formel

$(R_3)_2NH$ ,(IV)

in der
die Reste $R_3$, die gleich oder verschieden sein können, Wasserstoffatome oder Alkylgruppen mit jeweils 1 bis 3 Kohlenstoffatomen darstellen, oder mit deren Säureadditionssalzen.

Die Umsetzung wird zweckmäßigerweise in einem Lösungsmittel wie Methanol, Ethanol, n-Propanol, Wasser, Methanol/Wasser, Tetrahydrofuran oder Dioxan bei Temperaturen zwischen 0 und 150°C, vorzugsweise bei Temperaturen zwischen 20 und 120°C, mit einem entsprechenden freien Amin oder mit einem entsprechenden Säureadditionssalz wie beispielsweise den entsprechenden Ammoniumcarbonaten, -acetaten oder -chloriden durchgeführt.

Eine Verbindung der allgemeinen Formel III erhält man beispielsweise durch Umsetzung eines entsprechenden Nitrils mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid oder durch Umsetzung eines entsprechenden Nitrils mit einem Alkoholat wie Natriummethylat in einem Lösungsmittel wie Dioxan oder Tetrahydrofuran, vorzugsweise jedoch in dem betreffenden Alkohol.

c) Zur Herstellung von Verbindungen der allgemeinen Formel I, in der mindestens einer der Reste B, C, D oder E eine Sulfinyl- oder Sulfonylgruppe enthält:
Oxidation einer Verbindung der allgemeinen Formel

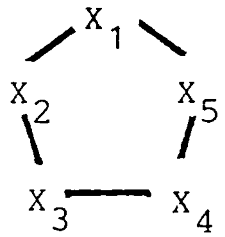

,(V)

in der

$X_1$ bis $X_5$ mit der Maßgabe wie eingangs definiert sind, daß mindestens einer der Reste $X_1$ bis $X_5$ eine Sulfenyl- oder Sulfinylgruppe enthält.

Die Oxidation wird vorzugsweise in einem Lösungsmittel oder Lösungsmittelgemisch, z.B. in Wasser, Wasser/Pyridin, Aceton, Methylenchlorid, Eisessig, Eisessig/Acetanhydrid, verdünnter Schwefelsäure oder Trifluoressigsäure, je nach dem verwendeten Oxidationsmittel zweckmäßigerweise bei Temperaturen zwischen -80 und 100 ° C durchgeführt.

Zur Herstellung einer entsprechenden S-Oxidverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20 ° C oder in Aceton bei 0 bis 60 ° C, mit einer Persäure wie Perameisensäure in Eisessig oder Trifluoressigsäure bei 0 bis 50 ° C oder mit m-Chlorperbenzoesäure in Methylenchlorid oder Chloroform bei -20 bis 60 ° C, mit Natriummetaperjodat in wäßrigem Methanol oder Äthanol bei -15 bis 25 ° C, mit Brom in Eisessig oder wäßriger Essigsäure gegebenenfalls in Gegenwart einer schwachen Base wie Natriumacetat, mit N-Brom-succinimid in Äthanol, mit tert.Butyl-hypochlorit in Methanol bei -80 bis -30 ° C, mit Jodbenzodichlorid in wäßrigem Pyridin bei 0 bis 50 ° C, mit Salpetersäure in Eisessig bei 0 bis 20 ° C, mit Chromsäure in Eisessig oder in Aceton bei 0 bis 20 ° C und mit Sulfurylchlorid in Methylenchlorid bei -70 ° C, der hierbei erhaltene Thioäther-Chlor-Komplex wird zweckmäßigerweise mit wäßrigem Äthanol hydrolysiert.

Zur Herstellung einer S,S-Dioxidverbindung der allgemeinen Formel I wird die Oxidation ausgehend von einer entsprechenden Alkylsulfinylverbindung zweckmäßigerweise mit einem oder mehr Äquivalenten des verwendeten Oxidationsmittels oder ausgehend von einer entsprechenden Alkylsulfenylverbindung zweckmäßigerweise mit zwei oder mehr Äquivalenten des verwendeten Oxidationsmittels durchgeführt, z.B. mit Wasserstoffperoxid in Eisessig/Acetanhydrid, Trifluoressigsäure oder in Ameisensäure bei 20 bis 100 ° C oder in Aceton bei 0 bis 60 ° C, mit einer Persäure wie Perameisensäure oder m-Chlorperbenzoesäure in Eisessig, Trifluoressigsäure, Methylenchlorid oder Chloroform bei Temperaturen zwischen 0 und 60 ° C, mit Salpetersäure in Eisessig bei 0 bis 20 ° C, mit Chromsäure oder Kaliumpermanganat in Eisessig, Wasser/Schwefelsäure oder in Aceton bei 0 bis 20 ° C.

d) Zur Herstellung von Verbindungen der allgemeinen Formel I, in der A eine durch eine Alkoxycarbonylgruppe mit insgesamt 2 bis 5 Kohlenstoffatomen oder durch eine Aralkoxycarbonylgruppe substituierte Amino-, Aminoalkyl-, Amidino- oder Guanidinogruppe darstellt:

Umsetzung einer Verbindung der allgemeinen Formel

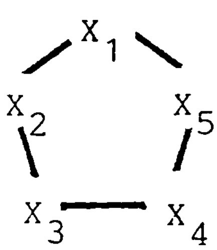

,(VI)

in der

$X_1$ bis $X_5$ mit der Maßgabe wie eingangs definiert sind, daß einer der Reste $X_1$ bis $X_5$ eine Gruppe der Formel

A' - B - C - N< ,

A' - B - C - CH< oder

$$A' - B - C - C\begin{smallmatrix}/\!\!/\\ \diagdown\end{smallmatrix}$$ darstellt,

in denen

B und C wie eingangs definiert sind und
A' eine Amino-, Aminoalkyl-, $H_2N-C(=NH)-$ oder $H_2N-C(=NH)-NH$-Gruppe darstellt, mit einer Verbindung der allgemeinen Formel

$Z_2$ - $COOR_4$ ,(VII)

in der
$R_4$ eine Alkylgruppe mit 1 bis 4 Kohlenstoffatomen oder eine Aralkylgruppe und
$Z_2$ eine nukleophile Austrittsgruppe wie ein Halogenatom, z. B. ein Chlor- oder Bromatom, darstellen.

Die Umsetzung wird zweckmäßigerweise in einem Lösungsmittel wie Tetrahydrofuran, Methylenchlorid, Chloroform oder Dimethylformamid zweckmäßigerweise in Gegenwart einer Base wie Natriumcarbonat, Kaliumcarbonat oder Natronlauge oder in Gegenwart einer tertiären organischen Base wie Triethylamin, N-Ethyl-diisopropylamin, N-Methyl-morpholin oder Pyridin, welche gleichzeitig als Lösungsmittel dienen können, bei Temperaturen zwischen -30 und 100° C, vorzugsweise jedoch bei Temperaturen zwischen -10 und 80° C, durchgeführt.

e) Zur Herstellung von Verbindungen der allgemeinen Formel I, in der F eine durch eine Alkoxygruppe mit 1 bis 6 Kohlenstoffatomen substituierte Carbonylgruppe darstellt, wobei ein Alkoxyrest mit 1 bis 3 Kohlenstoffatomen in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann:
Umsetzung einer Verbindung der allgemeinen Formel

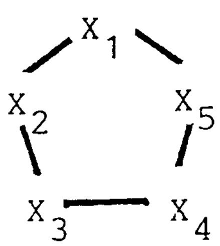

,(VIII)

in der
$X_1$ bis $X_5$ mit der Maßgabe wie eingangs definiert sind, daß einer der Reste $X_1$ bis $X_5$ eine Gruppe der Formel

F" - E - D - N< ,
F" - E - D - CH< oder

$$F'' - E - D - C\begin{smallmatrix}/\!\!/\\ \diagdown\end{smallmatrix}$$ darstellt,

in denen

E und D wie eingangs definiert sind und
F" eine Carboxy- oder Alkoxycarbonylgruppe darstellt, mit einem Alkohol der allgemeinen Formel

HO - $R_5$ ,(IX)

in der
$R_5$ eine Alkylgruppe mit 1 bis 6 Kohlenstoffatomen, die in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, darstellt.

Die Umsetzung wird zweckmäßigerweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, gegebenenfalls in Gegenwart von 4-Dimethylamino-pyridin, N,N'-Dicyclohexylcarbodiimid/N-Hydroxysuccinimid oder 1-Hydroxy-benztriazol, N,N'-Carbonyldiimidazol- oder N,N'-Thionyldiimidazol oder Triphenylphosphin/Tetrachlorkohlenstoff, zweckmäßigerweise bei Temperaturen zwischen 0 und 150 ° C, vorzugsweise bei Temperaturen zwischen 0 und 50 ° C, durchgeführt.

Die Umsetzung einer entsprechende Alkoxyverbindung mit einem Alkohol der allgemeinen Formel IX wird vorzugsweise in einem entsprechenden Alkohol als Lösungsmittel gegebenenfalls in Gegenwart eines weiteren Lösungsmittels wie Methylenchlorid oder Ether vorzugsweise in Gegenwart einer Säure wie Salzsäure bei Temperaturen zwischen 0 und 100 ° C, vorzugsweise bei Temperaturen zwischen 20 und 80 ° C, durchgeführt.

f) Zur Herstellung von Verbindungen der allgemeinen Formel I, in der A eine Aminoalkylgruppe darstellt:
Reduktion einer Verbindung der allgemeinen Formel

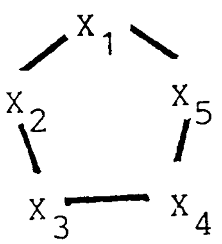

,(X)

in der
$X_1$ bis $X_5$ mit der Maßgabe wie eingangs definiert sind, daß einer der Reste $X_1$ bis $X_5$ eine Gruppe der Formel

A" - B - C - N< ,
A" - B - C - CH< oder

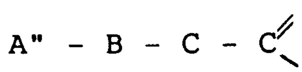 darstellt,

in denen

B und C wie eingangs definiert sind und
A" eine Cyanogruppe oder eine Cyanoalkylgruppe darstellt.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Lithiumborhydrid oder Lithiumaluminiumhydrid bei Temperaturen zwischen 0 und 100 ° C, vorzugsweise bei Temperaturen zwischen 20 und 80 ° C, durchgeführt.

g) Zur Herstellung von Verbindungen der allgemeinen Formel I, in der einer der Reste $X_1$ bis $X_5$ eine A - B - C - N< oder F - E - D - N< Gruppe darstellt:
Umsetzung einer Verbindung der allgemeinen Formel

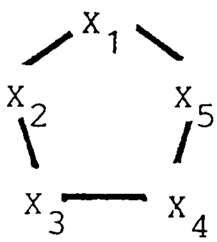

,(XI)

in der
$X_1$ bis $X_5$ mit der Maßgabe wie eingangs definiert sind, daß einer der Reste $X_1$ bis $X_5$ eine Iminogruppe darstellt, mit einer Verbindung der allgemeinen Formel

$Z_3$ - $R_6$ ,(XII)

in der
$R_6$ eine Gruppe der Formel
A - B - C - oder
F - E - D - darstellt, in denen
A bis F wie eingangs definiert sind und $Z_3$ eine nukleophile Austrittsgruppe wie ein Halogenatom oder einen Sulfonsäureesterrest, z.B. ein Chlor-, Brom- oder Jodatom oder eine Methansulfonyloxy- oder p-Toluolsulfonyloxygruppe darstellt.

Die Alkylierung wird zweckmäßigerweise in einem Lösungsmittel oder Lösungsmittelgemisch wie Methylenchlorid, Dimethylformamid, Dimethylsulfoxid, Benzol, Chlorbenzol, Tetrahydrofuran, Benzol/Tetrahydrofuran oder Dioxan vorzugsweise in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid oder eines Alkalihydrids wie Natriumhydrid oder einer tertiären organischen Base wie Ethyl-diisopropylamin zweckmäßigerweise bei Temperaturen zwischen 0 und 150 °C, vorzugsweise bei Temperaturen zwischen 0 und 50 °C, durchgeführt.

h) Zur Herstellung von Verbindungen der allgemeinen Formel I, in der einer der Reste $X_1$ bis $X_5$ eine A - B - C - N< oder F - E - D - N< Gruppe, der andere der Reste eine

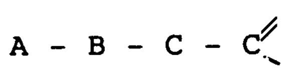 oder 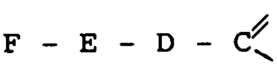

Gruppe und die verbleibenden Reste $X_1$ bis $X_5$ Stickstoffatome darstellen:
Umsetzung einer gegebenenfalls im Reaktionsgemisch hergestellten Verbindung der allgemeinen Formel

$$R' - N_2^{(+)} X \qquad ,(XIII)$$

mit einer Verbindung der allgemeinen Formel

R'' - CH = N - NH - $Z_4$ ,(XIV)

in denen
der Rest R' eine A-B-C- oder F-E-D-Gruppe darstellt, wobei jeweils der Rest C oder der Rest D eine Aryl- oder Heteroarylgruppe, die über ein Kohlenstoffatom an den 5-gliedrigen Ring gebunden ist, darstellt und
der Rest R'' der jeweils anderen der Gruppen A-B-C- und F-E-D entspricht, und
X das Anion einer anorganischen Säure wie das Chlorid-, Bromid- oder Jodidanion darstellen.

Die Umsetzung wird vorzugsweise in einem wässerigen Lösungsmittel wie Methanol/Wasser, Ethanol/Wasser oder Tetrahydrofuran/Wasser in Gegenwart einer Base wie Pyridin bei niederen Temperaturen, z. B. bei Temperaturen zwischen -20 und -10 °C, durchgeführt.

i) Zur Herstellung von Thiazolen der allgemeinen Formel I:

Umsetzung einer Verbindung der allgemeinen Formel

R' - CO - $CH_2$ - $Z_5$ ,(XV)

mit einer Verbindung der allgemeinen Formel

R'' - CS - $NH_2$ ,(XVI)

in denen
einer der Reste R' oder R'' eine A - B - C - Gruppe und der andere der Reste R' oder R'' eine F - E - D - Gruppe und $Z_5$ eine nukleophile Austrittsgruppe wie ein Chlor-, Brom- oder Jodatom darstellen.

Die Umsetzung wird zweckmäßigerweise in einem Lösungsmittel wie Methanol, Ethanol oder Isopropanol bei erhöhten Temperaturen, z.B. bei der Siedetemperatur des verwendeten Lösungsmittels, durchgeführt.

j) Zur Herstellung von Thiazolderivaten der allgemeinen Formel I:
Cyclisierung einer gegebenenfalls im Reaktionsgemisch gebildeten Verbindung der allgemeinen Formel

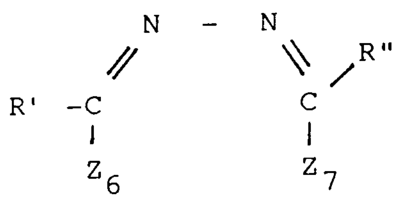

,(XVII)

in der
$Z_6$ und $Z_7$, die gleich oder verschieden sein können, Hydroxy-, Alkoxy-, Mercapto-, Alkylmercapto- oder Aminogruppen, einer der Reste R' oder R'' eine A - B - C - Gruppe und der andere der Reste R' oder R'' eine F - E - D - Gruppe darstellen.

Die Umsetzung wird zweckmäßigerweise in einem Lösungsmittel wie Tetrahydrofuran, Dioxan oder Pyridin bei Temperaturen bis zur Siedetemperatur des verwendeten Lösungsmittels, z.B. bei Temperaturen zwischen 50 und 80°C, durchgeführt.

k) Zur Herstellung von Verbindungen der allgemeinen Formel I, in der A und B oder A, B und C zusammen eine N-Amidino cyclische Iminogruppe mit 4 bis 7 Ringgliedern darstellen:
Umsetzung einer Verbindung der allgemeinen Formel

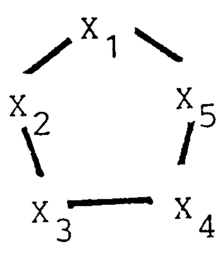

,(XVIII)

in der
$X_1$ bis $X_5$ mit der Maßgabe wie eingangs definiert sind, daß B oder B und C zusammen eine cyclische Iminogruppe mit 4 bis 7 Ringgliedern darstellen, mit einem S-Alkyl-isothioharnstoff.

Die Umsetzung wird zweckmäßigerweise in einem Lösungsmittel wie Dimethylformamid und vorzugsweise in Gegenwart einer Base wie Natriumcarbonat bei erhöhten Temperaturen, z.B. bei Temperaturen zwischen 80 und 120°C, durchgeführt.

l) Zur Herstellung von Verbindungen der allgemeinen Formel I, in der $X_1$ bis $X_5$ mit der Maßgabe wie eingangs definiert sind, daß einer der Reste $X_1$ bis $X_5$ eine Gruppe der Formel

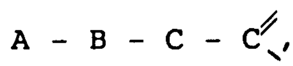

ein zweiter der Reste $X_1$ bis $X_5$ eine Gruppe der Formel

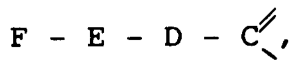

ein dritter der Reste $X_1$ bis $X_5$ ein Sauerstoffatom, ein vierter und fünfter der Reste $X_1$ bis $X_5$ eine

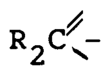

Gruppe darstellen:
Dehydratisierung einer Verbindung der allgemeinen Formel

$A - B - C - CO - CHR_2 - CHR_2 - CO - D - E - F$ ,(XIX)

in der
A bis F und $R_2$ wie eingangs definiert sind.

Die Umsetzung wird zweckmäßigerweise in einem Lösungsmittel wie Methylenchlorid, Chloroform, Benzol, Toluol, Xylol oder Chlorbenzol in Gegenwart eines wasserentziehenden Mittels wie Phosphorpentoxid, Acetanhydrid, Trifluoressigsäureanhydrid, Polyphosphorsäure, Schwefelsäure oder Phosphoroxychlorid bei Temperaturen zwischen 20 und 150 ° C, vorzugsweise bei Temperaturen zwischen 60 und 120 ° C, durchgeführt. Die Umsetzung kann auch ohne Lösungsmittel durchgeführt werden.

m) Zur Herstellung von Verbindungen der allgemeinen Formel I, in der A eine Guanidinogruppe darstellt:
Umsetzung einer Verbindung der allgemeinen Formel

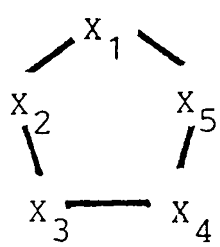

,(XX)

in der
$X_1$ bis $X_5$ mit der Maßgabe wie eingangs definiert sind, daß A eine Aminogruppe darstellt, mit Cyanamid oder dessen Säureadditionssalz.

Die Umsetzung wird zweckmäßigerweise in einem Lösungsmittel wie Dioxan, Dioxan/Wasser oder Tetrahydrofuran vorzugsweise bei Temperaturen zwischen 60 und 120 ° C, z.B. bei der Siedetemperatur des Reaktionsgemisches, durchgeführt.

n) Zur Herstellung von Verbindungen der allgemeinen Formel I, in der A eine Amino- oder Aminoalkylgruppe darstellt:
Umsetzung einer Verbindung der allgemeinen Formel

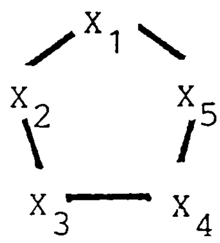

,(XXI)

in der
$X_1$ bis $X_5$ mit der Maßgabe wie eingangs definiert sind, daß einer der Reste $X_1$ bis $X_5$ eine $H_2N$-CO-T-B-C-Gruppe enthält, wobei B und C wie eingangs definiert sind und T eine Bindung oder eine Alkylengruppe mit 1 bis 5 Kohlenstoffatomen darstellt, mit einer Phenyljod(III)verbindung der allgemeinen Formel

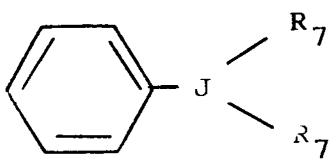

,(XXII)

in der
$R_7$ jeweils den Acylrest einer organischen Carbonsäure wie die Acetoxy- oder Trifluoracetoxygruppe darstellt.

Die Umsetzung wird vorzugsweise in einem wäßrigen Lösungsmittel wie Wasser oder Wasser/Acetonitril bei Temperaturen zwischen 0 und 50 °C, vorzugsweise jedoch bei Raumtemperatur, durchgeführt.

o) Zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt:
Reduktion einer Verbindung der allgemeinen Formel

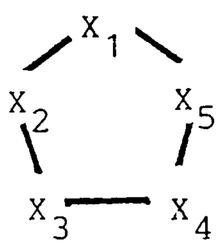

,(XXIII)

in der
$X_1$ bis $X_5$ mit der Maßgabe wie eingangs definiert sind, daß einer der Reste $X_1$ bis $X_5$ eine Gruppe der Formel

A''' - B - C - enthält, in der

B und C wie eingangs definiert sind und
A''' eine N-Hydroxy-iminogruppe enthält.

Die Reduktion wird vorzugsweise in einem geeigneten Lösungsmittel wie Methanol, Methanol/Wasser, Methanol/Wasser/Ammoniak, Ethanol, Ether, Tetrahydrofuran, Dioxan oder Dimethylformamid, gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, bei Temperaturen zwischen 0 und 100 °C, vorzugsweise bei Temperaturen zwischen 20 und 80 °C, durchgeführt.

p) Zur Herstellung von Verbindungen der allgemeinen Formel I, in der mindestens einer der Reste $X_1$ bis $X_5$ eine durch eine oder zwei Alkyl- oder Aralkylgruppen substituierte Aminogruppe oder eine durch eine

Alkylgruppe substituierte Iminogruppe enthält:
Umsetzung einer Verbindung der allgemeinen Formel

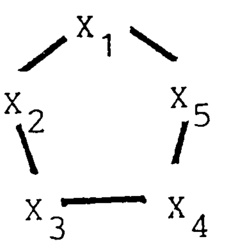 ,(XXIV)

in der
$X_1$ bis $X_5$ mit der Maßgabe wie eingangs definiert sind, daß einer der Reste $X_1$ bis $X_5$ eine Amino-, Alkylamino- oder Iminogruppe enthält, mit einer Verbindung der allgemeinen Formel

$Z_8$ - ($R_8$-C-$R_9$) - $Z_9$ ,(XXV)

in der
$R_8$ und $R_9$, die gleich oder verschieden sein können, Wasserstoffatome, Alkyl-, Aralkyl- oder Arylgruppen, eine der Gruppen $Z_8$ oder $Z_9$ eine nukleophile Austrittsgruppe wie ein Halogenatom, z.B. ein Chlor-, Brom- oder Jodatom, oder eine Sulfonsäureestergruppe, z.B. eine Methansulfonyloxy- oder p-Toluolsulfonyloxygruppe, und die andere der Gruppen $Z_8$ oder $Z_9$ ein Wasserstoffatom oder eine Alkylgruppe oder
$Z_8$ und $Z_9$ zusammen ein Sauerstoffatom bedeuten.

Die Alkylierung mit einer Verbindung der Formel XXV, in der $Z_8$ oder $Z_9$ eine nukleophile Austrittsgruppe darstellt, wird zweckmäßigerweise in einem Lösungsmittel wie Methylenchlorid, Tetrahydrofuran, Dioxan, Dimethylsulfoxid oder Dimethylformamid gegebenenfalls in Gegenwart einer Base wie Natriumcarbonat, Kaliumcarbonat oder Natronlauge oder in Gegenwart einer tertiären organischen Base wie N-Ethyl-diisopropylamin oder N-Methyl-morpholin, welche gleichzeitig als Lösungsmittel dienen können, bei Temperaturen zwischen -30 und 100 °C, vorzugsweise jedoch bei Temperaturen zwischen -10 und 80 °C, durchgeführt.

Die Alkylierung mit einer Carbonylverbindung der allgemeinen Formel XXV wird vorzugsweise in Gegenwart eines komplexen Metallhydrids wie Natriumborhydrid, Lithiumborhydrid oder Natriumcyanborhydrid in einem Lösungsmittel wie Wasser, Methanol, Ethanol oder Methanol/Wasser zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 5 bar durchgeführt.

q) Zur Herstellung von Verbindungen der allgemeinen Formel I, in der A eine durch eine Dialkylphosphorylgruppe mit jeweils 1 bis 3 Kohlenstoffatomen im Alkylteil substituierte Amino-, Aminoalkyl-, Amidino- oder Guanidinogruppe darstellt:
Umsetzung einer Verbindung der allgemeinen Formel

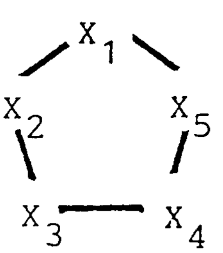 ,(XXVI)

in der
$X_1$ bis $X_5$ mit der Maßgabe wie eingangs definiert sind, daß einer der Reste $X_1$ bis $X_5$ eine Gruppe der Formeln

A' - B - C - N< ,
A' - B - C - CH< oder

A' - B - C - C< darstellt,

in denen

B und C wie eingangs definiert sind und
A' eine Amino-, Aminoalkyl-, Amidino- oder Guanidinogruppe darstellt, mit einer Verbindung der allgemeinen Formel

$Z_{10}$ - PO($OR_{10}$)$_2$ ,(XXVII)

in der
$R_{10}$ eine Alkylgruppe mit 1 bis 3 Kohlenstoffatomen und $Z_{10}$ eine nukleophile Austrittsgruppe wie eine Cyanogruppe, ein Chlor- oder Bromatom darstellen.

Die Umsetzung wird zweckmäßigerweise in einem Lösungsmittel wie Dimethylformamid bei Temperaturen zwischen 0 und 100 ° C, vorzugsweise bei Temperaturen zwischen 15 und 50 ° C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, tert.Butyl-, Trityl-, Benzyl- oder Tetrahydropyranylgruppe,
als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder mittels Etherspaltung, z.B. in Gegenwart von Jodtrimethylsilan, bei Temperaturen zwischen 0 und 100 ° C, vorzugsweise bei Temperaturen zwischen 10 und 50 ° C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50 ° C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50 ° C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50 ° C.

Erhält man erfindungsgemäß eine Verbindung der allgemeinen Formel I, welche eine Carboxygruppe enthält, so kann diese mittels Veresterung in eine entsprechende Esterverbindung übergeführt werden.

Die anschließende Veresterung wird zweckmäßigerweise in einem geeigneten Lösungsmittel, z. B. in einem entsprechenden Alkohol wie Methanol, Ethanol oder Isopropanol, Methylenchlorid, Tetrahydrofuran, Dioxan, Pyridin, Toluol oder Dimethylsulfoxid in Gegenwart eines säureaktivierenden und/oder wasserentziehenden Mittels wie Chlorwasserstoff, konz. Schwefelsäure, Thionylchlorid, Chlorameisensäureethylester, Carbonyldiimidazol oder N,N'-Dicyclohexyl-carbodiimid oder dessen Isoharnstoffestern, gegebenenfalls in

Gegenwart eines Reaktionsbeschleunigers wie Kupferchlorid, durch Umesterung, z. B. mit einem entsprechenden Kohlensäurediester, oder durch Umsetzung mit einem entsprechenden Halogenid vorzugsweise in Gegenwart einer Base wie Kaliumcarbonat und gegebenenfalls in Gegenwart eines Reaktionsbeschleunigers wie Kaliumjodid bei Temperaturen zwischen 0 und 100°C, vorzugsweise jedoch bei Temperaturen zwischen 20°C und der Siedetemperatur des betreffenden Lösungsmittels, durchgeführt.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihren aktivierten Derivaten oder Alkoholen, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)- oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Essigsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxylgruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Ammoniak, Cyclohexylamin, Äthanolamin, Diäthanolamin und Triäthanolamin in Betracht.

Die als Ausgangsstoffe verwendeten Verbindungen sind teilweise literaturbekannt oder man erhält diese nach literaturbekannten Verfahren wie diese in den Beispielen I bis XII beschrieben werden.

Wie bereits eingangs erwähnt, weisen die neuen 5-gliedrigen Heterocyclen der allgemeinen Formel I und deren Additionssalze, insbesondere deren physiologisch verträgliche Additionssalze mit anorganischen oder organischen Säuren oder Basen, wertvolle Eigenschaften auf. So weisen die neuen Verbindungen der allgemeinen Formel I, in denen A eine gegebenenfalls am Stickstoff substituierte Amino-, Aminoalkyl-, Amidino- oder Guanidinogruppen oder eine gegebenenfalls in vivo in eine gegebenenfalls am Stickstoff substituierte Amino-, Amidino- oder Guanidinogruppe überführbare Gruppe, z.B. eine durch eine Alkoxycarbonylgruppe am Stickstoff substituierte Amino-, Aminoalkyl-, Amidino- oder Guanidinogruppe, enthält oder B oder B und C zusammen eine am Stickstoffatom gegebenenfalls alkylierte cyclische Iminogruppe darstellen und -D-E-F Carboxyl-, Sulfo-, Phosphono-, O-Alkyl-phosphono- oder 5-Tetrazolylgruppen oder in vivo in Carboxyl-, Sulfo-, Phosphono-, O-Alkyl-phosphono- oder Tetrazolylgruppe überführbare Gruppen, z.B. durch eine Alkoxygruppe substituierte Carbonylgruppen, enthält, wertvolle pharmakologische Eigenschaften auf, neben einer entzündungshemmenden und den Knochenabbau hemmenden Wirkung insbesondere antithrombotische, antiaggregatorische und tumor- bzw. metastasenhemmende Wirkungen.

Die Verbindungen der allgemeinen Formel I, in der A eine Cyano- oder Cyanoalkylgruppe darstellt, stellen wertvolle Zwischenprodukte zur Herstellung der entsprechenden Aminoalkyl- und Amidinoverbindungen der allgemeinen Formel I dar.

Beispielsweise wurden die Verbindungen der allgemeinen Formel I auf ihre biologischen Wirkungen wie folgt untersucht:

1. Fibrinogen-Bindung an Humanthrombozyten

Das durch Punktion einer Antekubitalvene gewonnene Blut wird mit Trinatriumcitrat (Endkonzentration: 13 mM) antikoaguliert und 10 Minuten bei 170 *g zentrifugiert. Das überstehende plättchenreiche Plasma wird auf eine Sepharose 2B-Säule (Pharmacia) gegeben und mit einer Lösung aus 90 mM Kochsalz, 14 mM Trinatriumcitrat, 5 mM Glucose und 50 mM Tris(hydroxymethyl)aminomethan, eingestellt auf pH 7,4, eluiert. Die vor den Plasmaproteinen erscheinenden gelfiltrierten Plättchen (GFP) werden für die Bindungsversuche verwendet.

50 µl einer 60 mM Calziumchlorid-Lösung, 50 µl einer 0,6 mM Adenosindiphosphat-Lösung, 100 µl Substanzlösung bzw. Lösungsmittel und 50 µl Fibrinogenlösung (enthaltend 3 µg 125-J-Fibrinogen) werden zu 750 µl GFP gegeben und bei Raumtemperatur 20 Minuten inkubiert. Die unspezifische Bindung wird in Gegenwart von 3 mg/ml kaltem Fibrinogen bestimmt.

900 µl des Inkubates werden vorsichtig auf 250 µl Silikonöl (AP 38: AR 20, 1:2 v/v, Wacker Chemie) in Eppendorf-Gefäße pipettiert und 2 Minuten bei 10 000 *g zentrifugiert. Der wäßrige Überstand und ein Teil des Öls werden abgezogen, die Gefäßspitze mit dem Plättchenpellet abgeschnitten und im Gamma-Zähler die Menge des gebundenen Fibrinogens bestimmt. Aus einer Konzentrationsreihe wird die Substanzkonzentration ermittelt, welche die Fibrinogenbindung zu 50 % hemmt und als $IC_{50}$ angegeben.

2. Antithrombotische Wirkung

Methodik

Die Thrombozytenaggregation wird nach der Methode von Born und Cross (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München. Vor der Collagen-Zugabe wird das Plasma jeweils 10 Minuten mit der Substanz bei 37° C inkubiert.

Aus den erhaltenen Meßzahlen wird graphisch eine $EC_{50}$ bestimmt, die sich auf eine 50%ige Änderung der "optical density" im Sinne einer Aggregationshemmung bezieht.

Die nachfolgende Tabelle enthält die gefundenen Ergebnisse:

| Substanz (Beispiel Nr.) | Fibrinogen-Bindungstest $IC_{50}$[nM] | Hemmung der Plättchenaggregation $EC_{50}$[nM] |
|---|---|---|
| 1 | 73 | 130 |
| 1(1) | 38 | 90 |
| 1(2) | 18 | 90 |
| 1(3) | 220 | 11 000 |
| 1(4) | 44 | 290 |
| 1(6) | 25 | 90 |
| 1(7) | 210 | 580 |
| 1(8) | 460 | 4 100 |
| 1(9) | 4 200 | 8 500 |
| 1(10) | 2 000 | 9 500 |
| 1(20) | 65 | 290 |
| 1(35) | 2 400 | 6 900 |
| 1(37) | 150 | 2 400 |
| 1(39) | 39 | 3 700 |
| 1(127) | 2 500 | 14 000 |
| 1(128) | 3 500 | 9 000 |
| 1(134) | 460 | 510 |
| 2 | 510 | 2 200 |
| 2(1) | 51 | 180 |
| 2(2) | 340 | 350 |
| 2(3) | 140 | 1 600 |
| 2(4) | 490 | 370 |
| 2(5) | 3 100 | 7 500 |
| 2(6) | 4 300 | 210 |
| 2(7) | 17 000 | 800 |
| 2(8) | 44 000 | 6 400 |
| 2(10) | - | 220 |
| 2(20) | 4 900 | 280 |
| 2(37) | 6 200 | 6 100 |
| 2(39) | 130 | 5 400 |
| 2(126) | 1 400 | 22 000 |
| 4(1) | 1 500 | - |
| 4(2) | 1 800 | - |

Die erfindungsgemäßen Verbindungen sind gut verträglich, da beispielsweise bei intravenöser Gabe von 30 mg/kg an jeweils drei Mäusen bei den Verbindungen der Beispiele 1 und 1(2) keine Tiere gestorben waren.

Auf Grund ihrer Hemmwirkung auf Zell-Zell- bzw. Zell-Matrix-Wechselwirkungen eignen sich die neuen 5-gliedrigen Heterocyclen der allgemeinen Formel I und ihre physiologisch verträglichen Additionssalze zur Bekämpfung bzw. Verhütung von Krankheiten, bei denen kleinere oder größere Zell-Aggregate auftreten oder Zell-Matrixinteraktionen eine Rolle spielen, z.B. bei der Bekämpfung bzw. Verhütung von venösen und arteriellen Thrombosen, von zerebrovasculären Erkrankungen, von Lungenembolien, des Herzinfarktes, der Arteriosklerose, der Osteoporose und der Metastasierung von Tumoren und der Therapie genetisch bedingter oder auch erworbener Störungen der Interaktionen von Zellen untereinander oder mit soliden Strukturen. Weiterhin eignen sich diese zur Begleittherapie bei der Thrombolyse mit Fibrinolytica oder Gefäßinterventionen wie transluminaler Angioplastie oder auch bei der Therapie von Schockzuständen, der Psoriasis des Diabetes und von Entzündungen.

Für die Bekämpfung bzw. Verhütung der vorstehend erwähnten Krankheiten liegt die Dosis zwischen 0,1 $\mu$g und 20 mg/kg Körpergewicht, vorzugsweise bei 1 $\mu$g bis 10 mg/kg Körpergewicht, bei bis zu 4 Gaben pro Tag. Hierzu lassen sich die erfindungsgemäß hergestellten Verbindungen der Formel I, gegebenenfalls in Kombination mit anderen Wirksubstanzen wie Thromboxan-Rezeptor-Antagonisten und Thromboxansynthesehemmer oder deren Kombinationen, Serotonin-Antagonisten, $\alpha$-Rezeptorantagonisten, Alkylnitrate wie Glycerintrinitrat, Phosphodiesterasehemmer, Prostacyclin und deren Analoga, Fibrinolytica wie tPA, Prourokinase, Urokinase, Streptokinase, oder Antikoagulantien wie Heparin, Dermatansulfat, aktiviertes Protein C, Vitamin K-Antagonisten, Hirudin, Inhibitoren des Thrombins oder anderer aktivierter

Gerinnungsfaktoren, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Stearylalkohol, Carboxymethylcellulose oder fetthaltigen Substanze wie Hartfett oder deren geeigneten Gemischen, in übliche galenische Zubereitungen wie Tabletten, Dragées, Kapseln, Pulver, Suspensionen, Lösungen, Sprays oder Zäpfchen einarbeiten.

Die nachfolgenden Beispiele sollen die Erfindung näher erläutern:

## Beispiel I

### 3-(4-Imidazolyl)-propionsäure-methylester

2,3 g 3-(4-Imidazolyl)-acrylsäure-methylester werden in 50 ml Methanol in Gegenwart von 0,5 g 10%iger Palladiumkohle 4 Stunden mit Wasserstoff von 5 bar bei Raumtemperatur behandelt. Nach Abfiltrieren des Katalysators wird das Filtrat im Vakuum eingedampft und der verbleibende Rückstand als Rohprodukt weitverwendet.

Ausbeute: 2,3 g (100 % der Theorie),

$R_f$-Wert: 0,35 (Kieselgel; Methylenchlorid/Methanol 9:1)

Analog wird folgende Verbindung erhalten:

(1) 3-(4-Imidazolyl)-propionsäure

Man setzt 10 Vol.-% 1N Salzsäure zu und hydriert eine Stunde bei 50 °C

$R_f$-Wert: 0,48 (Kieselgel; Methylenchlorid/Methanol/konz. Ammoniak = 4:1:0,25)

Der Methylester wird hieraus durch Behandeln mit gesättigter methanolischer Salzsäure erhalten.

## Beispiel II

### 5-(4-Cyan-4'-biphenylyl)-tetrazol

Eine Lösung von 0,5 g Aluminiumtrichlorid in 3 ml Tetrahydrofuran wird zu einer gerührten Suspension von 1,5 g 4,4'-Dicyanbiphenyl und 0,75 g Natriumazid in 3 ml Tetrahydrofuran getropft und 16 Stunden zum Rückfluß erhitzt. Man fügt weitere 0,2 g Natriumazid und 0,15 g Aluminiumtrichlorid, gelöst in 1 ml Tetrahydrofuran, zu und erhitzt nochmals 6 Stunden zum Rückfluß. Man stellt mit 1N Salzsäure sauer und destilliert das Tetrahydrofuran im Vakuum ab. Das ausgefallene Rohprodukt wird durch Chromatographie an Kieselgel gereinigt (Elutionsmittel: Methylenchlorid/Methanol/konz. Ammoniak = 4:1:0,25).

Ausbeute: 0,9 g (50 % der Theorie),

$R_f$-Wert: 0,28 (Kieselgel; Methylenchlorid/Methanol/konz. Ammoniak = 4:1:0,25)

## Beispiel III

### 4-(2-Methoxycarbonyl-ethyl)-thiobenzoesäure-amid

5 g 4-(2-Methoxycarbonyl-ethyl)-benzamid, 5 g 2,4-Bis(4-methoxy-phenyl)-1,3-dithia-2,4-diphosphetan-2,4-disulfid und 50 ml Tetrahydrofuran werden 4 Stunden bei Raumtemperatur gerührt. Der Niederschlag wird abfiltriert, das Filtrat zur Trockene eingedampft und der Rückstand ohne weitere Reinigung weiterverwendet.

$R_f$-Wert: 0,39 (Kieselgel; Cyclohexan/Essigester = 1:1)

## Beispiel IV

### 4-(2-Methoxycarbonyl-ethyl)-benzamidin-hydrochlorid

Hergestellt analog Beispiel 2 aus 3-(4-Cyan-phenyl)-propionsäure-methylester.

Schmelzpunkt: 175-178 °C

$R_f$-Wert: 0,78 (Reversed-Phase-Platte; 5%ige Natriumchloridlösung/Methanol = 4:6)

## Beispiel V

### 3-(4-Cyan-phenyl)-propionsäure-methylester

30,1 g 3-(4-Cyan-phenyl)-propionsäure werden in 760 ml Methanol gelöst und mit 30 ml gesättigter methanolischer Salzsäure versetzt. Man rührt 16 Stunden bei Raumtemperatur, dampft im Vakuum ein, nimmt mit tert.Butyl-methylether auf und wäscht mit Wasser. Das nach dem Eindampfen der organischen Phase verbleibende Rohprodukt wird ohne weitere Reinigung weiterverwendet.
Ausbeute: 30,7 g (89 % der Theorie),
Schmelzpunkt: 44-48 ° C
Analog wird folgende Verbindung erhalten:
(1) 3-(3-Indolyl)-propionsäure-methylester
$R_f$-Wert: 0,39 (Kieselgel; Cyclohexan/Essigester = 3:1)

Beispiel VI

3-(4-Cyan-phenyl)-propionsäure

39,4 g (4-Cyan-benzyl)-malonsäure, 45 ml Pyridin und 0,8 ml Piperidin werden 1,5 Stunden bei 100 ° C gerührt. Nach dem Abkühlen versetzt man mit 450 ml Eiswasser. Säuert mit 300 ml 2N Salzsäure an und extrahiert mit Essigester. Die organische Phase wird mit gesättigter Kochsalzlösung gewaschen und eingeengt. Der verbleibende Rückstand wird ohne weitere Reinigung weiterverwendet.
Ausbeute: 30,1 g (94 % der Theorie),
$R_f$-Wert: 0,46 (Kieselgel; Methylenchlorid/Methanol = 19:1)

Beispiel VII

(4-Cyan-benzyl)-malonsäure

63 g (4-Cyan-benzyl)-methan-tricarbonsäure-triethylester werden in 160 ml Methanol gelöst und mit einer Mischung aus 37 ml 15N Natronlauge und 16 ml Methanol tropfenweise unter Rühren versetzt. Man erhitzt 30 Minuten zum Rückfluß, versetzt mit Eiswasser und schüttelt mit Essigester aus. Die wäßrige Phase wird mit halbkonzentrierter Salzsäure angesäuert und mit Essigester extrahiert. Die Essigesterphase wird mit gesättigter Natriumchloridlösung gewaschen und eingeengt. Das zurückbleibende Rohprodukt wird ohne weitere Reinigung weiterverarbeitet.
Ausbeute: 39,4 g (98 % der Theorie),
$R_f$-Wert: 0,47 (Kieselgel; Methylenchlorid/Methanol = 19:1)

Beispiel VIII

(4-Cyan-benzyl)-methan-tricarbonsäure-triethylester

44,9 g Methan-tricarbonsäure-triethylester werden in 180 ml Dimethylformamid gelöst und mit 21,9 g Kalium-tert.butylat versetzt. Man kühlt auf Raumtemperatur ab, gibt 36,2 g 4-Cyan-benzylbromid zu und rührt 16 Stunden bei Raumtemperatur. Man filtriert vom gebildeten Kaliumbromid ab und dampft das Lösungsmittel im Vakuum ab. Das zurückbleibende Rohrprodukt wird ohne weitere Reinigung weiterverwendet.
Ausbeute: 63 g (99 % der Theorie),
$R_f$-Wert: 0,80 (Kieselgel; Methylenchlorid/Methanol = 19:1)
Analog wird folgende Verbindung erhalten:
(1) 7-(4-Cyan-4'-biphenylyl)-6-ethoxycarbonyl-4,7-dioxo-heptansäure-ethylester
Hergestellt aus (4-Cyan-4'-biphenylyl)-carbonyl-essigsäureethylester und 5-Brom-laevulinsäure durch Rückflußkochen in Aceton in Gegenwart von Kaliumkarbonat.
$R_f$-Wert: 0,42 (Kieselgel; Essigester/Cyclohexan = 1:25, zweifach entwickelt)

Beispiel IX

N-(4-Cyan-benzoyl)-N'-[4-(2-methoxycarbonyl-ethyl)-benzoyl]hydrazin

Zu einer Suspension von 1,86 g 4-Cyan-benzhydrazid (hergestellt aus 4-Cyan-benzoesäuremethylester und 80%igem Hydrazinhydrat) in 25 ml Methylenchlorid gibt man 3,1 g N-Ethyldiisopropylamin und tropft anschließend eine Lösung von 4-(2-Methoxycarbonyl-ethyl)-benzoylchlorid (hergestellt aus dem zugehörigen Benzonitril über das Amid und die Säure) zu und läßt 24 Stunden bei Raumtemperatur stehen. Die Methylenchloridphase wird mit Wasser, 1N Salzäure und Wasser gewaschen und eingeengt. Der Rückstand wird mit wenig Methanol digeriert und das entstandene Festprodukt abfiltriert.
Ausbeute: 1,7 g (42 % der Theorie),
$R_f$-Wert: 0,51 (Kieselgel; Methylenchlorid/Methanol = 19:1)
Analog werden folgende Verbindungen erhalten:

(1) 1-[(4-Cyan-4'-biphenylyl)-carbonyl]-thiosemicarbazid Man verwendet Eisessig als Lösungsmittel und Natriumacetat als Base.
Schmelzpunkt: 231 °C (Zers.)

(2) N-[(4-Cyan-4'-biphenylyl)-carbonyl]-N'-(2-methoxycarbonyl-ethylcarbonyl)-hydrazin
$R_f$-Wert: 0,78 (Kieselgel; Methylenchlorid/Methanol = 17:3)

Beispiel X

(4-Cyan-4'-biphenylyl)-carbonyl-essigsäure-ethylester

14,9 g Malonsäure-monoethylester werden in 750 ml Tetrahydrofuran gelöst und bei -65 bis -70 °C mit 141 ml einer 1,6 molaren Lösung von n-Butyllithium in Hexan versetzt. Man rührt 15 Minuten bei -65 °C und noch eine Stunde bei 0 °C, kühlt dann auf -65 °C ab und tropft unter Rühren eine Lösung von 18,1 g 4'-Cyan-4-biphenylylcarbonylchlorid in 75 ml Tetrahydrofuran zu. Man rührt 45 Minuten bei -60 °C weiter, läßt auf 0 °C kommen und rührt weitere 3 Stunden bei 0 °C. Das Reaktionsgemisch wird in eine eiskalte Mischung aus 125 ml 1N Salzsäure und 300 ml Ether eingerührt. Die organische Phase wird mit Natriumbicarbonatlösung und Wasser gewaschen und die wäßrigen Phasen mit Ether extrahiert. Die vereinigten organischen Phasen werden zur Trockne eingedampft und der Rückstand über Kieselgel gereinigt (Elutionsmittel: Essigester/Cyclohexan = 1:2,5).
Ausbeute: 16,1 g (69 % der Theorie),
Schmelzpunkt: 89-91 °C
$R_f$-Wert: 0,64 (Kieselgel; Essigester/Cyclohexan = 1:2,5, zweifach entwickelt)

Beispiel XI

3-Chlor-6-(4-methoxycarbonylamidino-phenyl)-pyridazin

Hergestellt aus 3-Chlor-6-(4-amidino-phenyl)-pyridazin und Chlorameisensäure-methylester analog Beispiel 4. $R_f$-Wert: 0,35 (Kieselgel; Methylenchlorid/Methanol = 15:1)

Beispiel XII

3-(4-Cyan-4'-biphenylyl)-5-mercapto-1,2,4-triazol

1,2 g 1-[(4-Cyan-4'-biphenylyl)-carbonyl]-thiosemicarbazid werden in einer Lösung von 0,43 g Natriumcarbonat in 5 ml Wasser 1,5 Tage auf dem Dampfbad erwärmt. Man versetzt mit Ammoniumchloridlösung, filtriert den Niederschlag ab und trocknet ihn bei 80 °C.
Ausbeute: 1,1 g (88 % der Theorie),
Schmelzpunkt: über 275 °C
$R_f$-Wert: 0,66 (Kieselgel; Essigester/Ethanol = 50:2)

Beispiel 1

1-[6-(4-Amidino-phenyl)-3-pyridazinyl]-4-(2-carboxy-ethyl)imidazol

Eine Mischung aus 0,18 g 1-[6-(4-Amidino-phenyl)-3-pyridazinyl]-4-(2-methoxycarbonyl-ethyl)-imidazol, 0,087 g Lithiumhydroxid-hydrat, 20 ml Tetrahydrofuran und 16 ml Wasser wird 2 Stunden bei Raumtemperatur gerührt. Man versetzt mit 1 g Ammoniumchlorid und rührt 30 Minuten nach. Das Tetrahydrofuran wird im Vakuum abdestilliert, der ausgefallene Niederschlag abgesaugt, mit Wasser gewaschen und getrocknet.
Ausbeute: 0,13 g (76 % der Theorie),
Schmelzpunkt: über 260 °C
$R_f$-Wert: 0,12 (Kieselgel; Methylenchlorid/Methanol/konz. Ammoniak = 2:1:0,25)

Analog werden folgende Verbindungen erhalten:

(1) 1-[6-(4-Amidino-phenyl)-3-pyridazinyl]-4-(2-carboxy-2-hydroxy-ethyl)-imidazol

$R_f$-Wert: 0,09 (Kieselgel; Methylenchlorid/Methanol/konz. Ammoniak = 2:1:0,25)

(2) 1-[6-(4-Amidino-phenyl)-3-pyridazinyl]-4-(2-amino-2-carboxy-ethyl)-imidazol-hydrochlorid

$R_f$-Wert: 0,04 (Kieselgel; Methylenchlorid/Methanol/konz. Ammoniak = 2:1:0,25)

| Ber. x 0,6 HCl x 0,75 $H_2O$: | | | | |
|---|---|---|---|---|
| | C 52,80 | H 4,78 | N 25,36 | Cl 5,51 |
| Gef.: | 53,29 | 4,76 | 24,97 | 5,99 |

(3) 5-(4-Amidino-phenyl)-2-[4-(2-carboxy-ethyl)-phenyl]-tetrazol

$R_f$-Wert: 0,06 (Kieselgel; Methylenchlorid/Methanol/konz. Ammoniak = 2:1:0,25)

| | | | |
|---|---|---|---|
| Ber. x $H_2O$: | C 57,62 | H 5,12 | N 23,72 |
| Gef.: | 57,57 | 5,17 | 23,41 |

(4) 5-(4-Amidino-4'-biphenylyl)-2-(2-carboxy-ethyl)-tetrazol

$R_f$-Wert: 0,06 (Kieselgel; Methylenchlorid/Methanol/konz. Ammoniak = 2:1:0,25)

(5) 4-(4-Amidino-phenyl)-2-[4-(2-carboxy-ethyl)-phenyl]-thiazol

Man arbeitet mit Natronlauge in Methanol

Schmelzpunkt: 310-315° C (Zers.)

$R_f$-Wert: 0,32 (Reversed-Phase-Platte RP8; 5%ige Natriumchloridlösung/Methanol = 4:6)

(6) 4-(4-Amidino-phenyl)-2-[4-(2-carboxy-ethyl)-phenyl]-1-methyl-imidazol

Man verfährt wie unter (5)

Schmelzpunkt: über 200° C

$R_f$-Wert: 0,79 (Reversed-Phase-Platte RP8; 5%ige Natriumchloridlösung/Methanol = 4:6)

| | | | |
|---|---|---|---|
| Ber. x 0,5 $H_2O$: | C 67,21 | H 5,92 | N 15,68 |
| Gef.: | 67,24 | 6,04 | 15,61 |

(7) 4-(4-Amidino-phenyl)-2-[4-(2-carboxy-ethyl)-phenyl]-imidazol

Man verfährt wie unter (5)

Schmelzpunkt: 310-315° C (Zers.)

$R_f$-Wert: 0,75 (Reversed-Phase-Platte RP8; 5%ige Natriumchloridlösung/Methanol = 4:6)

| | | | |
|---|---|---|---|
| Ber.: C | 68,25 | H 5,43 | N 16,76 |
| Gef.: | 67,72 | 5,36 | 16,71 |

(8) 3-(4-Amidino-phenyl)-5-[4-(2-carboxy-ethyl)-phenyl]-1,2,4-triazol

Man verfährt wie unter (5)

$R_f$-Wert: 0.08 (Kieselgel; Methylenchlorid/Methanol = 7:3)

(9) 2-(4-Amidino-phenyl)-5-[4-(2-carboxy-ethyl)-phenyl]-1,3,4-oxadiazol

Man verfährt wie unter (5)

$R_f$-Wert: 0,08 (Kieselgel; Methylenchlorid/Methanol = 7,5:2,5)

(10) 2-(4-Amidino-phenyl)-5-[4-(2-carboxy-ethyl)-phenyl]-1,3,4-thiadiazol

$R_f$-Wert: 0,17 (Kieselgel; Methylenchlorid/Methanol = 7:3)

(11) 1-[5-(4-Amidino-phenyl)-2-pyrimidyl)]-4-(2-amino-2-carboxy-ethyl)-imidazol

(12) 1-[5-(4-Amidino-phenyl)-4-methyl-2-pyrimidyl]-4-(2-amino-2-carboxy-ethyl)-imidazol

(13) 1-[5-(4-Amidino-phenyl)-2-pyrazinyl]-4-(2-amino-2-carboxy-ethyl)-imidazol

(14) 1-[5-(4-Amidino-phenyl)-4-methyl-2-pyrimidyl]-4-(2-carboxy-ethyl)-imidazol

(15) 1-[5-(4-Amidino-phenyl)-2-pyrimidyl]-4-(2-carboxyethyl)-imidazol

(16) 1-(4-Amidino-4'-biphenylyl)-4-(2-carboxy-ethyl)-imidazol

(17) 4-(4-Amidino-4'-biphenylyl)-2-(2-carboxy-ethyl)-thiazol

(18) 5-(4-Amidino-4'-biphenylyl)-2-(2-carboxy-ethyl)-oxazol

(19) 2-(4-Amidino-4'-biphenylyl)-5-(2-carboxy-ethyl)-1,3,4-oxadiazol
(20) 2-(4-Amidino-4'-biphenylyl)-5-(2-carboxy-ethyl)-1,3,4-thiadiazol
Man arbeitet mit Natriumhydroxid
$R_f$-Wert: 0,16 (Kieselgel; Methylenchlorid/Methanol = 17:3)
(21) 2-(4-Amidino-4'-biphenylyl)-4-(2-carboxy-ethyl)-thiazol
(22) 2-(4-Amidino-4'-biphenylyl)-5-(2-carboxy-ethyl)-thiophen
(23) 2-(4-Amidino-4'-biphenylyl)-5-(2-carboxy-ethyl)-furan
(24) 1-(4-Amidino-3'-brom-4'-biphenylyl)-4-(2-carboxy-ethyl)imidazol
(25) 1-[6-(4-Amidino-phenyl)-3-pyridazinyl]-4-(2-carboxy-ethyl)-2-methyl-imidazol
(26) 1-[6-(4-Amidino-phenyl)-3-pyridazinyl]-4-(2-carboxy-ethyl)-2-isopropyl-imidazol
(27) 1-[6-(4-Amidino-phenyl)-3-pyridazinyl]-4-(2-carboxy-ethyl)-2-hexyl-imidazol
(28) 1-[6-(4-Amidino-phenyl)-3-pyridazinyl]-4-(2-carboxy-ethyl)-2-(2-phenyl-ethyl)-imidazol
(29) 1-[6-(4-Amidino-phenyl)-3-pyridazinyl]-4-(2-carboxy-ethyl)-2-phenyl-imidazol
(30) 1-[6-(4-Amidino-phenyl)-3-pyridazinyl]-4-(2-carboxy-ethyl)-2-(3-pyridyl)-imidazol
(31) 3-(4-Amidino-4'-biphenylyl)-5-(2-carboxy-ethyl)-1,2,4-triazol
(32) 1-[6-(4-Amidino-phenyl)-3-pyridazinyl]-4-(3-carboxy-propyl)-imidazol
(33) 2-(4-Amidino-3'-methoxy-4'-biphenylyl)-5-(2-carboxy-ethyl)-1,3,4-thiadiazol
(34) 2-(4-Amidino-3'-methyl-4'-biphenylyl)-5-(2-carboxy-ethyl)-1,3,4-thiadiazol
(35) 1-[6-(4-Amidino-phenyl)-3-pyridazinyl]-4-(2-carboxy-2-dibenzylamino-ethyl)-imidazol
$R_f$-Wert: 0,21 (Kieselgel; Methylenchlorid/Methanol/konz. Ammoniak = 2:1:0,25)
(36) 1-[6-(4-Amidino-phenyl)-3-pyridazinyl]-4-(2-carboxy-2-dimethylamino-ethyl)-imidazol
(37) 1-[6-(4-Amidino-phenyl)-3-pyridazinyl]-3-(2-carboxy-ethyl)-indol
Schmelzpunkt: 288-292 ° C
$R_f$-Wert: 0,03 (Kieselgel; Methylenchlorid/Methanol/konz. Ammoniak = 2:1:0,25)
(38) 4-(4-Amidino-4'-biphenylyl)-2-(2-carboxy-ethyl)-5-methyl-thiazol
(39) 3-(4-Amidino-4'-biphenylyl)-5-carboxymethylthio-1,2,4-triazol
Man arbeitet mit Natriumhydroxid.
Schmelzpunkt: über 245 ° C

| | | | |
|---|---|---|---|
| Ber. x 4 $H_2O$: | C 48,00 | H 5,44 | N 16,46 |
| Gef.: | 47,52 | 5,61 | 16,45 |

(40) 4-(4-Amidino-4'-biphenylyl)-2-carboxymethylamino-thiazol
(41) 2-[1-(4-Amidino-phenyl)-4-piperidinyl]-4-(2-carboxy-ethyl)-thiazol
(42) 2-[4-(4-Amidino-phenyl)-1-piperazinyl]-4-(2-carboxy-ethyl)-thiazol
(43) 2-[1-(5-Amidino-2-pyridyl)-4-piperidinyl]-4-(2-carboxy-ethyl)-thiazol
(44) 1-[4-(5-Amidino-2-pyrimidyl)-phenyl]-4-(2-carboxy-ethyl)-imidazol
(45) 1-[4-(5-Amidino-2-pyrazinyl)-phenyl]-4-(2-carboxy-ethyl)-imidazol
(46) 4-[4-(1-Amidino-4-piperidinyl)-phenyl]-2-(2-carboxy-ethyl)-thiazol
(47) 1-[4-(4-Amidino-phenyl)-cyclohexyl]-4-(2-carboxy-ethyl)-imidazol
(48) 1-[6-(4-Amidino-phenyl)-3-pyridazinyl]-3-(2,2-bis-carboxy-ethyl)-indol
(49) 2-[1-(4-Amidino-phenyl)-4-piperidinyl]-5-(2-carboxy-ethyl)-1,3,4-oxadiazol
(50) 2-[1-(5-Amidino-2-pyridyl)-4-piperidinyl]-5-(2-carboxy-ethyl)-1,3,4-oxadiazol
(51) 5-[1-(4-Amidino-phenyl)-4-piperidinyl]-2-(2-carboxy-ethyl)-tetrazol
(52) 5-[1-(5-Amidino-2-pyridyl)-4-piperidinyl]-2-(2-carboxy-ethyl)-tetrazol
(53) 2-[6-(4-Amidino-phenyl)-3-pyridazinyl]-4-(2-carboxy-ethyl)-1,2,3-triazol
(54) 1-[6-(4-Amidino-phenyl)-3-pyridazinyl]-4-(2-carboxy-ethyl)-1,2,3-triazol
(55) 1-[6-(4-Amidino-2-methyl-phenyl)-3-pyridazinyl]-4-(2-carboxy-ethyl)-imidazol
(56) 1-[6-(4-Aminomethyl-phenyl)-3-pyridazinyl]-4-(2-carboxy-ethyl)-imidazol
(57) 2-(4-Amidino-phenyl)-4-[4-(2-carboxy-ethyl)-phenyl]-thiazol
(58) 2-(4-Amidino-phenyl)-4-[4-(2-carboxy-ethyl)-phenyl]-1-methyl-imidazol
(59) 5-(4-Amidino-phenyl)-2-[4-(2-carboxy-ethyl)-phenyl]-thiazol
(60) 2-(4-Amidino-phenyl)-4-[4-(2-carboxy-ethyl)-phenyl]-imidazol
(61) 1-(4-Amidino-phenyl)-3-[4-(2-carboxy-ethyl)-phenyl]-5-methyl-1,2,4-triazol
(62) 3-(4-Amidino-phenyl)-5-[4-(2-carboxy-ethyl)-phenyl]-pyrazol
(63) 5-(4-Amidino-phenyl)-3-[4-(2-carboxy-ethyl)-phenyl]-1-methyl-pyrazol
(64) 3-(4-Amidino-phenyl)-5-[4-(2-carboxy-ethyl)-phenyl]-1-phenyl-pyrazol

(65) 1-(4-Amidino-phenyl)-4-[4-(2-carboxy-ethyl)-phenyl]-2-methyl-imidazol
(66) 1-(4-Amidino-phenyl)-4-[4-(2-carboxy-ethyl)-phenyl]-2-phenyl-imidazol
(67) 4-(4-Amidino-phenyl)-1-[4-(2-carboxy-ethyl)-phenyl]-imidazol
(68) 1-(4-Amidino-phenyl)-3-[4-(2-carboxy-ethyl)-phenyl]-2H-pyrazol-5-on
(69) 1-(4-Amidino-phenyl)-3-[4-(2-carboxy-ethyl)-phenyl]-2-methyl-2H-pyrazol-5-on
(70) 3-(4-Amidino-phenyl)-1-[4-(2-carboxy-ethyl)-phenyl]-2H-pyrazol-5-on
(71) 3-(4-Amidino-phenyl)-1-[4-(2-carboxy-ethyl)-phenyl]-2-methyl-2H-pyrazol-5-on
(72) 1-(4-Amidino-phenyl)-3-[4-(2-carboxy-ethyl)-phenyl]-1,2,4-triazol
(73) 3-(4-Amidino-phenyl)-1-[4-(2-carboxy-ethyl)-phenyl]-1,2,4-triazol
(74) 4-(4-Amidino-phenyl)-2-[4-(2-carboxy-ethyl)-phenyl]-oxazol
(75) 4-(4-Amidino-phenyl)-2-[4-(2-carboxy-ethyl)-phenyl]-1-phenyl-imidazol
(76) 1-(4-Amidino-phenyl)-3-[4-(2-carboxy-ethyl)-phenyl]-pyrazol
(77) 1-(4-Amidino-phenyl)-3-[4-(2-carboxy-ethyl)-phenyl]-5-methyl-pyrazol
(78) 3-(4-Amidino-phenyl)-1-[4-(2-carboxy-ethyl)-phenyl]-pyrazol
(79) 3-(4-Amidino-phenyl)-1-[4-(2-carboxy-ethyl)-phenyl]-5-methyl-pyrazol
(80) 2-(4-Amidino-phenyl)-5-[4-(2-carboxy-ethyl)-phenyl]-furan
(81) 2-(4-Amidino-phenyl)-5-[4-(2-carboxy-ethyl)-phenyl]-tetrahydrofuran
(82) 3-(4-Amidino-phenyl)-5-[4-(2-carboxy-ethyl)-phenyl]-isoxazol
(83) 4-(4-Amidino-phenyl)-2-[4-(2-carboxy-ethyl)-phenyl]-5-methyl-imidazol
(84) 1-(4-Amidino-2-fluor-phenyl)-3-[4-(2-carboxy-ethyl)-phenyl]-2H-pyrazol-5-on
(85) 2-(4-Amidino-phenyl)-5-[4-(2-carboxy-ethyl)-phenyl]-thiophen
(86) 3-(4-Amidino-phenyl)-4-carboxy-5-[4-(2-carboxy-ethyl)-phenyl]-pyrazol
(87) 3-(4-Amidino-phenyl)-4-aminocarbonyl-5-[4-(2-carboxy-ethyl)-phenyl]-pyrazol
(88) 2-[4-(2-Carboxy-ethyl)-phenyl]-4-(3-guanidino-phenyl)-imidazol
(89) 1-(4-Amidino-3'-methylthio-4'-biphenylyl)-4-(2-carboxy-ethyl)-imidazol
(90) 1-(4-Amidino-3'-methylsulfinyl-4'-biphenylyl)-4-(2-carboxy-ethyl)-imidazol
(91) 1-(4-Amidino-3'-methylsulfonyl-4'-biphenylyl)-4-(2-carboxy-ethyl)-imidazol
(92) 1-[5-(4-Amidino-phenyl)-2-pyridyl]-4-(2-carboxy-ethyl)-imidazol
(93) 4-(4-Amidino-phenyl)-2-[4-(2-carboxy-ethyl)-1-piperidinyl]-imidazol
(94) 4-(4-Amidino-phenyl)-2-[4-(2-carboxy-vinyl)-phenyl]-imidazol
(95) 1-[6-(4-Amidino-phenyl)-5-methyl-3-pyridazinyl]-4-(2-carboxy-ethyl)-imidazol
(96) 4-(2-Carboxy-ethyl)-1-[6-(4-methylamidino-phenyl)-3-pyridazinyl]-imidazol
(97) 1-[6-(4-n-Butylamidino-phenyl)-3-pyridazinyl]-4-(2-carboxy-ethyl)-imidazol
(98) 3-(4-Amidino-4'-biphenylyl)-5-(2-carboxy-ethyl)-pyrazol
(99) 1-[6-(4-Amidino-2-fluor-phenyl)-3-pyridazinyl]-4-(2-carboxy-ethyl)-imidazol
(100) 1-[6-(4-Amidino-2-methoxy-phenyl)-3-pyridazinyl]-4-(2-carboxy-ethyl)-imidazol
(101) 4-(4-Amidino-phenyl)-2-(4-carboxymethyloxy-phenyl)-imidazol
(102) 4-(4-Amidino-phenyl)-2-(4-carboxymethylthio-phenyl)-imidazol
(103) 4-(4-Amidino-phenyl)-2-(4-carboxymethylsulfinyl-phenyl)-imidazol
(104) 4-(4-Amidino-phenyl)-2-(4-carboxymethylsulfonyl-phenyl)-imidazol
(105) 4-(4-Amidino-phenyl)-2-(4-carboxymethylamino-phenyl)-imidazol
(106) 2-[4-(N-Acetyl-N-carboxymethyl-amino)-phenyl]-4-(4-amidino-phenyl)-imidazol
(107) 2-[4-(N-Acetyl-N-carboxymethyl-amino)-3-brom-phenyl]-4-(4-amidino-phenyl)-imidazol
(108) 2-[4-(N-Acetyl-N-carboxymethyl-amino)-3-fluor-phenyl]-4-(4-amidino-phenyl)-imidazol
(109) 4-(4-Amidino-phenyl)-2-(4-carboxymethyloxy-3-methyl-phenyl)-imidazol
(110) 4-(4-Amidino-phenyl)-2-[4-(2-carboxy-ethyl)-3-nitro-phenyl]-imidazol
(111) 4-(4-Amidino-phenyl)-2-[3-amino-4-(2-carboxy-ethyl)-phenyl]-imidazol
(112) 2-[3-Acetylamino-4-(2-carboxy-ethyl)-phenyl]-4-(4-amidino-phenyl)-imidazol
(113) 4-(4-Amidino-phenyl)-2-[3-benzoylamino-4-(2-carboxy-ethyl)-phenyl]-imidazol
(114) 4-(4-Amidino-phenyl)-2-[4-(2-carboxy-ethyl)-3-methansulfonylamino-phenyl]-imidazol
(115) 4-(4-Amidino-phenyl)-2-[4-(2-carboxy-ethyl)-3-hydroxy-phenyl]-imidazol
(116) 4-(4-Amidino-phenyl)-2-[4-(2-carboxy-ethyl)-3-methoxy-phenyl)-imidazol
(117) 4-(4-Amidino-phenyl)-2-(4-carboxymethyloxy-3-methylthio-phenyl)-imidazol
(118) 4-(4-Amidino-phenyl)-2-(4-carboxymethyloxy-3-methyl-sulfinyl-phenyl)-imidazol
(119) 4-(4-Amidino-phenyl)-2-(4-carboxymethyloxy-3-methyl-sulfonyl-phenyl)-imidazol
(120) 4-(4-Amidino-phenyl)-2-[4-(N-carboxymethyl-methyl-amino)-phenyl]-imidazol
(121) 4-(4-Amidino-phenyl)-2-[1-(2-carboxy-ethyl)-2-oxo-4-pyridyl]-imidazol
(122) 4-(4-Amidino-phenyl)-2-[2-(2-carboxy-ethyl)-5-pyridyl]-imidazol

(123) 4-(4-Amidino-phenyl)-1-[6-(2-carboxy-ethyl)-3-pyridazinyl]-imidazol
(124) 4-(4-Amidino-phenyl)-2-[4-(2-carboxy-ethyl)-cyclo-hexyl]-imidazol
(125) 4-(4-Amidino-4'-biphenylyl)-2-(2-carboxy-ethyl)-imidazol
(126) 4-(4-Amidino-4'-biphenylyl)-2-(2-carboxy-ethyl)-1-methyl-imidazol
(127) 2-(4-Amidino-4'-biphenyl)-5-(2-carboxy-ethyl)-3-methoxycarbonyl-furan
Man läßt 60 Minuten bei 5° C reagieren.
Schmelzpunkt: 251-252° C (Zers., sintert ab 204° C)
$R_f$-Wert: 0,34 (Kieselgel; Methylenchlorid/Methanol/Eisessig = 8:2:0,1)
(128) 2-(4-Amidino-4'-biphenyl)-3-carboxy-5-(2-carboxy-ethyl)-furan
Man verwendet einen viermolaren Überschuß an Lithiumhydroxid und läßt 6,5 Stunden bei Raumtemperatur reagieren.
Schmelzpunkt: 298° C (Zers., sintert ab 184° C)
$R_f$-Wert: 0,45 (Kieselgel; Methylenchlorid/Methanol/Eisessig = 3:1:0,1)
(129) 4-[4-(1-Amino-cyclopropyl)-phenyl]-2-[4-(2-carboxy-ethyl)-phenyl]-1-methyl-imidazol
(130) 4-[4-(1-Amino-cyclopentyl)-phenyl]-2-[4-(2-carboxy-ethyl)-phenyl]-1-methyl-imidazol
(131) 4-(4-Amidino-phenyl)-2-[4-(1,3-bis-carboxy-2-propyl)-phenyl]-1-methyl-imidazol
(132) 4-(4-Amidino-phenyl)-2-(3,4-dicarboxymethyloxy-phenyl)-1-methyl-imidazol
(133) 2-(4-Amino-cyclohexyl)-4-[4-(2-carboxy-ethyl)-phenyl]-imidazol
(134) 4-(2-Amino-2-carboxy-ethyl)-1-[6-(4-aminomethyl-phenyl)-3-pyridazinyl]-imidazol-dihydrochlorid
Man verseift das Amid mit 1N Natronlauge bei 100° C.
$R_f$-Wert: 0,32 (Kieselgel; Methylenchlorid/Methanol/konz. Ammoniak = 2:1:0,25)
(135) 1-[6-(4-Amidino-phenyl)-3-pyridazinyl]-4-(2-carboxy-ethenyl)-imidazol
(136) 4-(4-Amidino-phenyl)-2-[4-(2-carboxy-2-methyl-propyl)-phenyl]-1-methyl-imidazol
(137) 4-(4-Aminomethyl-phenyl)-2-[4-(2-carboxy-ethyl)-phenyl]-1-methyl-imidazol
(138) 4-[4-(2-Amino-ethyl)-phenyl]-2-[4-(2-carboxy-ethyl)-phenyl]-1-methyl-imidazol
(139) 4-[4-(1-Amino-ethyl)-phenyl]-2-[4-(2-carboxy-ethyl)-phenyl]-1-methyl-imidazol
(140) 4-[4-(2-Amino-2-propyl)-phenyl]-2-[4-(2-carboxy-ethyl)-phenyl]-1-methyl-imidazol
(141) 4-(1-Amino-5-indanyl)-2-[4-(2-carboxy-ethyl)-phenyl]-1-methyl-imidazol
(142) 4-(1-Amino-1,2,3,4-tetrahydro-6-naphthyl)-2-[4-(2-carboxy-ethyl)-phenyl]-1-methyl-imidazol
(143) 1-[6-(4-Amidino-phenyl)-3-pyridazinyl]-4-(2-carboxy-propyl)-imidazol
(144) 4-(2-Amino-2-carboxy-ethyl)-1-[4-(4-aminomethyl-piperidino)-phenyl]-imidazol
(145) 4-(2-Amino-2-carboxy-ethyl)-1-[4-(4-aminomethyl-2-oxo-piperidino)-phenyl]-imidazol
(146) 3-(4-Amidino-phenyl)-1-[4-(2-amino-2-carboxy-ethyl)-phenyl]-2H-pyrazol-5-on
(147) 2-[4-(2-Carboxy-ethyl)-phenyl]-1-methyl-4-(4-methyl-aminomethyl-phenyl)-imidazol
(148) 2-[4-(2-Carboxy-ethyl)-phenyl]-4-[4-(dimethylamino-methyl)-phenyl]-1-methyl-imidazol
(149) 4-(4-Amino-cyclohexyl)-2-[4-[(2-carboxy-ethyl)-amino-carbonyl]-phenyl]-1-methyl-imidazol
(150) 2-[4-[(2-Carboxy-ethyl)-aminocarbonyl]-phenyl]-1-methyl-4-(4-piperidinyl)-imidazol
(151) 2-[4-[(2-Carboxy-ethyl)-aminocarbonyl]-phenyl]-1-methyl-4-(1-methyl-4-piperidinyl)-imidazol
(152) 1-[6-(4-Amidino-phenyl)-3-pyridazinyl]-4-(2-carboxy-2-methoxy-ethyl)-imidazol
(153) 4-(4-Amidino-phenyl)-2-[(4-(2-carboxy-ethyl)-phenyl]-1-(2-phenyl-ethyl)-imidazol

Beispiel 2

1-[6-(4-Amidino-phenyl)-3-pyridazinyl]-4-(2-methoxycarbonyl-ethyl)-imidazol

Eine Mischung aus 1,1 g 1-[6-(4-Cyan-phenyl)-3-pyridazinyl]-4-(2-methoxycarbonyl-ethyl)-imidazol, 1500 ml absolutem Methanol und 50 ml Methylenchlorid wird unter Rühren und Eiskühlung mit trockenem Chlorwasserstoff gesättigt. Man rührt weitere 16 Stunden bei Raumtemperatur und destilliert das Lösungsmittel im Vakuum ab. Der Rückstand wird in 250 ml absolutem Methanol aufgenommen und mit 8 g Ammoniumcarbonat versetzt. Man rührt 30 Minuten bei Raumtemperatur, saugt den Niederschlag ab und dampft das Filtrat im Vakuum ein. Der Eindampfrückstand wird mit dem vorher gewonnenen Niederschlag vereinigt und säulenchromatographisch gereinigt (Elutionsmittel: Methylenchlorid/Methanol/konz. Ammoniak = 2:1:0,25).
Ausbeute: 0,36 g (31 % der Theorie),
$R_f$-Wert: 0,22 (Kieselgel; Methylenchlorid/Methanol/konz. Ammoniak = 2:1:0,25)
Analog werden folgende Verbindungen erhalten:
(1) 1-[6-(4-Amidino-phenyl)-3-pyridazinyl]-4-(2-hydroxy-2-methoxycarbonyl-ethyl)-imidazol
$R_f$-Wert: 0,17 (Kieselgel; Methylenchlorid/Methanol/konz. Ammoniak = 2:1:0,25)

(2) 1-[6-(4-Amidino-phenyl)-3-pyridazinyl]-4-(2-amino-2-methoxycarbonyl-ethyl)-imidazol-tris-trifluoracetat
Als Ausgangsprodukt dient 1-[6-(4-Cyan-phenyl)-3-pyridazinyl]-4-(2-tert.butyloxycarbonylamino-2-methoxycarbonyl-ethyl)-imidazol
Die rohe freie Base wird durch Aufnehmen in Methylenchlorid, Versetzen mit Trifluoressigsäure, Einengen und Reinigen über Kieselgel (Elutionsmittel: Methylenchlorid/Methanol/konz. Ammoniak = 2:1:0,25) in das Tris-trifluoracetat überführt.
$R_f$-Wert: 0,18 (Kieselgel; Methylenchlorid/Methanol/konz. Ammoniak = 2:1:0,25)
(3) 5-(4-Amidino-phenyl)-2-[4-(2-methoxycarbonyl-ethyl)-phenyl]-tetrazol-hydrochlorid
$R_f$-Wert: 0,41 (Kieselgel; Methylenchlorid/Methanol/konz. Ammoniak = 4:1:0,25)

| | | | | |
|---|---|---|---|---|
| Ber. x HCl: | C 55,89 | H 4,95 | N 21,73 | Cl 9,16 |
| Gef.: | 55,33 | 4,95 | 21,47 | 9,51 |

(4) 5-(4-Amidino-4'-biphenylyl)-2-(2-methoxycarbonyl-ethyl)-tetrazol-hydrochlorid
$R_f$-Wert: 0,31 (Kieselgel; Methylenchlorid/Methanol/konz. Ammoniak = 4:1:0,25)
(5) 4-(4-Amidino-phenyl)-2-[4-(2-methoxycarbonyl-ethyl)-phenyl]-thiazol-hydrochlorid
Schmelzpunkt: 235-238 °C
$R_f$-Wert: 0,23 (Reversed-Phase-Platte RP8; 5%ige Natriumchloridlösung/Methanol = 4:6)
(6) 4-(4-Amidino-phenyl)-2-[4-(2-methoxycarbonyl-ethyl)-phenyl]-1-methyl-imidazol-hydrochlorid
Schmelzpunkt: 228-230 °C (Zers.)
$R_f$-Wert: 0,50 (Kieselgel; Methylenchlorid/Methanol = 4:1)
(7) 4-(4-Amidino-phenyl)-2-[4-(2-methoxycarbonyl-ethyl)-phenyl]-imidazol-hydrochlorid
$R_f$-Wert: 0,60 (Reversed-Phase-Platte RP8; 5%ige Natriumchloridlösung/Methanol = 4:6)

| Ber. x 1,1 HCl x 0,5 $H_2O$: | | | | |
|---|---|---|---|---|
| | C 60,43 | H 5,60 | N 14,09 | Cl 9,81 |
| Gef.: | 60,67 | 5,41 | 13,84 | 9,76 |

(8) 3-(4-Amidino-phenyl)-5-[4-(2-methoxycarbonyl-ethyl)-phenyl]-1,2,4-triazol
$R_f$-Wert: 0,11 (Kieselgel; Methylenchlorid/Methanol = 8,5:1,5)
(9) 2-(4-Amidino-phenyl)-5-[4-(2-methoxycarbonyl-ethyl)-phenyl]-1,3,4-oxadiazol
$R_f$-Wert: 0,37 (Kieselgel; Methylenchlorid/Methanol = 8:2)
(10) 2-(4-Amidino-phenyl)-5-[4-(2-methoxycarbonyl-ethyl)-phenyl]-1,3,4-thiadiazol
$R_f$-Wert: 0,45 (Kieselgel; Methylenchlorid/Methanol = 7:3)
(11) 1-[5-(4-Amidino-phenyl)-2-pyrimidyl]-4-(2-amino-2-methoxycarbonyl-ethyl)-imidazol
(12) 1-[5-(4-Amidino-phenyl)-4-methyl-2-pyrimidyl]-4-(2-amino-2-methoxycarbonyl-ethyl)-imidazol
(13) 1-[5-(4-Amidino-phenyl)-2-pyrazinyl]-4-(2-amino-2-methoxycarbonyl-ethyl)-imidazol
(14) 1-[5-(4-Amidino-phenyl)-4-methyl-2-pyrimidyl]-4-(2-methoxycarbonyl-ethyl)-imidazol
(15) 1-[5-(4-Amidino-phenyl)-2-pyrimidyl]-4-(2-methoxycarbonyl-ethyl)-imidazol
(16) 1-(4-Amidino-4'-biphenylyl)-4-(2-methoxycarbonyl-ethyl)-imidazol
(17) 4-(4-Amidino-4'-biphenylyl)-2-(2-methoxycarbonyl-ethyl)-thiazol
(18) 5-(4-Amidino-4'-biphenylyl)-2-(2-methoxycarbonyl-ethyl)-oxazol
(19) 2-(4-Amidino-4'-biphenylyl)-5-(2-methoxycarbonyl-ethyl)-1,3,4-oxadiazol
(20) 2-(4-Amidino-4'-biphenylyl)-5-(2-methoxycarbonyl-ethyl)-1,3,4-thiadiazol $R_f$-Wert: 0,09 (Kieselgel; Methylenchlorid/Methanol = 9:1)
(21) 2-(4-Amidino-4'-biphenylyl)-4-(2-methoxycarbonyl-ethyl)-thiazol
(22) 2-(4-Amidino-4'-biphenylyl)-5-(2-methoxycarbonyl-ethyl)-thiophen
(23) 2-(4-Amidino-4'-biphenylyl)-5-(2-methoxycarbonyl-ethyl)-furan
(24) 1-(4-Amidino-3'-brom-4'-biphenylyl)-4-(2-methoxycarbonyl-ethyl)-imidazol
(25) 1-[6-(4-Amidino-phenyl)-3-pyridazinyl]-4-(2-methoxycarbonyl-ethyl)-2-methyl-imidazol
(26) 1-[6-(4-Amidino-phenyl)-3-pyridazinyl]-2-isopropyl-4-(2-methoxycarbonyl-ethyl)-imidazol
(27) 1-[6-(4-Amidino-phenyl)-3-pyridazinyl]-2-hexyl-4-(2-methoxycarbonyl-ethyl)-imidazol
(28) 1-[6-(4-Amidino-phenyl)-3-pyridazinyl]-4-(2-methoxycarbonyl-ethyl)-2-(2-phenyl-ethyl)-imidazol
(29) 1-[6-(4-Amidino-phenyl)-3-pyridazinyl]-4-(2-methoxycarbonyl-ethyl)-2-phenyl-imidazol
(30) 1-[6-(4-Amidino-phenyl)-3-pyridazinyl]-4-(2-methoxycarbonyl-ethyl)-2-(3-pyridyl)-imidazol
(31) 3-(4-Amidino-4'-biphenylyl)-5-(2-methoxycarbonyl-ethyl)-1,2,4-trizaol

(32) 1-[6-(4-Amidino-phenyl)-3-pyridazinyl]-4-(3-methoxycarbonyl-propyl)-imidazol
(33) 2-(4-Amidino-3'-methoxy-4'-biphenylyl)-5-(2-methoxycarbonyl-ethyl)-1,3,4-thiadiazol
(34) 2-(4-Amidino-3'-methyl-4'-biphenylyl)-5-(2-methoxycarbonyl-ethyl)-1,3,4-thiadiazol
(35) 1-[6-(4-Amidino-phenyl)-3-pyridazinyl]-4-(2-dibenzyl-amino-2-methoxycarbonyl-ethyl)-imidazol
$R_f$-Wert: 0,16 (Kieselgel; Methylenchlorid/Methanol/konz. Ammoniak = 4:1:0,25)
(36) 1-[6-(4-Amidino-phenyl)-3-pyridazinyl]-2-(2-dimethyl-amino-2-methoxycarbonyl-ethyl)-imidazol
(37) 1-[6-(4-Amidino-phenyl)-3-pyridazinyl]-3-(2-methoxycarbonyl-ethyl)-indol
Schmelzpunkt: sintert ab 160 ° C
$R_f$-Wert: 0,17 (Kieselgel; Methylenchlorid/Methanol/konz. Ammoniak = 4:1:0,25)
(38) 4-(4-Amidino-4'-biphenylyl)-2-(2-methoxycarbonyl-ethyl)-5-methyl-thiazol
(39) 3-(4-Amidino-4'-biphenylyl)-5-methoxycarbonylmethyl-thio-1,2,4-triazol Schmelzpunkt: 210 ° C (sintert ab 180 ° C)
(40) 4-(4-Amidino-4'-biphenylyl)-2-methoxycarbonylmethyl-amino-thiazol
(41) 2-[1-(4-Amidino-phenyl)-4-piperidinyl]-4-(2-methoxycarbonyl-ethyl)-thiazol
(42) 2-[4-(4-Amidino-phenyl)-1-piperazinyl]-4-(2-methoxycarbonyl-ethyl)-thiazol
(43) 2-[1-(5-Amidino-2-pyridyl)-4-piperidinyl]-4-(2-methoxycarbonyl-ethyl)-thiazol
(44) 1-[4-(5-Amidino-2-pyrimidyl)-phenyl]-4-(2-methoxycarbonyl-ethyl)-imidazol
(45) 1-[4-(5-Amidino-2-pyrazinyl)-phenyl]-4-(2-methoxycarbonyl-ethyl)-imidazol
(46) 1-[4-(4-Amidino-phenyl)-cyclohexyl]-4-(2-methoxycarbonyl-ethyl)-imidazol
(47) 1-[6-(4-Amidino-phenyl)-3-pyridazinyl]-3-(2,2-bis-methoxycarbonyl-ethyl)-indol
(48) 2-[1-(4-Amidino-phenyl)-4-piperidinyl]-5-(2-methoxycarbonyl-ethyl)-1,3,4-oxadiazol
(49) 2-[1-(5-Amidino-2-pyridyl)-4-piperidinyl]-5-(2-methoxycarbonyl-ethyl)-1,3,4-oxadiazol
(50) 5-[1-(4-Amidino-phenyl)-4-piperidinyl]-2-(2-methoxycarbonyl-ethyl)-tetrazol
(51) 5-[1-(5-Amidino-2-pyridyl)-4-piperidinyl]-2-(2-methoxycarbonyl-ethyl)-tetrazol
(52) 2-[6-(4-Amidino-phenyl)-3-pyridazinyl]-4-(2-methoxycarbonyl-ethyl)-1,2,3-triazol
(53) 1-[6-(4-Amidino-phenyl)-3-pyridazinyl]-4-(2-methoxycarbonyl-ethyl)-1,2,3-triazol
(54) 1-[6-(4-Amidino-2-methyl-phenyl)-3-pyridazinyl]-4-(2-methoxycarbonyl-ethyl)-imidazol
(55) 2-(4-Amidino-phenyl)-4-[4-(2-methoxycarbonyl-ethyl)-phenyl]-thiazol
(56) 2-(4-Amidino-phenyl)-4-[4-(2-methoxycarbonyl-ethyl)-phenyl]-1-methyl-imidazol
(57) 5-(4-Amidino-phenyl)-2-[4-(2-methoxycarbonyl-ethyl)-phenyl]-thiazol
(58) 2-(4-Amidino-phenyl)-4-[4-(2-methoxycarbonyl-ethyl)-phenyl]-imidazol
(59) 1-(4-Amidino-phenyl)-3-[4-(2-methoxycarbonyl-ethyl)-phenyl]-5-methyl-1,2,4-triazol
(60) 3-(4-Amidino-phenyl)-5-[4-(2-methoxycarbonyl-ethyl)-phenyl]-pyrazol
(61) 5-(4-Amidino-phenyl)-3-[4-(2-methoxycarbonyl-ethyl)-phenyl]-1-methyl-pyrazol
(62) 3-(4-Amidino-phenyl)-5-[4-(2-methoxycarbonyl-ethyl)-phenyl]-1-phenyl-pyrazol
(63) 1-(4-Amidino-phenyl)-4-[4-(2-methoxycarbonyl-ethyl)-phenyl]-2-methyl-imidazol
(64) 1-(4-Amidino-phenyl)-4-[4-(2-methoxycarbonyl-ethyl)-phenyl]-2-phenyl-imidazol
(65) 4-(4-Amidino-phenyl)-1-[4-(2-methoxycarbonyl-ethyl)-phenyl]-imidazol
(66) 1-(4-Amidino-phenyl)-3-[4-(2-methoxycarbonyl-ethyl)-phenyl]-2H-pyrazol-5-on
(67) 1-(4-Amidino-phenyl)-3-[4-(2-methoxycarbonyl-ethyl)-phenyl]-2-methyl-2H-pyrazol-5-on
(68) 3-(4-Amidino-phenyl)-1-[4-(2-methoxycarbonyl-ethyl)-phenyl]-2H-pyrazol-5-on
(69) 3-(4-Amidino-phenyl)-1-[4-(2-methoxycarbonyl-ethyl)-phenyl]-2-methyl-2H-pyrazol-5-on
(70) 1-(4-Amidino-phenyl)-3-[4-(2-methoxycarbonyl-ethyl)-phenyl]-1,2,4-triazol
(71) 3-(4-Amidino-phenyl)-1-[4-(2-methoxycarbonyl-ethyl)-phenyl]-1,2,4-triazol
(72) 4-(4-Amidino-phenyl)-2-[4-(2-methoxycarbonyl-ethyl)-phenyl]-oxazol
(73) 4-(4-Amidino-phenyl)-2-[4-(2-methoxycarbonyl-ethyl)-phenyl]-1-phenyl-imidazol
(74) 1-(4-Amidino-phenyl)-3-[4-(2-methoxycarbonyl-ethyl)-phenyl]-pyrazol
(75) 1-(4-Amidino-phenyl)-3-[4-(2-methoxycarbonyl-ethyl)-phenyl]-5-methyl-pyrazol
(76) 3-(4-Amidino-phenyl)-1-[4-(2-methoxycarbonyl-ethyl)-phenyl]-pyrazol
(77) 3-(4-Amidino-phenyl)-1-[4-(2-methoxycarbonyl-ethyl)-phenyl]-5-methyl-pyrazol
(78) 2-(4-Amidino-phenyl)-5-[4-(2-methoxycarbonyl-ethyl)-phenyl]-furan
(79) 2-(4-Amidino-phenyl)-5-[4-(2-methoxycarbonyl-ethyl)-phenyl]-tetrahydrofuran
(80) 3-(4-Amidino-phenyl)-5-[4-(2-methoxycarbonyl-ethyl)-phenyl]-isoxazol
(81) 4-(4-Amidino-phenyl)-2-[4-(2-methoxycarbonyl-ethyl)-phenyl]-5-methyl-imidazol
(82) 1-(4-Amidino-2-fluor-phenyl)-3-[4-(2-methoxycarbonyl-ethyl)-phenyl]-2H-pyrazol-5-on
(83) 2-(4-Amidino-phenyl)-5-[4-(2-methoxycarbonyl-ethyl)-phenyl]-thiophen
(84) 3-(4-Amidino-phenyl)-4-methoxycarbonyl-5-[4-(2-methoxycarbonyl-ethyl)-phenyl]-pyrazol
(85) 3-(4-Amidino-phenyl)-4-aminocarbonyl-5-[4-(2-methoxycarbonyl-ethyl)-phenyl]-pyrazol

(86) 1-[6-(4-Amidino-phenyl)-3-pyridazinyl]-4-[2-(5-tetrazolyl)-ethyl]-imidazol
(87) 1-[6-(4-Amidino-phenyl)-3-pyridazinyl]-4-(2-phosphono-ethyl)-imidazol
(88) 1-[6-(4-Amidino-phenyl)-3-pyridazinyl]-4-[2-(O-ethyl-phosphono)-ethyl]-imidazol
(89) 1-[6-(4-Amidino-phenyl)-3-pyridazinyl]-4-(2-sulfo-ethyl)-imidazol
(90) 1-[6-(4-Amidino-phenyl)-3-pyridazinyl]-4-(2-ethoxycarbonyl-ethyl)-imidazol Man arbeitet mit ethanolischer Salzsäure.
(91) 1-(4-Amidino-3'-methylthio-4'-biphenylyl)-4-(2-methoxycarbonyl-ethyl)-imidazol
(92) 1-(4-Amidino-3'-methylsulfonyl-4'-biphenylyl)-4-(2-methoxycarbonyl-ethyl)-imidazol
(93) 1-(5-(4-Amidino-phenyl)-2-pyridyl]-4-(2-methoxycarbonyl-ethyl)-imidazol
(94) 4-(4-Amidino-phenyl)-2-[4-(2-methoxycarbonyl-ethyl)-1-piperidinyl]-imidazol
(95) 4-(4-Amidino-phenyl)-2-[4-(2-methoxycarbonyl-vinyl)-phenyl]-imidazol
(96) 1-(6-(4-Amidino-phenyl)-5-methyl-3-pyridazinyl]-4-(2-methoxycarbonyl-ethyl]-imidazol
(97) 4-(2-Methoxycarbonyl-ethyl)-1-[6-(4-methylamidino-phenyl)-3-pyridazinyl]-imidazol
Der Iminoester wird in absolutem Methanol aufgenommen und mit einem 20-fachen Überschuß einer methanolischen Methylaminlösung umgesetzt.
(98) 1-[6-(4-n-Butylamidino-phenyl)-3-pyridazinyl]-4-(2-methoxycarbonyl-ethyl)-imidazol Herstellung analog (97) mit n-Butylamin.
(99) 3-(4-Amidino-4'-biphenylyl)-5-(2-methoxycarbonyl-ethyl)-pyrazol
(100) 1-[6-(4-Amidino-2-fluor-phenyl)-3-pyridazinyl]-4-(2-methoxycarbonyl-ethyl)-imidazol
(101) 1-[6-(4-Amidino-2-methoxy-phenyl)-3-pyridazinyl]-4-(2-methoxycarbonyl-ethyl)-imidazol
(102) 4-(4-Amidino-phenyl)-2-(4-methoxycarbonyloxy-phenyl)-imidazol
(103) 4-(4-Amidino-phenyl)-2-(4-methoxycarbonylmethylthio-phenyl)-imidazol
(104) 4-(4-Amidino-phenyl)-2-(4-methoxycarbonylmethylsulfonyl-phenyl)-imidazol
(105) 4-(4-Amidino-phenyl)-2-(4-methoxycarbonylmethylamino-phenyl)-imidazol
(106) 2-[4-(N-Acetyl-N-methoxycarbonylmethyl-amino)-phenyl]-4-(4-amidino-phenyl)-imidazol
(107) 2-[4-(N-Acetyl-N-methoxycarbonylmethyl-amino)-3-brom-phenyl]-4-(4-amidino-phenyl)-imidazol
(108) 2-[4-(N-Acetyl-N-methoxycarbonylmethyl-amino)-3-fluor-phenyl]-4-(4-amidino-phenyl)-imidazol
(109) 4-(4-Amidino-phenyl)-2-(4-methoxycarbonylmethyloxy-3-methyl-phenyl)-imidazol
(110) 4-(4-Amidino-phenyl)-2-[4-(2-methoxycarbonyl-ethyl)-3-nitro-phenyl]-imidazol
(111) 4-(4-Amidino-phenyl)-2-[3-amino-4-(2-methoxycarbonyl-ethyl)-phenyl]-imidazol
(112) 2-[3-Acetylamino-4-(2-methoxycarbonyl-ethyl)-phenyl]-4-(4-amidino-phenyl)-imidazol
(113) 4-(4-Amidino-phenyl)-2-[3-benzoylamino-4-(2-methoxycarbonyl-ethyl)-phenyl]-imidazol
(114) 4-(4-Amidino-phenyl)-2-[3-methansulfonylamino-4-(2-methoxycarbonyl-ethyl)-phenyl]-imidazol
(115) 4-(4-Amidino-phenyl)-2-[3-hydroxy-4-(2-methoxycarbonyl-ethyl)-phenyl]-imidazol
(116) 4-(4-Amidino-phenyl)-2-[3-methoxy-4-(2-methoxycarbonyl-ethyl)-phenyl]-imidazol
(117) 4-(4-Amidino-phenyl)-2-(4-methoxycarbonylmethyloxy-3-methylthio-phenyl)-imidazol
(118) 4-(4-Amidino-phenyl)-2-(4-methoxycarbonylmethyloxy-3-methylsulfonyl-phenyl)-imidazol
(119) 4-(4-Amidino-phenyl)-2-[4-(N-methoxycarbonylmethyl-methylamino)-phenyl]-imidazol
(120) 4-(4-Amidino-phenyl)-2-[1-(2-methoxycarbonyl-ethyl)-2-oxo-4-pyridyl]-imidazol
(121) 4-(4-Amidino-phenyl)-2-[2-(2-methoxycarbonyl-ethyl)-5-pyridyl]-imidazol
(122) 4-(4-Amidino-phenyl)-1-[6-(2-methoxycarbonyl-ethyl)-3-pyridazinyl]-imidazol
(123) 4-(4-Amidino-phenyl)-2-[4-(2-methoxycarbonyl-ethyl)-cyclohexyl]-imidazol
(124) 4-(4-Amidino-4'-biphenylyl)-2-(2-methoxycarbonyl-ethyl)-1-methyl-imidazol
(125) 4-(4-Amidino-4'-biphenylyl)-2-(2-methoxycarbonyl-ethyl)-imidazol
(126) 2-(4-Amidino-4'-biphenylyl)-3-methoxycarbonyl-5-(2-methoxycarbonyl-ethyl)-furan-hydrochlorid $R_f$-Wert: 0,52 (Kieselgel; Methylenchlorid/Methanol = 8:2)
(127) 4-(4-Amidino-phenyl)-2-[4-(1,3-dimethoxycarbonyl-2-propyl)-phenyl]-1-methyl-imidazol
(128) 4-(4-Amidino-phenyl)-2-[3,4-bis(methoxycarbonylmethyloxy)-phenyl]-1-methyl-imidazol
(129) 1-[6-(4-Amidino-phenyl)-3-pyridazinyl]-4-(2-methoxycarbonyl-ethenyl)-imidazol
(130) 4-(4-Amidino-phenyl)-2-[4-(2-methoxycarbonyl-2-methyl-propyl)-phenyl]-1-methyl-imidazol
(131) 1-[6-(4-Amidino-phenyl)-3-pyridazinyl]-4-(2-methoxycarbonyl-propyl)-imidazol
(132) 3-(4-Amidino-phenyl)-1-[4-(2-amino-2-methoxycarbonyl-ethyl)-phenyl]-2H-pyrazol-5-on
(133) 1-[6-(4-Amidino-phenyl)-3-pyridazinyl]-4-(2-methoxy-2-methoxycarbonyl-ethyl)-imidazol
(134) 4-(4-Amidino-phenyl)-2-[4-(2-methoxycarbonyl-ethyl)-phenyl]-1-(2-phenyl-ethyl)-imidazol

## Beispiel 3

1-[6-(4-Cyan-phenyl)-3-pyridazinyl]-4-(2-methoxycarbonyl-ethyl)-imidazol

Eine Mischung aus 2,15 g 3-Chlor-6-(4-cyan-phenyl)-pyridazin, 2,1 g 3-(4-Imidazolyl)-propionsäuremethylester, 2 g Kalium-carbonat und 4 ml Dimethylsulfoxid wird unter Argon 3 Stunden bei 130 °C gerührt. Das Reaktionsgemisch wird in Wasser eingerührt und mit Methylenchlorid extrahiert. Das hierbei ungelöst bleibende Produkt wird abfiltriert. Eine weitere Fraktion wird durch Eindampfen der Methylenchloridphase und Chromatographie des Rückstandes über Kieselgel (Elutionsmittel: Methylenchlorid/Methanol = 9:1) erhalten. Die beiden Fraktionen werden vereinigt und ohne zusätzliche Reinigung weiterverarbeitet.
Ausbeute: 1,2 g (36 % der Theorie)
$R_f$-Wert: 0,53 (Kieselgel; Methylenchlorid/Methanol = 9:1)
Analog werden folgende Verbindungen erhalten:

(1) 1-(6-(4-Cyan-phenyl)-3-pyridazinyl]-4-(2-hydroxy-2-methoxycarbonyl-ethyl)-imidazol
$R_f$-Wert: 0,46 (Kieselgel; Methylenchlorid/Methanol = 9:1)

(2) 1-[6-(4-Cyan-phenyl)-3-pyridazinyl]-4-(2-tert.butyloxy-carbonylamino-2-methoxycarbonyl-ethyl)-imidazol
Man arbeitet in Dimethylformamid mit Natriumhydrid als Base bei Raumtemperatur.
Schmelzpunkt: 170-185 °C (Zers.)
$R_f$-Wert: 0,23 (Kieselgel; Methylenchlorid/Methanol = 40:1)

(3) 1-[6-(4-Cyan-phenyl)-3-pyridazinyl]-3-(2-methoxycarbonyl-ethyl)-indol
Man arbeitet in Dimethylformamid mit Natriumhydrid als Base, bei Raumtemperatur.
$R_f$-Wert: 0,86 (Kieselgel; Methylenchlorid/Methanol = 9:1)

(4) 4-(2-tert.Butyloxycarbonylamino-2-methoxycarbonyl-ethyl)-1-[6-(4-methoxycarbonylamidino-phenyl)-3-pyridazinyl]-imidazol
$R_f$-Wert: 0,29 (Kieselgel; Methylenchlorid/Methanol = 15:1)

## Beispiel 4

### 1-[6-(4-Methoxycarbonylamidino-phenyl)-3-pyridazinyl]-4-(2-methoxycarbonyl-ethyl)-imidazol

0,35 g 1-[6-(4-Amidino-phenyl)-3-pyridazinyl]-4-(2-methoxycarbonyl-ethyl)-imidazol werden in einer Mischung aus 25 ml Methanol und 20 ml Methylenchlorid gelöst. Man fügt 0,62 g Chlorameisensäuremethylester zu und hält den pH-Wert durch Zugabe von 1N Natronlauge zwischen 8,5 und 9. Nachdem das Ausgangsmaterial verschwunden ist, destilliert man das Lösungsmittel im Vakuum ab, setzt Wasser zu und schüttelt mit Methylenchlorid aus. Der nach dem Eindampfen der organischen Phasen verbleibende Rückstand wird säulenchromatographisch über Kieselgel gereinigt (Elutionsmittel: Methylenchlorid/Methanol = 20:1).
Ausbeute: 0,05 g (12 % der Theorie),
$R_f$-Wert: 0,43 (Kieselgel; Methylenchlorid/Methanol = 9:1)
Analog werden folgende Verbindungen erhalten:

(1) 4-(4-Methoxycarbonylamidino-phenyl)-2-[4-(2-methoxycarbonyl-ethyl)-phenyl]-1-methyl-imidazol
$R_f$-Wert: 0,50 (Reversed-Phase-Platte RP8; 5%ige Natrium-chloridlösung/Methanol = 4:6)

(2) 4-(4-Methoxycarbonylamidino-phenyl)-2-[4-(2-methoxycarbonyl-ethyl)-phenyl]-imidazol
$R_f$-Wert: 0,53 (Reversed-Phase-Platte RP8; 5%ige Natrium-chloridlösung/Methanol = 4:6)

(3) 1-[6-(4-Ethoxycarbonylamidino-phenyl)-3-pyridazinyl]-4-(2-ethoxycarbonyl-ethyl)-imidazol
$R_f$-Wert: 0,53 (Kieselgel; Methylenchlorid/Methanol = 9:1)

(4) 4-(2-Dibenzylamino-2-methoxycarbonyl-ethyl)-1-[6-(4-methoxycarbonylamidino-phenyl)-3-pyridazinyl]-imidazol-hydrochlorid
$R_f$-Wert: 0,53 (Kieselgel; Methylenchlorid/Methanol = 9:1)

| | | | |
|---|---|---|---|
| Ber. x HCl: | C 63,79 | H 5,35 | N 15,32 |
| Gef.: | 63,58 | 5,43 | 15,26 |

(5) 1-[6-(4-Methoxycarbonylamidino-phenyl)-3-pyridazinyl]-4-(2-methoxycarbonyl-2-methoxycarbonylamino-ethyl)-imidazol

(6) 5-(4-Methoxycarbonylamidino-4'-biphenylyl)-2-(2-methoxycarbonyl-ethyl)-tetrazol
$R_f$-Wert: 0,89 (Kieselgel; Methylenchlorid/Methanol/konz. Ammoniak = 4:1:0,25)

(7) 2-(4-Methoxycarbonylamidino-4'-biphenylyl)-5-(2-methoxycarbonyl-ethyl)-1,3,4-thiadiazol

(8) 5-(2-Isopropyloxycarbonyl-ethyl)-2-(4-methoxycarbonyl-amidino-4'-biphenylyl)-1,3,4-thiadiazol

(9) 4-[4-[(1-Acetoxy-ethyl)-oxycarbonylamidino]-phenyl]-2-[4-(2-methoxycarbonyl-ethyl)-phenyl]-1-methyl-imidazol
Man arbeitet in Methylenchlorid mit N-Ethyl-diisopropylamin
(10) 4-[4-(Acetoxymethyloxycarbonylamidino)-phenyl]-2-[4-(2-methoxycarbonyl-ethyl)-phenyl]-1-methyl-imidazol
Man arbeitet in Methylenchlorid mit N-Ethyl-diisopropylamin
(11) 1-[6-[4-(Acetoxymethyloxycarbonylamidino)-phenyl]-3-pyridazinyl]-4-(2-methoxycarbonyl-ethyl)-imidazol
Man arbeitet in Methylenchlorid mit N-Ethyl-diisopropylamin
(12) 2-[4-(Acetoxymethyloxycarbonylamidino)-4'-biphenylyl]-5-(2-methoxycarbonyl-ethyl)-1,3,4-thiadiazol
Man arbeitet in Methylenchlorid mit N-Ethyl-diisopropylamin
(13) 4-[4-(Butyryloxymethyloxycarbonylamidino)-phenyl]-2-[4-(2-methoxycarbonyl-ethyl)-phenyl]-1-methyl-imidazol
Man arbeitet in Methylenchlorid mit N-Ethyl-diisopropylamin
(14) 4-(4-Allyloxycarbonylamidino-phenyl)-2-[4-(2-methoxycarbonyl-ethyl)-phenyl]-1-methyl-imidazol
(15) 4-[4-(2-Cyclohexyloxycarbonyl-ethyl)-phenyl]-4-(4-methoxycarbonylamidino-phenyl)-1-methyl-imidazol
(16) 4-[4-(2-Cyclopentyloxycarbonyl-ethyl)-phenyl]-4-(4-methoxycarbonylamidino-phenyl)-1-methyl-imidazol
(17) 4-(4-Methoxycarbonylamidino-phenyl)-1-methyl-2-[4-[2-[(2-phenyl-ethyl)-oxycarbonyl]-ethyl]-phenyl]-imidazol

Beispiel 5

5-(4-Cyan-phenyl)-2-[4-(2-methoxycarbonyl-ethyl)-phenyl]-tetrazol

2,16 g 3-(4-Amino-phenyl)-propionsäure-methylester werden in einer Mischung aus 8 ml Wasser, 8 ml Ethanol und 2,6 ml konz. Salzsäure gelöst, auf 0°C gekühlt, tropfenweise mit einer Lösung von 0,7 g Natriumnitrit in 4 ml Wasser versetzt und noch 20 Minuten bei 0°C gerührt. Die entstandene Losung wird zu einer auf -15°C gekühlten Lösung von 2,85 g 4-Cyan-benzaldehyd-benzolsulfonyl-hydrazon in 60 ml Pyridin im Verlauf von 30 Minuten getropft. Man rührt weitere 30 Minuten bei -15°C nach, verdünnt mit Wasser und extrahiert mit Methylenchlorid. Die organischen Phasen werden mit verdünnter Salzsäure und Wasser gewaschen und eingedampft. Das zurückbleibende Rohprodukt wird säulenchromatographisch über Kieselgel gereinigt (Elutionsmittel: Methylenchlorid/Methanol = 50:1). Ausbeute: 2,8 g (84 % der Theorie),
$R_f$-Wert: 0,53 (Kieselgel; Cyclohexan/Essigester = 2:1)

Beispiel 6

5-(4-Cyan-4'-biphenylyl)-2-(2-methoxycarbonyl-ethyl)-tetrazol

1 g 5-(4-Cyan-4'-biphenylyl)-tetrazol, 15 ml Ethanol und 0,45 g Kalium-tert.butylat werden zusammen zum Rückfluß erhitzt und mit 0,44 ml 3-Brom-propionsäure-methylester versetzt. Man erhitzt weitere 16 Stunden, kühlt auf Raumtemperatur ab und wäscht den ausgefallenen Niederschlag mit Wasser.
Ausbeute: 0,4 g (30 % der Theorie),
$R_f$-Wert: 0,69 (Kieselgel; Methylenchlorid/Methanol/Eisessig = 15:1:0,2)
Analog werden folgende Verbindungen erhalten:
(1) 4-(4-Cyan-phenyl)-2-[4-(2-methoxycarbonyl-ethyl)-phenyl]-1-methyl-imidazol
Man verwendet Natriumhydrid als Base und Dimethylformamid als Lösungsmittel
Schmelzpunkt: 107-109°C
$R_f$-Wert: 0,60 (Kieselgel; Methylenchlorid/Essigester/Cyclohexan = 5:2:1)
(2) 3-(4-Cyan-4'-biphenylyl)-5-methoxycarbonylmethylthio-1,2,4-triazol
Man arbeitet in Methanol.
Schmelzpunkt: 206-208°C
$R_f$-Wert: 0,70 (Kieselgel; Essigester/Petrolether = 7:3)

Beispiel 7

4-(4-Cyan-phenyl)-2-[4-(2-methoxycarbonyl-ethyl)-phenyl]-thiazol

5,3 g 4-(2-Methoxycarbonyl-ethyl)-thiobenzoesäure-amid und 5,4 g 2-Brom-4'-cyan-acetophenon werden in 500 ml Methanol gelöst, 24 Stunden zum Rückfluß erhitzt und anschließend 40 Stunden bei Raumtemperatur gerührt. Der ausgefallene Niederschlag wird in 200 ml Essigester aufgenommen, mit halbgesättigter Kaliumcarbonatlösung, Wasser und gesättigter Kochsalzlösung ausgeschüttelt und eingedampft.
Ausbeute: 3,0 g (36 % der Theorie),
Schmelzpunkt: 124 °C
$R_f$-Wert: 0,27 (Kieselgel; Cyclohexan/Essigester = 4:1)
Analog wird folgende Verbindung erhalten:
(1) 4-(4-Cyan-phenyl)-2-[4-(2-methoxycarbonyl-ethyl)-phenyl]-imidazol
Man arbeitet in Dioxan und setzt Natriumcarbonat als Base zu. Als Ausgangsmaterial dient 4-(2-Methoxycarbonyl-ethyl)-benzamidin-hydrochlorid
Schmelzpunkt: 137-139 °C
$R_f$-Wert: 0,50 (Kieselgel; Methylenchlorid/Essigester/Cyclohexan = 5:2:1)

Beispiel 8

2-(4-Cyan-phenyl)-5-[4-(2-methoxycarbonyl-ethyl)-phenyl]-1,3,4-thiadiazol

Eine Mischung aus 1,9 g N-(4-Cyan-benzoyl)-N'-[4-(2-methoxycarbonyl-ethyl)-benzoyl]-hydrazin, 2,35 g 2,4-Bis-(4-methoxy-phenyl)-1,3-dithia-2,4-diphosphetan-2,4-disulfid und 35 ml Tetrahydrofuran wird unter Rühren eine Stunde zum Rückfluß erhitzt. Nach dem Abkühlen gießt man auf eine Lösung von 1,8 g Natriumhydroxid in 135 ml Eiswasser, filtriert den ausgefallenen Niederschlag ab und wäscht ihn mit Wasser. Ausbeute: 1 g (53 % der Theorie),
$R_f$-Wert: 0,93 (Kieselgel; Methylenchlorid/Methanol = 19:1)
Analog wird folgende Verbindung erhalten:
(1) 2-(4-Cyan-4'-biphenylyl)-5-(2-methoxcarbonyl-ethyl)-1,3,4-thiadiazol
$R_f$-Wert: 0,90 (Kieselgel; Methylenchlorid/Methanol = 9:1)

Beispiel 9

3-(4-Cyan-phenyl)-5-[4-(2-methoxycarbonyl-ethyl)-phenyl]-1,2,4-triazol

3,0 g 4-(2-Methoxycarbonyl-ethyl)-iminobenzoesäure-methyl-ester (hergestellt aus 4-(2-Methoxycarbonyl-ethyl)-benzonitril und methanolischer Salzsäure), 2,0 g 4-Cyan-benzhydrazid und 45 ml Pyridin werden 6 Stunden auf 50 °C erwärmt. Man gießt auf 250 ml Wasser, extrahiert mit 150 ml Methylenchlorid, wäscht die organische Phase mit Wasser und 2N Salzsäure, engt ein und trennt den Rückstand durch Chromatographie an Kieselgel in die Komponenten (Elutionsmittel: Methylenchlorid/Methanol = 9:1).
Ausbeute: 1,0 g (22 % der Theorie),
$R_f$-Wert: 0,68 (Kieselgel; Methylenchlorid/Methanol = 8:2)
Analog wird folgende Verbindung erhalten:
(1) 2-(4-Cyan-phenyl)-5-[4-(2-methoxycarbonyl-ethyl)-phenyl]-1,3,4-oxadiazol
Die Verbindung entsteht als eines der Nebenprodukte in dem oben beschriebenen Ansatz.
$R_f$-Wert: 0,91 (Kieselgel; Methylenchlorid/Methanol = 8:2)

Beispiel 10

4-(2-Amino-2-aminocarbonyl-ethyl)-1-[6-(4-aminomethyl-phenyl)-3-pyridazinyl]-imidazol

1,9 g 4-(2-Amino-2-methoxycarbonyl-ethyl)-1-[6-(4-cyan-phenyl)-3-pyridazinyl]-imidazol werden in 300 ml methanolischem Ammoniak in Gegenwart von 0,5 g Raney-Nickel mit Wasserstoff von 5 bar 12 Stunden bei Raumtemperatur hydriert. Man filtriert vom Katalysator ab, dampft im Vakuum ein und reinigt den Rückstand durch Chromatographie an Kieselgel (Elutionsmittel: Methylenchlorid/Methanol/konz. Ammoniak = 1:1:0,1). Ausbeute: 0,45 g (24 % der Theorie),
$R_f$-Wert: 0,41 (Kieselgel; Methylenchlorid/Methanol/konz. Ammoniak = 4:1:0,25)
Analog werden folgende Verbindungen erhalten:
(1) 4-[4-(2-Amino-ethyl)-phenyl]-2-[4-(2-methoxycarbonyl-ethyl)-phenyl]-1-methyl-imidazol

Man verwendet 10%ige Palladium-Kohle und arbeitet in einem 10:1-Gemisch aus Methanol und methanolischer Salzsäure.
(2) 4-(4-Aminomethyl-phenyl)-2-[4-(2-methoxycarbonyl-ethyl)-phenyl]-1-methyl-imidazol
Man arbeitet analog (1).
(3) 1-[6-(4-Aminomethyl-phenyl)-3-pyridazinyl]-4-(2-methoxycarbonyl-ethyl)-imidazol-hydrochlorid
Man arbeitet analog (1).

Beispiel 11

4-[4-(1-Amidino-4-piperidinyl)-phenyl]-2-(2-methoxycarbonyl-ethyl)-thiazol

Herstellung aus 2-(2-Methoxycarbonyl-ethyl)-4-[4-(4-piperidinyl)-phenyl]-thiazol und S-Ethylisothioharnstoff-hydrobromid durch vierstündiges Erwärmen auf 100°C in Dimethylformamid in Gegenwart von Natriumcarbonat.

Beispiel 12

1-(4-Amidino-3'-methylsulfinyl-4'-biphenylyl)-4-(2-methoxycarbonyl-ethyl)-imidazol

Herstellung durch Oxidation von 1-(4-Amidino-3'-methylthio-4'-biphenylyl)-4-(2-methoxycarbonyl-ethyl)-imidazol mit Wasserstoffperoxid in Eisessig.
Analog werden folgende Verbindugen erhalten:
(1) 4-(4-Amidino-phenyl)-2-(4-methoxycarbonylmethyloxy-3-methylsulfinyl-phenyl)-imidazol
(2) 4-(4-Amidino-phenyl)-2-(4-methoxycarbonylmethylsulfinylphenyl)-imidazol

Beispiel 13

1-[6-(4-Amidino-phenyl)-3-pyridazinyl]-4-(2-n-butyloxycarbonyl-ethyl)-imidazol

Herstellung aus 1-[6-(4-Amidino-phenyl)-3-pyridazinyl]-4-(2-methoxycarbonyl-ethyl)-imidazol durch dreitägiges Rühren mit gesättigter n-butanolischer Salzsäure bei Raumtemperatur.
Analog werden folgende Verbindungen erhalten:
(1) 4-(4-Amidino-phenyl)-2-[4-(2-cyclohexyloxycarbonyl-ethyl)-phenyl]-1-methyl-imidazol
(2) 4-(4-Amidino-phenyl)-2-[4-(2-cyclopentyloxycarbonyl-ethyl)-phenyl]-1-methyl-imidazol
(3) 4-(4-Amidino-phenyl)-2-[4-(2-cycloctyloxycarbonyl-ethyl)-phenyl]-1-methyl-imidazol
(4) 4-(4-Amidino-phenyl)-2-[4-(2-cyclohexylmethyloxycarbonyl-ethyl)-phenyl]-1-methyl-imidazol
(5) 4-(4-Amidino-phenyl)-1-methyl-2-[4-[2-(2-phenyl-ethyl)-oxycarbonylethyl]-phenyl]-imidazol

Beispiel 14

2-(4-Cyan-4'-biphenylyl)-3-ethoxycarbonyl-5-(2-ethoxycarbonyl-ethyl)-furan

6,2 g 7-(4-Cyan-4'-biphenyl)-6-ethoxycarbonyl-4,7-dioxo-heptansäure-ethylester werden in 250 ml Toluol auf dem Dampfbad 6 Stunden mit 8,2 g Phosphorpentoxid behandelt. Man filtriert vom Niederschlag ab, wäscht dreimal mit 150 ml heißem Toluol nach und dampft das Filtrat im Vakuum ein. Der Rückstand wird mit Ether kristallin gerieben, abfiltriert und mit Ether nachgewaschen. Aus den Mutterlaugen erhält man eine weitere Fraktion.
Ausbeute: 4,4 g (70 % der Theorie),
Schmelzpunkt: 115-116°C
$R_f$-Wert: 0,61 (Kieselgel; Essigester/Cyclohexan = 1:2,5, zweifach entwickelt)

Beispiel 15

4-(3-Guanidino-phenyl)-2-[4-(2-methoxycarbonyl-ethyl)-phenyl]-imidazol-hydrochlorid

Herstellung aus 4-(3-Amino-phenyl)-2-[4-(2-methoxycarbonyl-ethyl)-phenyl]-imidazol-hydrochlorid durch dreistündiges Rückflußkochen mit Cyanamid in Dioxan.

Beispiel 16

2-[4-[(2-Methoxycarbonyl-ethyl)-aminocarbonyl]-phenyl]-1-methyl-4-(4-piperidinyl)-imidazol

Herstellung durch Behandeln von 4-(1-Benzyloxycarbonyl-4-piperidinyl)-2-[4-[(2-methoxycarbonyl-ethyl)-aminocarbonyl]-phenyl]-1-methyl-imidazol mit Wasserstoff von 3 bar in Gegenwart von 5%iger Palladiumkohle in Methanol.

Analog werden folgende Verbindungen erhalten:

(1) 4-(4-Amino-cyclohexyl)-2-[4-[(2-methoxycarbonyl-ethyl)-aminocarbonyl]-phenyl]-1-methyl-imidazol

(2) 2-[4-(2-Methoxycarbonyl-ethyl)-phenyl]-1-methyl-4-(4-methylaminomethyl-phenyl)-imidazol

(3) 2-(4-Amino-cyclohexyl)-4-[4-(2-methoxycarbonyl-ethyl)-phenyl]-imidazol

(4) 4-(4-Amidino-phenyl)-1-methyl-2-[4-[2-(pivaloyloxymethyloxycarbonyl)-ethyl]-phenyl]-imidazol

(5) 4-(4-Amidino-phenyl)-2-[4-[2-[(1-ethoxycarbonyloxy-ethyl)-oxycarbonyl]-ethyl]-phenyl]-1-methyl-imidazol

(6) 4-(4-Amidino-phenyl)-2-[4-[2-[(1-cyclohexyloxycarbonyloxy-ethyl)-oxycarbonyl]-ethyl]-phenyl]-1-methyl-imidazol

(7) 2-[4-[2-(Acetoxymethyloxycarbonyl)-ethyl]-phenyl]-4-(4-amidino-phenyl)-1-methyl-imidazol

(8) 4-(4-Amidino-phenyl)-2-[4-[2-(butyryloxymethyloxycarbonyl]-ethyl]-phenyl]-1-methyl-imidazol

(9) 4-(4-Amidino-phenyl)-2-[4-[2-(isobutyryloxymethyloxycarbonyl)-ethyl]-phenyl]-1-methyl-imidazol

(10) 4-(4-Amidino-phenyl)-2-[4-[2-(benzoyloxymethyloxycarbonyl)-ethyl]-phenyl]-1-methyl-imidazol

(11) 1-[6-(4-Amidino-phenyl)-3-pyridazinyl]-4-[2-[(cyclohexyloxycarbonyloxymethyl)-oxycarbonyl]-ethyl]-imidazol

(12) 2-(4-Amidino-4'-biphenylyl)-5-[2-[(cyclohexyloxycarbonyloxymethyl)-oxycarbonyl]-ethyl]-1,3,4-thiadiazol

(13) 1-[6-(4-Amidino-phenyl)-3-pyridazinyl]-4-[2-[(1-ethoxy-carbonyloxy-ethyl)-oxycarbonyl]-ethyl]-imidazol

(14) 2-(4-Amidino-4'-biphenylyl)-5-[2-[(1-ethoxycarbonyloxy-ethyl)-oxycarbonyl]-ethyl]-1,3,4-thiadiazol

(15) 1-[6-(4-Amidino-phenyl)-3-pyridazinyl]-4-[2-(pivaloyloxymethyloxycarbonyl)-ethyl]-imidazol

(16) 2-(4-Amidino-4'-biphenylyl)-5-[2-(pivaloyloxymethyloxy-carbonyl)-ethyl]-1,3,4-thiadiazol

Beispiel 17

4-(2-Amino-2-methoxycarbonyl-ethyl)-1-[6-(4-cyan-phenyl)-3-pyridazinyl]-imidazol

Eine Lösung von 3,1 g 4-(2-tert.Butyloxycarbonylamino-2-methoxycarbonyl-ethyl)-1-[6-(4-cyan-phenyl)-3-pyridazinyl]-imidazol in 50 ml Methylenchlorid wird auf 0°C abgekühlt und mit 20 ml Trifluoressigsäure versetzt. Man rührt 16 Stunden bei Raumtemperatur, dampft im Vakuum bei Raumtemperatur ein und reinigt den Rückstand durch Chromatographie an Kieselgel (Elutionsmittel: Methylenchlorid/Methanol/konz. Ammoniak = 10:1:0,1)

Ausbeute: 1,9 g (79 % der Theorie)

Schmelzpunkt: 110-112°C

$R_f$-Wert: 0,37 (Kieselgel; Methylenchlorid/Methanol/konz. Ammoniak = 10:1:0,1)

Analog werden folgende Verbindungen erhalten:

(1) 4-[4-(1-Amino-cyclopentyl)-phenyl]-2-[4-(2-methoxycarbonyl-ethyl)-phenyl]-1-methyl-imidazol

(2) 4-(2-Amino-2-methoxycarbonyl-ethyl)-1-[4-(4-aminomethyl-piperidino)-phenyl]-imidazol

(3) 4-(2-Amino-2-methoxycarbonyl-ethyl)-1-[4-(4-aminomethyl-2-oxo-piperidino)-phenyl]-imidazol

(4) 4-[4-(1-Amino-cyclopropyl)-phenyl]-2-[4-(2-methoxycarbonyl-ethyl)-phenyl]-1-methyl-imidazol

Beispiel 18

4-[4-(2-Amino-2-propyl)-phenyl]-2-[4-(2-methoxycarbonyl-ethyl)-phenyl]-1-methyl-imidazol

Herstellung aus 4-[4-(2-Aminocarbonyl-2-propyl)-phenyl]-2-[4-(2-methoxycarbonyl-ethyl)-phenyl]-1-methyl-imidazol durch Behandeln mit [Bis(trifluoracetoxy)iod]benzol in Acetonitril/Wasser bei Raumtemperatur.

Beispiel 19

4-[4-(1-Amino-ethyl)-phenyl]-2-[4-(2-methoxycarbonyl-ethyl)-phenyl]-1-methyl-imidazol

Herstellung aus 4-[4-(1-Hydroxyimino-ethyl)-phenyl]-2-[4-(2-methoxycarbonyl-ethyl)-phenyl]-1-methyl-imidazol durch Reduktion mit Wasserstoff von 5 bar in einem 50:1-Gemisch aus Methanol und methanolischer Salzsäure bei Raumtemperatur in Gegenwart von 10%iger Palladiumkohle.

Analog werden folgende Verbindungen erhalten:

(1) 4-(1-Amino-5-indanyl)-2-[4-(2-methoxycarbonyl-ethyl)-phenyl]-1-methyl-imidazol

(2) 4-(1-Amino-1,2,3,4-tetrahydro-6-naphthyl)-2-[4-(2-methoxycarbonyl-ethyl)-phenyl]-1-methyl-imidazol

Beispiel 20

1-[6-(4-Amidino-phenyl)-3-pyridazinyl]-4-(2-hydroxy-2-methoxycarbonyl-ethyl)-imidazol-hydrochlorid

Eine Mischung aus 0,07 g 1-[6-(4-Amidino-phenyl)-3-pyridazinyl]-4-(2-carboxy-2-hydroxy-ethyl)-imidazol, 100 ml Methanol und 20 ml methanolischer Salzsäure wird 3 Stunden bei Raumtemperatur gerührt und anschließend im Vakuum eingedampft.
Ausbeute: 0,08 g (100 % der Theorie),
Schmelzpunkt: sintert ab 240 °C
$R_f$-Wert: 0,17 (Kieselgel; Methylenchlorid/Methanol/konz. Ammoniak = 2:1:0,25)
Analog werden folgende Verbindungen erhalten:

(1) 4-(2-Amino-2-methoxycarbonyl-ethyl)-1-[6-(4-aminomethyl-phenyl)-3-pyridazinyl]-imidazol

(2) 2-(4-Amidino-4'-biphenylyl)-5-(2-isopropyloxycarbonyl-ethyl)-1,3,4-thiadiazol

Man geht vom zugehörigen Methylester aus und arbeitet in isopropanolischer Salzsäure.

Beispiel 21

4-[4-(Dimethylaminomethyl)-phenyl]-2-[4-(2-methoxycarbonyl-ethyl)-phenyl]-1-methyl-imidazol

Herstellung aus 4-(4-Aminomethyl-phenyl)-2-[4-(2-methoxycarbonyl-ethyl)-phenyl]-1-methyl-imidazol durch Behandeln mit Formaldehyd und Natriumcyanborhydrid.

Analog wird folgende Verbindung erhalten:

(1) 2-[4-[(2-Methoxycarbonyl-ethyl)-aminocarbonyl]-phenyl]-1-methyl-4-(1-methyl-4-piperidinyl)-imidazol

Beispiel 22

1-[6-(4-Cyan-phenyl)-3-pyridazinyl]-4-(2-dibenzylamino-2-methoxycarbonyl-ethyl)-imidazol

1,1 g 4-(2-Amino-2-methoxycarbonyl-ethyl)-1-[6-(4-cyan-phenyl)-3-pyridazinyl]-imidazol werden in 20 ml Methylenchlorid gelöst, mit 60 ml Benzylchlorid und 10 ml Triethylamin versetzt und 6 Stunden bei 75-80 °C gerührt. Man dampft im Vakuum ein, versetzt mit Wasser und extrahiert mit Essigester. Die Essigesterphase wird eingedampft und der Rückstand durch Chromatographie über Kieselgel gereinigt (Elutionsmittel: Methylenchlorid/Methanol = 20:1).
Ausbeute: 0,55 g (32 % der Theorie),
$R_f$-Wert: 0,93 (Kieselgel; Methylenchlorid/Methanol/konz. Ammoniak = 4:1:0,25)

Beispiel 23

4-(4-Benzyloxycarbonylamidino-phenyl)-1-methyl-2-[4-[2-(pivaloyloxymethyloxycarbonyl)-ethyl]-phenyl]-imidazol

Herstellung durch Umsetzung von 4-(4-Benzyloxycarbonylamidino-phenyl)-2-[4-(2-carboxy-ethyl)-phenyl]-1-methyl-imidazol mit Pivaloyloxymethylchlorid in Dimethylsulfoxid in Gegenwart von Kaliumcarbonat unter Zusatz von Kaliumiodid bei Raumtemperatur.

Analog werden folgende Verbindungen erhalten:

(1) 4-(4-Benzyloxycarbonylamidino-phenyl)-2-[4-[2-[(1-ethoxy-carbonyloxy-ethyl)-oxycarbonyl]-ethyl]-phenyl]-1-methyl-imidazol

(2) 4-(4-Benzyloxycarbonylamidino-phenyl)-2-[4-[2-[(1-cyclohexyloxycarbonyloxy-ethyl)-oxycarbonyl]-ethyl]-phenyl]-1-methyl-imidazol

(3) 2-[4-[2-(Acetoxymethyloxycarbonyl)-ethyl]-phenyl]-4-(4-benzyloxycarbonylamidino-phenyl)-1-methyl-imidazol

(4) 4-(Benzyloxycarbonylamidino-phenyl)-2-[4-[2-(butyryloxy-methyloxycarbonyl)-ethyl]-phenyl]-1-methyl-imidazol

(5) 4-(Benzyloxycarbonylamidino-phenyl)-2-[4-[2-(isobutyryloxymethyloxycarbonyl)-ethyl]-phenyl]-1-methyl-imidazol

(6) 2-[4-[2-(Benzoyloxymethyloxycarbonyl)-ethyl]-4-(4-benzyloxycarbonylamidino-phenyl)-1-methyl-imidazol

(7) 1-[6-(4-Benzyloxycarbonylamidino-phenyl)-3-pyridazinyl]-4-[2-[(cyclohexyloxycarbonyloxymethyl)-oxycarbonyl]-ethyl]-imidazol

(8) 2-(4-Benzyloxycarbonylamidino-4'-biphenylyl)-5-[2-[(cyclohexyloxycarbonyloxymethyl)-oxycarbonyl]-ethyl]-1,3,4-thiadiazol

(9) 1-[6-(4-Benzyloxycarbonylamidino-phenyl)-3-pyridazinyl]-4-[2-[(1-ethoxycarbonyloxy-ethyl)-oxycarbonyl]-ethyl]-imidazol

(10) 2-(4-Benzyloxycarbonylamidino-4'-biphenylyl)-5-[2-[(1-ethoxycarbonyloxy-ethyl)-oxycarbonyl]-ethyl]-1,3,4-thiadiazol

(11) 1-[6-(4-Benzyloxycarbonylamidino-phenyl)-3-pyridazinyl]-4-[2-(pivaloyloxymethyloxycarbonyl)-ethyl]-imidazol

(12) 2-(4-Benzyloxycarbonylamidino-4'-biphenylyl)-5-[2-(pivaloyloxymethyloxycarbonyl)-ethyl]-1,3,4-thiadiazol

## Beispiel 24

### 4-(4-Diethylphosphorylamidino-phenyl)-2-[4-(2-methoxycarbonyl-ethyl)-phenyl]-1-methyl-imidazol

Herstellung durch Umsetzung von 4-(4-Amidino-phenyl)-2-[4-(2-methoxycarbonyl-ethyl)-phenyl]-1-methyl-imidazol mit Diethylphosphorylcyanid in Dimethylformamid.

## Beispiel 25

### Trockenampulle mit 2,5 mg Wirkstoff pro 1 ml

| Zusammensetzung: | |
|---|---|
| Wirkstoff | 2,5 mg |
| Mannitol | 50,0 mg |
| Wasser für Injektionszwecke ad | 1,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

## Beispiel 26

### Trockenampulle mit 35 mg Wirkstoff pro 2 ml

| Zusammensetzung: | |
|---|---|
| Wirkstoff | 35,0 mg |
| Mannitol | 100,0 mg |
| Wasser für Injektionszwecke ad | 2,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 27

Tablette mit 50 mg Wirkstoff

| Zusammensetzung: | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 28

Tablette mit 350 mg Wirkstoff

| Zusammensetzung: | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

Beispiel 29

Kapseln mit 50 mg Wirkstoff

| Zusammensetzung: | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

Beispiel 30

Kapseln mit 350 mg Wirkstoff

| Zusammensetzung: | |
|---|---|
| (1) Wirkstoff | 300,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

## Patentansprüche

**1.** 5-gliedrige Heterocyclen der allgemeinen Formel

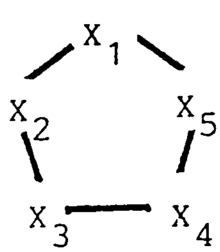

,(I)

in der mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält,

einer der Reste $X_1$ bis $X_5$ eine Gruppe der Formeln

A - B - C - N< ,

A - B - C - CH< oder

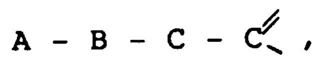

in denen

A eine Cyanogruppe, eine geradkettige oder verzweigte Cyanoalkylgruppe mit insgesamt 2 bis 4 Kohlenstoffatomen, eine nicht direkt an einen Phenylring der Gruppen B oder C gebundene Aminogruppe, eine geradkettige oder verzweigte Aminoalkylgruppe mit 1 bis 4 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 bis 6 Kohlenstoffatomen, durch eine Aralkoxycarbonyl-, Arylcarbonyl-, Aryloxycarbonyl-, Alkanoyloxymethoxycarbonyl-, Cycloalkanoyloxymethoxycarbonyl-, Aralkanoyloxymethoxycarbonyl-, Aroyloxymethoxycarbonyl-, Phosphono-, Dialkylphosphoryl oder O-Alkyl-phosphonogruppe ersetzt sein kann, in denen jeweils die Alkanoylteile insgesamt 2 bis 7 Kohlenstoffatome und die Cycloalkanoylteile insgesamt 4 bis 8 Kohlenstoffatome enthalten sowie der Methoxyteil jeweils durch eine Cycloalkylgruppe mit 3 bis 6 Kohlenstoffatomen, durch eine Aralkyl-, Aryl- oder Alkylgruppe oder durch zwei Alkylgruppen, die zusammen mit dem Methylenkohlenstoffatom auch einen 5- oder 6-gliedrigen Ring bilden können, substituiert sein kann, oder, falls B oder B und C zusammen ein cyclisches Imin mit 4 bis 7 Ringgliedern darstellen, auch ein an den Iminstickstoff gebundenes Wasserstoffatom oder ein an den Iminstickstoff gebundener Alkylrest,

B eine Bindung,

eine Alkylen- oder Alkenylengruppe,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können und $R_1$ jeweils ein Wasserstoffatom, eine Alkylgruppe mit 1 bis 5 Kohlenstoffatomen, eine Aralkyl-, Aryl- oder Heteroarylgruppe bedeutet,

eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine $-CO-NR_1$-Gruppe ersetzt sein können und dann eines der Stickstoffatome statt an den Rest $R_1$ auch an den Rest C gebunden sein kann,

eine gegebenenfalls durch eine Alkyl-, Aralkyl- oder Arylgruppe substituierte Cyclopropylengruppe, eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, und

C eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich

oder verschieden sein können,

eine Indanylen- oder 1,2,3,4-Tetrahydronaphthylengruppe, in denen jeweils der gesättigte Ring an den Rest A oder B und der aromatische Ring an das im Ring befindliche Atom des jeweiligen Restes $X_1$ bis $X_5$ gebunden ist,

eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine -CO-$NR_1$-Gruppe ersetzt sein können und dann eines der Stickstoffatome statt an den Rest $R_1$ auch an den Rest B oder an das im Ring befindliche Atom des jeweiligen Restes $X_1$ bis $X_5$ gebunden sein kann, wobei jedoch C keine Pyrimidinylengruppe darstellen kann, wenn das heterocyclische Ringsystem ein Dithiolanring und gleichzeitig der Rest A eine Aminogruppe darstellt,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann, oder

eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarten Methylengruppen durch eine Carbonylgruppe ersetzt sein können, darstellen,

ein zweiter der Reste $X_1$ bis $X_5$ eine Gruppe der Formeln

F - E - D - N< ,
F - E - D - CH< oder

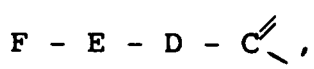

in denen

D eine gegebenenfalls durch eine Hydroxy-, Alkoxy-, Alkylsulfenyl-, $(R_1)_2N$-, (Alkylcarbonyl)$NR_1$-, (Aralkylcarbonyl)$NR_1$-, (Arylcarbonyl)$NR_1$-, (Heteroarylcarbonyl)$NR_1$-, (Alkoxycarbonyl)$NR_1$-, (Aralkoxycarbonyl)$NR_1$-, (Aryloxycarbonyl)$NR_1$-, ($(R_1)_2NCO$)$NR_1$-, (Alkylsulfonyl)$NR_1$-, (Aralkylsulfonyl)-$NR_1$-, (Arylsulfonyl)$NR_1$- oder $R_1OCO$-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$-, $(R_1)_2NSO_2$-oder $R_1OCO$-alkoxy-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können,

eine Pyridinylen-, Pyrimidinylen-, Pyrazinylen-, Pyridazinylen- oder Triazinylengruppe, die jeweils im Kohlenstoffgerüst durch ein Chloratom, durch eine Alkyl- oder Alkoxygruppe substituiert sein kann, wobei zusätzlich eine oder zwei -CH=N-Gruppen jeweils durch eine -CO-$NR_1$-Gruppe ersetzt sein können und dann eines der Stickstoffatome statt an den Rest $R_1$ auch an den Rest E, sofern dieser keine Bindung darstellt und nicht über ein Sauerstoff- oder Schwefelatom an den Rest D gebunden ist, oder an das im Ring befindliche Atom des jeweiligen Restes $X_1$ bis $X_5$ gebunden sein kann,

eine gegebenenfalls durch eine Alkyl-, Aralkyl- oder Arylgruppe substituierte Cycloalkylengruppe mit 4 bis 5 Kohlenstoffatomen, in der eine CH-Einheit durch ein Stickstoffatom und zusätzlich eine zum Stickstoffatom benachbarte Methylengruppe durch eine Carbonylgruppe ersetzt sein kann,

eine gegebenenfalls durch eine Alkyl-, Aralkyl- oder Arylgruppe substituierte Cycloalkylengruppe mit 6 oder 7 Kohlenstoffatomen, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, oder

eine über den Rest $W_1$ mit dem im Ring befindlichen Atom des jeweiligen Restes $X_1$ bis $X_5$ verknüpfte Alkylengruppe mit 1 bis 6 Kohlenstoffatomen, in der $W_1$ eine $NR_1$-Gruppe, ein Sauerstoff- oder Schwefelatom darstellt,

E eine Bindung,

eine geradkettige oder verzweigte Alkylengruppe mit 1 bis 5 Kohlenstoffatomen oder eine Alkenylengruppe mit 2 bis 5 Kohlenstoffatomen, die jeweils durch eine $R_1$OCO-alkyl-Gruppe substituiert sein kann, oder

eine über den Rest $W_2$ mit dem Rest D verknüpfte Alkylengruppe, in der $W_2$ ein Sauerstoff- oder Schwefelatom, eine Sulfinyl-, Sulfonyl-, $-NR_1-$, (Alkylcarbonyl)N-, -(Aralkylcarbonyl)N-, -(Arylcarbonyl)N-, (Heteroarylcarbonyl)N-, -(Alkylsulfonyl)N-, (Arylsulfonyl)N-, $-CONR_1-$ oder $-NR_1CO$-Gruppe darstellt,

F eine nicht an ein Heteroatom der Reste D oder E gebundene Carbonylgruppe, die durch eine Hydroxygruppe, durch eine Aminogruppe, durch eine Alkoxygruppe mit 1 bis 6 Kohlenstoffatomen, in der ein Alkoxyrest mit 1 bis 3 Kohlenstoffatomen in 1-, 2- oder 3-Stellung durch eine Cycloalkylgruppe mit 4 bis 8 Kohlenstoffatomen, durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, durch eine Cycloalkoxygruppe mit 4 bis 8 Kohlenstoffatomen, durch eine Alkanoyloxymethoxygruppe mit insgesamt 2 bis 7 Kohlenstoffatomen im Alkanoylteil, durch eine Cycloalkanoyloxymethoxygruppe mit insgesamt 4 bis 8 Kohlenstoffatomen im Cycloalkanoylteil, durch eine Alkoxycarbonyloxymethoxygruppe mit 1 bis 6 Kohlenstoffatomen im Alkylteil, durch eine Cycloalkoxycarbonyloxymethoxygruppe mit 3 bis 7 Kohlenstoffatomen im Cycloalkylteil, durch eine Aroyloxymethoxy-, Aralkanoyloxymethoxy-, Aryloxycarbonyloxymethoxy- oder Aralkoxycarbonyloxymethoxygruppe, in denen der Methoxyteil jeweils durch eine Alkylgruppe mit 1 bis 6 Kohlenstoffatomen, durch eine Cycloalkylgruppe mit 3 bis 7 Kohlenstoffatomen, durch eine Aralkyl- oder Arylgruppe substituiert sein kann, substituiert ist, eine Sulfo-, Phosphono-, O-Alkylphosphono- oder Tetrazol-5-ylgruppe darstellen, wobei, falls A eine Aminogruppe oder eine Cyanogruppe darstellt, der kürzeste Abstand zwischen dieser Gruppe und dem Rest F mindestens 10 Bindungen beträgt und generell A keine Cyanogruppe darstellen kann, wenn das heterocyclische Ringsystem ein Pyrazolinring und gleichzeitig die Reste C und D unsubstituierte Phenylengruppen und gleichzeitig die Reste B und E eine Bindung darstellen oder, wenn das heterocyclische Ringsystem ein Oxazol- oder Oxazolinring und gleichzeitig der Rest C eine unsubstituierte Phenylengruppe und B eine Bindung darstellen,

ein dritter der Reste $X_1$ bis $X_5$ ein Schwefelatom, eine Sulfinyl-, Sulfonyl-, $R_1N<$,

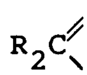

oder $(R_2)_2C<$ Gruppe oder ein N-Atom, wobei $R_1$ jeweils wie eingangs definiert ist und

$R_2$ ein Wasserstoff-, Chlor- oder Bromatom, eine Alkylgruppe mit 1 bis 7 Kohlenstoffatomen, eine Arylalkyl-, Aryl-, Heteroaryl-, Alkoxy-, $(R_1)_2N$-, $R_1OOC$- oder $(R_1)_2NCO$-Gruppe bedeutet,

ein vierter der Reste $X_1$ bis $X_5$ ein Sauerstoff-, Schwefel- oder Stickstoffatom, eine Sulfonyl- oder

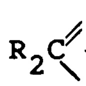 -Gruppe

oder auch eine Carbonylgruppe, wenn diese nicht zwischen zwei Stickstoffatomen steht,

ein fünfter der Reste $X_1$ bis $X_5$ ein Stickstoffatom, eine

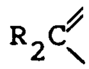

oder $(R_2)_2C<$ Gruppe oder auch zwei benachbarte Reste der Reste $X_1$ bis $X_5$ zusammen eine o-Phenylengruppe bedeuten, darstellen,

wobei, soweit nichts anderes erwähnt wurde,

die vorstehend erwähnten Alkyl-, Alkylen-, Alkenylen- oder Alkoxyteile jeweils 1 bis 3 Kohlenstoffatome enthalten können, sowie

unter den vorstehend erwähnten Begriffen "eine Arylgruppe" oder "eine Aroylgruppe" eine Phenyl-, Naphthyl- oder Benzoylgruppe, die durch eine Trifluormethyl-, Carboxy-, $(R_1)_2NCO$-,Alkoxycarbonyl-, Alkylcarbonyl-, Alkylsulfenyl-, Alkylsulfinyl-, Alkylsulfonyl-, Nitro-, $(R_1)_2N$-, Alkylcarbonyl-$NR_1$-, Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono-, di- oder trisubstituiert sein können, und

unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist,

deren Tautomere, deren Stereoisomere einschließlich deren Gemische und deren Salze.

**2.** Furan-, Tetrahydrofuran-, 2,3-Dihydro-furan-, 2,5-Dihydro-furan-, Thiophen-, 2,3-Dihydro-thiophen-, 2,5-Dihydro-thiophen-, Tetrahydrothiophen-, S-Oxido-tetrahydrothiophen-, S,S-Dioxido-tetrahydrothiophen-, 1,2-Dithiolan-, 1,3-Dithiolan-, 1,3-Dithiolan-S,S,S',S'-tetraoxid-, Pyrrol-, Indol-, Isoindol-, 2,3-Dihydro-indol-, 2,3-Dihydro-isoindol-, 2-Indolon-, Imidazol-, 4,5-Dihydro-imidazol-, Tetrahydroimidazol-, Benzimidazolin-, Pyrazol-, 2H-Pyrazol-5-on-, 4,5-Dihydro-pyrazol-, 1,5-Dihydro-pyrazol-, Indazol-, 2,3-Dihydro-indazol-, Oxazol-, Isoxazol-, Oxazolin-, Oxazolidin-, Thiazol-, Isothiazol-, Thiazolin-, Thiazolidin-, 1,3,4-Oxadiazol-, 1,3,4-Thiadiazol-, 1,2,3-Triazol-, 1,2,4-Triazol- und Tetrazolderivate gemäß Anspruch 1,

deren Tautomere, deren Stereoisomere einschließlich deren Gemische und deren Salze.

**3.** 5-gliedrige Heterocyclen der allgemeinen Formel I gemäß Anspruch 1 oder 2, in denen mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält,

einer der Reste $X_1$ bis $X_5$ eine Gruppe der Formeln

A - B - C - N< ,
A - B - C - CH< oder

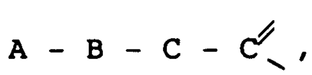

in denen

A eine Cyanogruppe, eine geradkettige oder verzweigte Cyanoalkylgruppe mit insgesamt 2 bis 4 Kohlenstoffatomen, eine nicht direkt an einen Phenylring der Gruppen B oder C gebundene Aminogruppe, eine geradkettige oder verzweigte Aminoalkylgruppe mit 1 bis 4 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Alkenyloxycarbonylgruppe mit insgesamt 4 oder 5 Kohlenstoffatomen, durch eine Aralkoxycarbonyl-, Aryloxycarbonyloxy- oder Arylcarbonylgruppe oder durch eine Alkanoyloxymethoxycarbonylgruppe, in der der Alkanoylteil insgesamt 2 bis 7 Kohlenstoffatome enthalten und der Methoxyteil durch eine Alkylgruppe substituiert sein kann, oder durch eine Phosphono-, Dialkylphosphoryl- oder O-Alkyl-phosphonogruppe ersetzt sein kann, oder, falls B oder B und C zusammen ein cyclisches Imin mit 6 Ringgliedern darstellen, auch ein an den Iminstickstoff gebundenes Wasserstoffatom oder ein an den Iminstickstoff gebundener Alkylrest,

B eine Bindung,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können und $R_1$ jeweils ein Wasserstoffatom, eine Alkylgruppe mit 1 bis 5 Kohlenstoffatomen, eine Aralkyl- oder Arylgruppe bedeutet,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine $-CO-NR_1$-Gruppe ersetzt sein können und dann eines der Stickstoffatome statt an den Rest $R_1$ auch an den Rest C gebunden sein kann,

eine Cycloalkylengruppe mit 3 bis 5 Kohlenstoffatomen, eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppe durch eine Carbonylgruppe ersetzt sein können, und

C eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$- oder $(R_1)_2NSO_2$-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1NH$-Gruppen mono- oder disubstituiert sein kann, wobei die Substituenten gleich oder verschieden sein können, eine Indanylen- oder 1,2,3,4-Tetrahydronaphthylengruppe, in denen jeweils der gesättigte Ring an den Rest A und der aromatische Ring an das im Ring befindliche Atom des jeweiligen Restes $X_1$ bis $X_5$ gebunden ist,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH=N-Gruppen jeweils durch eine $-CO-NR_1$-Gruppe ersetzt sein können und dann eines der Stickstoffatome statt an den Rest $R_1$ auch an den Rest B oder an das im Ring befindliche Atom des jeweiligen Restes $X_1$ bis $X_5$ gebunden sein kann, sofern dieses ein Kohlenstoffatom darstellt,

eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, darstellen,

ein zweiter der Reste $X_1$ bis $X_5$ eine Gruppe der Formeln

F - E - D - N< ,
F - E - D - CH< oder

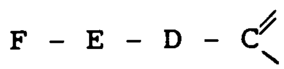 ,

in denen

D eine gegebenenfalls durch eine Hydroxy-, Alkoxy-, Alkylsulfenyl-, $(R_1)_2N$-, (Alkylcarbonyl)$NR_1$-, (Aralkylcarbonyl)$NR_1$-, (Arylcarbonyl)$NR_1$-, (Heteroarylcarbonyl)$NR_1$-, (Alkoxycarbonyl)$NR_1$-, (Aralkoxycarbonyl)$NR_1$-, (($R_1$)$_2$NCO)$NR_1$-, (Alkylsulfonyl)$NR_1$-, (Aralkylsulfonyl)$NR_1$-, (Arylsulfonyl)$NR_1$- oder $R_1$OCO-Gruppe substituierte geradkettige oder verzweigte Alkylen- oder Alkenylengruppe, in denen jeweils der Alkylenteil 1 bis 5 Kohlenstoffatome und der Alkenylenteil 2 bis 5 Kohlenstoffatome enthalten kann,

eine Phenylengruppe, die durch Fluor-, Chlor- oder Bromatome, durch Alkylgruppen mit 1 bis 4 Kohlenstoffatomen, durch Trifluormethyl-, Hydroxy-, Alkoxy-, Alkylsulfenyl-, Alkylsulfinyl-, Alkylsulfonyl-, Nitro-, $(R_1)_2N$-, $(R_1)_2NCO$-, $(R_1)_2NSO_2$- oder $R_1$OCO-alkoxy-Gruppen oder durch durch eine Alkylcarbonyl-, Aralkylcarbonyl-, Arylcarbonyl-, Heteroarylcarbonyl-, Alkylsulfonyl-, Aralkylsulfonyl- oder Arylsulfonylgruppe substituierte $R_1$NH-Gruppen mono- oder disubstituiert sein kann, wobei die Substituenten gleich oder verschieden sein können,

eine Pyridinylen-, Pyrimidinylen-, Pyrazinylen- oder Pyridazinylengruppe, die jeweils im Kohlenstoffgerüst durch eine Alkylgruppe substituiert sein können, wobei zusätzlich eine oder zwei -CH = N-Gruppen jeweils durch eine -CO-$NR_1$-Gruppe ersetzt sein können und dann eines der Stickstoffatome statt an den Rest $R_1$ auch an den Rest E, sofern dieser keine Bindung darstellt und nicht über ein Heteroatom an den Rest D gebunden ist, oder an das im Ring befindliche Atom des jeweiligen Restes $X_1$ bis $X_5$, sofern dieses ein Kohlenstoffatom darstellt, gebunden sein kann,

eine Cyclohexylengruppe, in der eine oder zwei CH-Einheiten je durch ein Stickstoffatom ersetzt sein können, wobei zusätzlich jeweils eine oder zwei zu einem Stickstoffatom benachbarte Methylengruppen durch eine Carbonylgruppe ersetzt sein können, oder

eine über den Rest $W_1$ mit dem im Ring befindlichen Atom des jeweiligen Restes $X_1$ bis $X_5$, sofern dieser ein Kohlenstoffatom darstellt, verknüpfte Alkylengruppe mit 1 bis 3 Kohlenstoffatomen, in der $W_1$ eine $NR_1$-Gruppe, ein Sauerstoff- oder Schwefelatom darstellt,

E eine Bindung,

eine geradkettige oder verzweigte Alkylengruppe mit 1 bis 5 Kohlenstoffatomen oder eine Alkenylengruppe mit 2 bis 5 Kohlenstoffatomen, die jeweils durch eine oder zwei $R_1$OCO-alkylgruppen substituiert sein können, oder

eine über den Rest $W_2$ mit dem Rest D verknüpfte Alkylengruppe, in der $W_2$ ein Sauerstoff- oder Schwefelatom, eine Sulfinyl-, Sulfonyl-, -$NR_1$-, (Alkylcarbonyl)N-, -(Aralkylcarbonyl)N-, -(Arylcarbonyl)N-, -(Heteroarylcarbonyl)N-, -(Alkylsulfonyl)N-, -(Arylsulfonyl)N-, -CO$NR_1$- oder -$NR_1$CO-Gruppe darstellt und nicht an ein Heteroatom des Restes D gebunden ist,

F eine nicht an ein Heteroatom der Reste D oder E gebundene Carbonylgruppe, die durch eine Hydroxygruppe, durch eine Aminogruppe, durch eine Alkoxygruppe mit 1 bis 5 Kohlenstoffatomen substituiert ist, in der ein Alkoxyrest mit 1 bis 3 Kohlenstoffatomen in 1-, 2- oder 3-Stellung durch eine Cycloalkylgruppe mit 5 bis 7 Kohlenstoffatomen, durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, durch eine Cycloalkoxygruppe mit 4 bis 8 Kohlenstoffatomen, durch eine Alkanoyloxymethoxygruppe mit insgesamt 2 bis 7 Kohlenstoffatomen im Alkanoylteil, durch eine Aroyloxymethoxygruppe, durch eine Alkoxycarbonyloxymethoxygruppe mit 1 bis 4 Kohlenstoffatomen im Alkylteil oder durch eine Cycloalkoxycarbonyloxymethoxygruppe mit 5 bis 6 Kohlenstoffatomen im Cycloalkylteil, in denen der Methoxyteil jeweils durch eine Alkylgruppe substituiert sein kann, substituiert ist, eine Sulfo-, Phosphono-, O-Alkylphosphono- oder Tetrazol-5-ylgruppe darstellen, wobei, falls A eine Aminogruppe oder eine Cyanogruppe darstellt, der kürzeste Abstand zwischen dieser

Gruppe und dem Rest F mindestens 10 Bindungen beträgt und generell A keine Cyanogruppe darstellen kann, wenn das heterocyclische Ringsystem ein Pyrazolinring und gleichzeitig die Reste C und D unsubstituierte Phenylengruppen und gleichzeitig die Reste B und E eine Bindung darstellen oder, wenn das heterocyclische Ringsystem ein Oxazol- oder Oxazolinring und gleichzeitig der Rest C eine unsubstituierte Phenylengruppe und B eine Bindung darstellen,

ein dritter der Reste $X_1$ bis $X_5$ eine $R_1N<$,

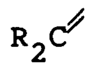

oder $(R_2)_2C<$ Gruppe oder ein N-Atom, wobei $R_1$ jeweils wie eingangs definiert ist und

$R_2$ ein Wasserstoff-, Chlor- oder Bromatom, eine Alkylgruppe mit 1 bis 7 Kohlenstoffatomen, eine Arylalkyl-, Aryl-, Heteroaryl-, Alkoxy-, $(R_1)_2N$-, $R_1OOC$- oder $(R_1)_2NCO$-Gruppe bedeutet,

ein vierter der Reste $X_1$ bis $X_5$ ein Sauerstoff-, Schwefel- oder Stickstoffatom oder eine

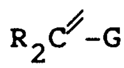-Gruppe

oder auch eine Carbonylgruppe, wenn diese nicht zwischen zwei Stickstoffatomen steht,

ein fünfter der Reste $X_1$ bis $X_5$ ein Stickstoffatom, eine

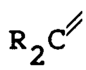

oder $(R_2>_2C<$ Gruppe oder auch zwei benachbarte Reste der Reste $X_1$ bis $X_5$ zusammen eine o-Phenylengruppe bedeuten, darstellen,

wobei, soweit nichts anderes erwähnt wurde,

die vorstehend erwähnten Alkyl-, Alkylen-, Alkenylen- oder Alkoxyteile jeweils 1 bis 3 Kohlenstoffatome enthalten können, sowie

unter den vorstehend erwähnten Begriffen "eine Arylgruppe" oder "eine Aroylgruppe" eine Phenyl-, Naphthyl- oder Benzoylgruppe, die durch eine Trifluormethyl-, Carboxy-, $(R_1)_2NCO$-,Alkoxycarbonyl-, Alkylcarbonyl-, Alkylsulfenyl-, Alkylsulfinyl-, Alkylsulfonyl-, Nitro-, $(R_1)_2N$-, Alkylcarbonyl-$NR_1$-, Aralkylcarbonyl-$NR_1$-, Arylcarbonyl-$NR_1$-, Heteroarylcarbonyl-$NR_1$-, Alkylsulfonyl-$NR_1$-, Aralkylsulfonyl-$NR_1$-, Arylsulfonyl-$NR_1$- oder $(R_1)_2N$-sulfonyl-Gruppe monosubstituiert oder durch Fluor-, Chlor- oder Bromatome, durch Hydroxy-, Alkoxy- oder Alkylgruppen mit 1 bis 4 Kohlenstoffatomen mono- oder disubstituiert sein können, und

unter dem vorstehend erwähnten Begriff "eine Heteroarylgruppe" ein 5-gliedriger heteroaromatischer Ring, welcher ein Sauerstoff-, Schwefel- oder Stickstoffatom, ein Stickstoffatom und ein Sauerstoff-, Schwefel- oder Stickstoffatom oder zwei Stickstoffatome und ein Sauerstoff-, Schwefel- oder Stickstoffatom oder ein 6-gliedriger heteroaromatischer Ring, welcher ein, zwei oder drei Stickstoffatome enthält und in dem zusätzlich eine oder zwei -CH=N-Gruppen durch eine -CO-$NR_1$-Gruppe ersetzt sein können, wobei die vorstehend erwähnten heteroaromatischen Ringe zusätzlich durch eine oder zwei Alkylgruppen oder durch ein Fluor-, Chlor- oder Bromatom, durch eine Hydroxy- oder Alkoxygruppe substituiert sein können, zu verstehen ist,

deren Tautomere, deren Stereoisomere einschließlich deren Gemische und deren Salze.

**4.** 5-gliedrige Heterocyclen der allgemeinen Formel I gemäß Anspruch 1 oder 2, in denen mit der

Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält,

einer der Reste $X_1$ bis $X_5$ eine Gruppe der Formeln

A - B - C - N< ,
A - B - C - CH< oder

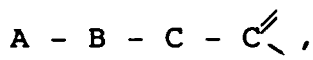

in denen

A eine nicht direkt an einen Phenylring der Gruppen B oder C gebundene Aminogruppe, eine geradkettige oder verzweigte Aminoalkylgruppe mit 1 bis 3 Kohlenstoffatomen, eine Amidino- oder Guanidinogruppe, wobei in den vorstehend erwähnten Amino-, Aminoalkyl-, Amidino- oder Guanidinogruppen jeweils an einem der Stickstoffatome ein oder zwei Wasserstoffatome durch eine oder zwei Alkylgruppen mit jeweils 1 bis 4 Kohlenstoffatomen oder ein Wasserstoffatom durch eine Alkoxycarbonylgruppe mit insgesamt 2 bis 5 Kohlenstoffatomen, durch eine Allyloxycarbonylgruppe, durch eine Benzyloxycarbonylgruppe oder durch eine Alkanoyloxymethoxycarbonylgruppe, in der der Alkanoylteil insgesamt 2 bis 4 Kohlenstoffatome enthalten und der Methoxyteil durch eine Methylgruppe substituiert sein kann, oder durch eine Phosphono-, Dimethylphosphoryl- oder Diethylphosphorylgruppe ersetzt sein kann, oder, falls B oder B und C zusammen ein cyclisches Imin mit 6 Ringgliedern darstellt, auch ein an den Iminstickstoff gebundenes Wasserstoffatom oder eine an den Iminstickstoff gebundene Methylgruppe,

B eine Bindung,

eine Phenylengruppe, die durch ein Fluor-, Chlor- oder Bromatom, durch eine Methyl- oder Methoxygruppe substituiert sein kann,

eine Pyridinylen-, Pyrimidinylen-, Pyrazinylen- oder Pyridazinylengruppe,

eine Cycloalkylengruppe mit 3 bis 5 Kohlenstoffatomen,

eine Piperidinylen- oder 2-Oxo-piperidinylengruppe und

C eine Phenylengruppe, die durch ein Fluor-, Chlor- oder Bromatom, durch eine Methyl-, Methoxy-, Methylsulfenyl-, Methylsulfinyl- oder Methylsulfonylgruppe substituiert sein kann,

eine Indanylen- oder 1,2,3,4-Tetrahydronaphthylengruppe, in denen jeweils der gesättigte Ring an den Rest A und der aromatische Ring an das im Ring befindliche Atom des jeweiligen Restes $X_1$ bis $X_5$ gebunden ist,

eine gegebenenfalls durch eine Methylgruppe substituierte Pyridinylen-, Pyrimidinylen-, Pyrazinylen- oder Pyridazinylengruppe oder

eine Cyclohexylengruppe, in der eine oder zwei CH-Einheiten je durch ein Stickstoffatom ersetzt sein können, darstellen,

ein zweiter der Reste $X_1$ bis $X_5$ eine Gruppe der Formeln

F - E - D - N< ,
F - E - D - CH< oder

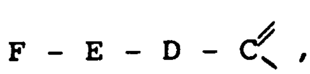

in denen

D eine gegebenenfalls durch eine Hydroxy-, Methoxy-, Amino-, Dimethylamino-, Dibenzylamino- oder Carboxygruppe, durch eine Alkoxycarbonylgruppe mit insgesamt 2 bis 4 Kohlenstoffatomen oder durch eine Alkoxycarbonylaminogruppe mit insgesamt 2 bis 5 Kohlenstoffatomen substituierte Alkylen- oder Alkenylengruppe, wobei die Alkylengruppe 1 bis 3 Kohlenstoffatomen und die Alkenylengruppe 2 oder 3 Kohlenstoffatome enthalten kann,

eine Phenylengruppe, die durch ein Fluor-, Chlor- oder Bromatom, durch eine Methyl-, Hydroxy-, Methoxy-, Methylsulfenyl-, Methylsulfinyl-, Methylsulfonyl-, Nitro-, Amino-, Acetamino-, Benzoylamino-, Methansulfonylamino-, Carboxymethoxy- oder Methoxycarbonylmethoxygruppe substituiert sein kann,

eine Pyridinylen-, Pyrimidinylen-, Pyrazinylen- oder Pyridazinylengruppe, in denen eine -CH=N-Gruppe durch eine -CO-NH-Gruppe ersetzt sein kann, wobei der Stickstoff statt an das Wasserstoffatom auch an den Rest E, sofern dieser keine Bindung darstellt und nicht über ein Heteroatom an den Rest D gebunden ist, oder an das im Ring befindliche Atom des jeweiligen Restes $X_1$ bis $X_5$, sofern dieser ein Kohlenstoffatom darstellt, gebunden sein kann,

eine Cyclohexylengruppe, in der eine CH-Einheit durch ein Stickstoffatom ersetzt sein kann, oder

eine über den Rest $W_1$ mit dem im Ring befindlichen Atom des jeweiligen Restes $X_1$ bis $X_5$, sofern dieses ein Kohlenstoffatom darstellt, verknüpfte Alkylengruppe mit 1 oder 2 Kohlenstoffatomen, in der $W_1$ eine Iminogruppe- oder ein Schwefelatom darstellt,

E eine Bindung,

eine geradkettige oder verzweigte Alkylengruppe mit 1 bis 4 Kohlenstoffatomen oder eine Alkenylengruppe mit 2 bis 4 Kohlenstoffatomen, die jeweils durch eine Carboxymethyl- oder Methoxycarbonylmethylgruppe substituiert sein können, oder

eine über den Rest $W_2$ mit dem Rest D verknüpfte Alkylengruppe mit 1 oder 2 Kohlenstoffatomen, in der $W_2$ ein Sauerstoff- oder Schwefelatom, eine Sulfinyl-, Sulfonyl-, Imino-, Methylimino- oder Acetyliminogruppe oder eine über die Carbonylgruppe an den Rest D gebundene Aminocarbonylgruppe darstellt, wobei E nicht an ein Heteroatom des Restes D gebunden sein kann, und

F eine nicht an ein Heteroatom des Restes D gebundene Carbonylgruppe, die durch eine Hydroxygruppe, durch eine Alkoxygruppe mit 1 bis 4 Kohlenstoffatomen, die in 1-, 2- oder 3-Stellung durch eine Cyclohexyl- oder Phenylgruppe substituiert sein kann, durch eine Cycloalkoxygruppe mit 5 bis 8 Kohlenstoffatomen, durch eine Alkanoylozymethoxygruppe mit insgesamt 2 bis 6 Kohlenstoffatomen im Alkanoylteil, durch eine Benzoyloxymethoxygruppe, durch eine Alkoxycarbonyloxymethoxygruppe mit 1 bis 3 Kohlenstoffatomen im Alkoxyteil oder durch eine Cyclohexyloxycarbonyloxymethoxygruppe, in denen der Methoxyteil jeweils durch eine Methylgruppe substituiert sein kann, substituiert ist, eine Sulfo-, Phosphono-, O-Methyl-phosphono-, O-Ethyl-phosphono- oder Tetrazol-5-yl-gruppe darstellen, wobei, falls A eine Aminogruppe darstellt, der kürzeste Abstand zwischen dieser Gruppe und dem Rest F mindestens 10 Bindungen beträgt,

ein dritter der Reste $X_1$ bis $X_5$ ein N-Atom, eine Imino-, Methylimino-, Ethylimino-, Phenylimino-, Benzylimino- oder 2-Phenylethylimino-,

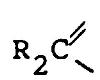

oder $(R_2)_2C<$ Gruppe, wobei $R_2$ ein Wasserstoffatom, eine Alkylgruppe mit 1 bis 7 Kohlenstoffatomen, eine Benzyl-, Phenylethyl-, Phenyl-, Pyridyl-, Carboxy-, Aminocarbonyl- oder Alkoxycarbonylgruppe mit insgesamt 2 bis 4 Kohlenstoffatomen bedeutet,

ein vierter der Reste $X_1$ bis $X_5$ ein Sauerstoff-, Schwefel- oder Stickstoffatom, eine

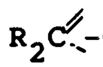-Gruppe

oder auch eine Carbonylgruppe, wenn diese nicht zwischen zwei Stickstoffatomen steht,

ein fünfter der Reste $X_1$ bis $X_5$ ein Stickstoffatom, eine

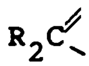

oder $(R_2>_2C<$ Gruppe, wobei $R_2$ wie vorstehend erwähnt definiert ist, oder auch 2 benachbarte Reste der Reste $X_1$ bis $X_5$ zusammen eine o-Phenylengruppe darstellen, bedeuten, darstellen,

deren Tautomere, deren Stereoisomere einschließlich deren Gemische und deren Salze.

**5.** 5-gliedrige Heterocyclen der allgemeinen Formel I gemäß Anspruch 1 oder 2, in denen mit der Maßgabe, daß der 5-gliedrige heterocyclische Ring keinen Pyrrolidin-, Pyrrolin-, Pyrrolinon- oder Pyrrolidinonring darstellt sowie mindestens ein Kohlenstoffatom enthält,

einer der Reste $X_1$ bis $X_5$ eine Gruppe der Formeln

A - B - C - N< oder

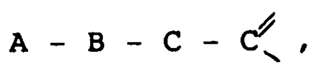

in denen

A eine gegebenenfalls an einem der Stickstoffatome durch eine Alkoxycarbonylgruppe mit insgesamt 2 oder 3 Kohlenstoffatomen substituierte Amidinogruppe,

B eine Bindung oder eine Phenylengruppe und

C eine Phenylen- oder Pyridazinylengruppe,

ein zweiter der Reste $X_1$ bis $X_5$ eine Gruppe der Formeln

F - E - D - N< oder

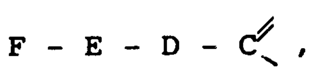

in denen

D eine gegebenenfalls durch eine Hydroxy-, Amino-, Dibenzylamino- oder tert.Butoxycarbonylaminogruppe substituierte Ethylengruppe, eine Phenylengruppe oder eine Methylenthiogruppe, in der das Schwefelatom an ein Kohlenstoffatom des Ringes gebunden ist,

E eine Bindung oder eine Ethylengruppe und

F eine Carbonylgruppe, die durch eine Hydroxy- oder Alkoxygruppe mit 1 oder 2 Kohlenstoffatomen substituiert ist, darstellen,

ein dritter der Reste $X_1$ bis $X_5$ ein N-Atom, eine Imino-, Methylimino- oder Methingruppe,

ein vierter der Reste $X_1$ bis $X_5$ ein Sauerstoff-, Schwefel- oder Stickstoffatom,

ein fünfter der Reste $X_1$ bis $X_5$ ein Stickstoffatom, eine Methin-, Carboxymethin-, Methoxycarbonylmethin- oder Ethoxycarbonylmethingruppe oder auch zwei benachbarte Reste der Reste $X_1$ bis $X_5$ zusammen eine o-Phenylengruppe darstellen, bedeuten,

deren Tautomere, deren Stereoisomere einschließlich deren Gemische und deren Salze.

**6.** Folgende Verbindungen der allgemeinen Formel I gemäß Anspruch 1 oder 2:
(a) 1-[6-(4-Amidino-phenyl)-3-pyridazinyl]-4-(2-carboxyethyl)-imidazol,
(b) 1-[6-(4-Amidino-phenyl)-3-pyridazinyl]-4-(2-carboxy-2-hydroxy-ethyl)-imidazol,
(c) 1-[6-(4-Amidino-phenyl)-3-pyridazinyl]-4-(2-amino-2-carboxy-ethyl)-imidazol,
(d) 5-(4-Amidino-phenyl)-2-[4-(2-carboxy-ethyl)-phenyl]-tetrazol,
(e) 5-(4-Amidino-4'-biphenylyl)-2-(2-carboxy-ethyl)-tetrazol,
(f) 4-(4-Amidino-phenyl)-2-[4-(2-carboxy-ethyl)-phenyl]-thiazol,
(g) 4-(4-Amidino-phenyl)-2-[4-(2-carboxy-ethyl)-phenyl]-1-methyl-imidazol,
(h) 4-(4-Amidino-phenyl)-2-[4-(2-carboxy-ethyl)-phenyl]-imidazol,
(i) 3-(4-Amidino-phenyl)-5-[4-(2-carboxy-ethyl)-phenyl]-1,2,4-triazol,
(j) 2-(4-Amidino-4'-biphenylyl)-5-(2-carboxy-ethyl)-1,3,4-thiadiazol,
(k) 1-[6-(4-Amidino-phenyl)-3-pyridazinyl]-4-(2-carboxy-2-dibenzylamino-ethyl)-imidazol,
(l) 1-[6-(4-Amidino-phenyl)-3-pyridazinyl]-3-(2-carboxy-ethyl)-indol,
(m) 3-(4-Amidino-4'-biphenylyl)-5-carboxymethylthio-1,2,4-triazol,
(n) 4-(2-Amino-2-carboxy-ethyl)-1-[6-(4-aminomethyl-phenyl)-3-pyridazinyl]-imidazol,
(o) 1-[6-(4-Amidino-phenyl)-3-pyridazinyl]-4-(2-methoxycarbonyl-ethyl)-imidazol,
(p) 1-[6-(4-Amidino-phenyl)-3-pyridazinyl]-4-(2-hydroxy-2-methoxycarbonyl-ethyl)-imidazol,
(q) 1-[6-(4-Amidino-phenyl)-3-pyridazinyl]-4-(2-amino-2-methoxycarbonyl-ethyl)-imidazol,
(r) 5-(4-Amidino-phenyl)-2-[4-(2-methoxycarbonyl-ethyl)-phenyl]-tetrazol,
(s) 5-(4-Amidino-4'-biphenylyl)-2-(2-methoxycarbonyl-ethyl)-tetrazol,
(t) 4-(4-Amidino-phenyl)-2-[4-(2-methoxycarbonyl-ethyl)-phenyl]-1-methyl-imidazol,
(u) 4-(4-Amidino-phenyl)-2-[4-(2-methoxycarbonyl-ethyl)-phenyl]-imidazol,
(v) 2-(4-Amidino-phenyl)-5-[4-(2-methoxycarbonyl-ethyl)-phenyl]-1,3,4-thiadiazol,
(w) 2-(4-Amidino-4'-biphenylyl)-5-(2-methoxycarbonyl-ethyl)-1,3,4-thiadiazol,
(x) 3-(4-Amidino-4'-biphenylyl)-5-methoxycarbonylmethylthio-1,2,4-triazol,
(y) 4-(4-Methoxycarbonylamidino-phenyl)-2-[4-(2-methoxycarbonyl-ethyl)-phenyl]-1-methyl-imidazol und
(z) 4-(4-Methoxycarbonylamidino-phenyl)-2-[4-(2-methoxycarbonyl-ethyl)-phenyl]-imidazol,
deren Tautomere und deren Salze.

**7.** Physiologisch verträgliche Additionssalze der Verbindungen nach mindestens einem der Ansprüche 1 bis 6 mit anorganischen oder organischen Säuren oder Basen.

**8.** Arzneimittel, enthaltend eine Verbindung nach mindestens einem der Ansprüche 1 bis 6 oder ein physiologisch verträgliches Additionssalz gemäß Anspruch 7 neben gegebenenfalls einem oder mehreren inerten Trägerstoffen und/oder Verdünnungsmitteln.

**9.** Verwendung einer Verbindung nach mindestens einem der Ansprüche 1 bis 7 zur Herstellung eines Arzneimittels, das zur Bekämpfung bzw. Verhütung von Krankheiten, bei denen kleinere oder größere Zell-Aggregate auftreten oder Zell-Matrixinteraktionen eine Rolle spielen, geeignet ist.

**10.** Verfahren zur Herstellung eines Arzneimittels gemäß Anspruch 8, dadurch gekennzeichnet, daß auf nichtchemischem Wege eine Verbindung nach mindestens einem der Ansprüche 1 bis 7 in einen oder mehrere inerte Trägerstoffe und/oder Verdünnungsmittel eingearbeitet wird.

**11.** Verfahren zur Herstellung der 5-gliedrigen Heterocyclen gemäß den Ansprüchen 1 bis 7, dadurch gekennzeichnet, daß
a) zur Herstellung von Verbindungen der allgemeinen Formel I, in der F eine Carboxylgruppe darstellt, eine Verbindung der allgemeinen Formel

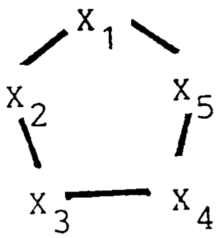

,(II)

in der
$X_1$ bis $X_5$ mit der Maßgabe wie in den Ansprüchen 1 bis 6 definiert sind, daß einer der Reste $X_1$ bis $X_5$ eine Gruppe der Formel

F' - E - D - N< ,
F' - E - D - CH< oder

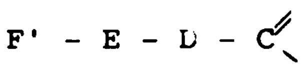 darstellt,

in denen

E und D wie in den Ansprüchen 1 bis 6 definiert sind und F' eine mittels Hydrolyse, Behandeln mit Säuren, Thermolyse oder Hydrogenolyse in eine Carboxylgruppe überführbare Gruppe bedeutet, in eine Verbindung der allgemeinen Formel I, in der F eine Carboxylgruppe darstellt, umgewandelt wird oder
b) zur Herstellung von Verbindungen der allgemeinen Formel I, in der A eine gegebenenfalls durch eine Alkylgruppe substituierte $H_2N$-C(=NH)-Gruppe darstellt, eine gegebenenfalls im Reaktionsgemisch gebildete Verbindung der allgemeinen Formel

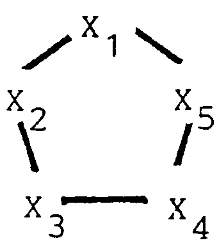

,(III)

in der
$X_1$ bis $X_5$ mit der Maßgabe wie in den Ansprüchen 1 bis 6 definiert sind, daß einer der Reste $X_1$ bis $X_5$ eine Gruppe der Formel

$Z_1$ - C(=NH) - B - C - N< ,
$Z_1$ - C(=NH) - B - C - CH< oder

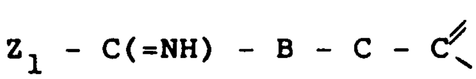 darstellt,

in denen

B und C wie in den Ansprüchen 1 bis 6 definiert sind und $Z_1$ eine Alkoxy-, Aralkoxy-, Alkylthio-, Aralkylthio- oder Aminogruppe darstellt, mit einem Amin der allgemeinen Formel

$(R_3)_2NH$ ,(IV)

in der
die Reste $R_3$, die gleich oder verschieden sein können, Wasserstoffatome oder Alkylgruppen mit jeweils 1 bis 3 Kohlenstoffatomen darstellen, oder mit deren Säureadditionssalzen umgesetzt wird oder
c) zur Herstellung von Verbindungen der allgemeinen Formel I, in der mindestens einer der Reste B, C, D oder E eine Sulfinyl- oder Sulfonylgruppe enthält, eine Verbindung der allgemeinen Formel

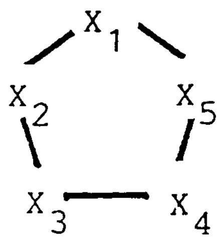

,(V)

in der
$X_1$ bis $X_5$ mit der Maßgabe wie in den Ansprüchen 1 bis 6 definiert sind, daß mindestens einer der Reste $X_1$ bis $X_5$ eine Sulfenyl- oder Sulfinylgruppe enthält, oxidiert wird oder
d) zur Herstellung von Verbindungen der allgemeinen Formel I, in der A eine durch eine Alkoxycarbonylgruppe mit insgesamt 2 bis 5 Kohlenstoffatomen oder durch eine Aralkoxycarbonylgruppe substituierte Amino-, Aminoalkyl-, Amidino- oder Guanidinogruppe darstellt, eine Verbindung der allgemeinen Formel

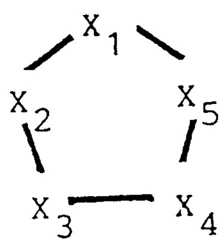

,(VI)

in der
$X_1$ bis $X_5$ mit der Maßgabe wie in den Ansprüchen 1 bis 6 definiert sind, daß einer der Reste $X_1$ bis $X_5$ eine Gruppe der Formel

A' - B - C - N< ,
A' - B - C - CH< oder

$A' - B - C - C\!\!/\!\!/$ darstellt,

in denen

B und C wie in den Ansprüchen 1 bis 6 definiert sind und A' eine Amino-, Aminoalkyl-, $H_2N-C(=NH)-$ oder $H_2N-C(=NH)-NH-$Gruppe darstellt, mit einer Verbindung der allgemeinen Formel

$Z_2 - COOR_4$ ,(VII)

in der
$R_4$ eine Alkylgruppe mit 1 bis 4 Kohlenstoffatomen oder eine Aralkylgruppe und
$Z_2$ eine nukleophile Austrittsgruppe darstellen, umgesetzt wird oder
e) zur Herstellung von Verbindungen der allgemeinen Formel I, in der F eine durch eine Alkoxygruppe mit 1 bis 6 Kohlenstoffatomen substituierte Carbonylgruppe darstellt, wobei ein Alkoxyrest mit 1 bis 3 Kohlenstoffatomen in 1-, 2- oder 3-Stellung durch eine Aryl- oder Heteroarylgruppe oder in 2-

oder 3-Stellung durch eine Pyrrolidin-2-on-1-yl-, Morpholino-, Thiomorpholino- oder 1-Oxido-thiomorpholinogruppe substituiert sein kann, eine Verbindung der allgemeinen Formel

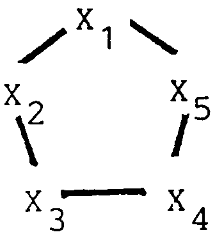 ,(VIII)

in der
$X_1$ bis $X_5$ mit der Maßgabe wie in den Ansprüchen 1 bis 6 definiert sind, daß einer der Reste $X_1$ bis $X_5$ eine Gruppe der Formel

F'' - E - D - N< ,
F'' - E - D - CH< oder

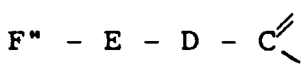 darstellt,

in denen

E und D wie in den Ansprüchen 1 bis 6 definiert sind und F'' eine Carboxy- oder Alkoxycarbonylgruppe darstellt, mit einem Alkohol der allgemeinen Formel

HO - $R_5$ ,(IX)

in der
$R_5$ eine Alkylgruppe mit 1 bis 6 Kohlenstoffatomen, die in 1-, 2- oder 3-Stellung durch eine Aryl- oder Heteroarylgruppe oder in 2- oder 3-Stellung durch eine Pyrrolidin-2-on-1-yl-, Morpholino-, Thiomorpholino- oder 1-Oxido-thiomorpholinogruppe substituiert sein kann, darstellt, umgesetzt wird oder
f) zur Herstellung von Verbindungen der allgemeinen Formel I, in der A eine Aminoalkylgruppe darstellt, eine Verbindung der allgemeinen Formel

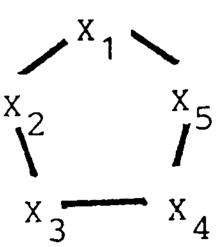 ,(X)

in der
$X_1$ bis $X_5$ mit der Maßgabe wie in den Ansprüchen 1 bis 6 definiert sind, daß einer der Reste $X_1$ bis $X_5$ eine Gruppe der Formel

A'' - B - C - N< ,
A'' - B - C - CH< oder

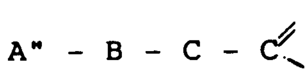 darstellt,

in denen

B und C wie in den Ansprüchen 1 bis 6 definiert sind und A" eine Cyanogruppe oder eine Cyanoalkylgruppe darstellt, reduziert wird oder

g) zur Herstellung von Verbindungen der allgemeinen Formel I, in der einer der Reste $X_1$ bis $X_5$ eine A - B - C - N< oder F - E - D - N< Gruppe darstellt, eine Verbindung der allgemeinen Formel

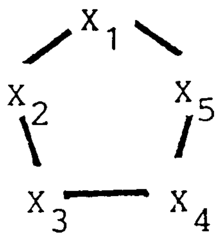

,(XI)

in der

$X_1$ bis $X_5$ mit der Maßgabe wie in den Ansprüchen 1 bis 6 definiert sind, daß einer der Reste $X_1$ bis $X_5$ eine Iminogruppe darstellt, mit einer Verbindung der allgemeinen Formel

$Z_3 - R_6$ ,(XII)

in der

$R_6$ eine Gruppe der Formel

A - B - C - oder

F - E - D - darstellt, in denen

A bis F wie in den Ansprüchen 1 bis 6 definiert sind und $Z_3$ eine nukleophile Austrittsgruppe darstellt, umgesetzt wird oder

h) zur Herstellung von Verbindungen der allgemeinen Formel I, in der einer der Reste $X_1$ bis $X_5$ eine A - B - C - N< oder F - E - D - N< Gruppe, der andere der Reste eine

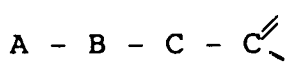 oder 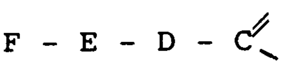

Gruppe und die verbleibenden Reste $X_1$ bis $X_5$ Stickstoffatome darstellen, eine gegebenenfalls im Reaktionsgemisch hergestellte Verbindung der allgemeinen Formel

$$R' - N_2^{(+)} X$$ ,(XIII)

mit einer Verbindung der allgemeinen Formel

$R'' - CH = N - NH - Z_4$ ,(XIV)

in denen

der Rest R' eine A-B-C- oder F-E-D-Gruppe darstellt, wobei jeweils der Rest C oder der Rest D eine Aryl- oder Heteroarylgruppe, die über ein Kohlenstoffatom an den 5-gliedrigen Ring gebunden ist, darstellt und

der Rest R" der jeweils anderen der Gruppen A-B-C- und F-E-D entspricht, und

X das Anion einer anorganischen Säure darstellen, umgesetzt wird oder

i) zur Herstellung von Thiazolen der allgemeinen Formel I, eine Verbindung der allgemeinen Formel

$R' - CO - CH_2 - Z_5$ ,(XV)

mit einer Verbindung der allgemeinen Formel

R'' - CS - $NH_2$ ,(XVI)

in denen
einer der Reste R' oder R'' eine A - B - C - Gruppe und der andere der Reste R' oder R'' eine F - E - D - Gruppe und $Z_5$ eine nukleophile Austrittsgruppe darstellen, umgesetzt wird oder
j) zur Herstellung von Diazolderivaten der allgemeinen Formel I, eine gegebenenfalls im Reaktionsgemisch gebildete Verbindung der allgemeinen Formel

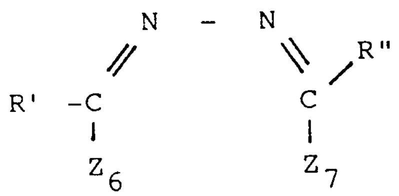

,(XVII)

in der
$Z_6$ und $Z_7$, die gleich oder verschieden sein können, Hydroxy-, Alkoxy-, Mercapto-, Alkylmercapto- oder Aminogruppen, einer der Reste R' oder R'' eine A - B - C - Gruppe und der andere der Reste R' oder R'' eine F - E - D - Gruppe darstellen, cyclisiert wird oder
k) zur Herstellung von Verbindungen der allgemeinen Formel I, in der A und B oder A, B und C zusammen eine N-Amidino cyclische Iminogruppe mit 4 bis 7 Ringgliedern darstellen, eine Verbindung der allgemeinen Formel

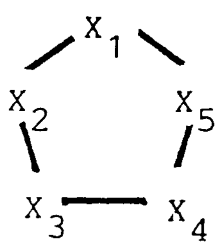

,(XVIII)

in der
$X_1$ bis $X_5$ mit der Maßgabe wie in den Ansprüchen 1 bis 6 definiert sind, daß B oder B und C zusammen eine cyclische Iminogruppe mit 4 bis 7 Ringgliedern darstellen, mit einem S-Alkyl-isothioharnstoff umgesetzt wird oder
l) zur Herstellung von Verbindungen der allgemeinen Formel I, in der $X_1$ bis $X_5$ mit der Maßgabe wie in den Ansprüchen 1 bis 6 definiert sind, daß einer der Reste $X_1$ bis $X_5$ eine Gruppe der Formel

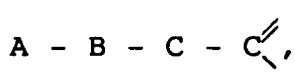

ein zweiter der Reste $X_1$ bis $X_5$ eine Gruppe der Formel

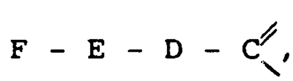

ein dritter der Reste $X_1$ bis $X_5$ ein Sauerstoffatom, ein vierter und fünfter der Reste $X_1$ bis $X_5$ eine

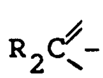

Gruppe darstellen, eine Verbindung der allgemeinen Formel

A - B - C - CO - $CHR_2$- $CHR_2$- CO - D -E - F ,(XIX)

in der
A bis F und $R_2$ wie in den Ansprüchen 1 bis 6 definiert sind, dehydratisiert wird oder
m) zur Herstellung von Verbindungen der allgemeinen Formel I, in der A eine Guanidinogruppe darstellt, eine Verbindung der allgemeinen Formel

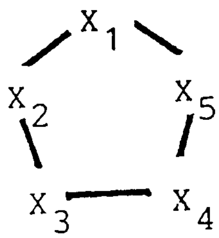
,(XX)

in der
$X_1$ bis $X_5$ mit der Maßgabe wie in den Ansprüchen 1 bis 6 definiert sind, daß A eine Aminogruppe darstellt, mit Cyanamid oder mit dessen Säureadditionssalz umgesetzt wird oder
n) zur Herstellung von Verbindungen der allgemeinen Formel I, in der A eine Amino- oder Aminoalkylgruppe darstellt, eine Verbindung der allgemeinen Formel

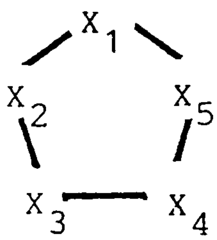
,(XXI)

in der
$X_1$ bis $X_5$ mit der Maßgabe wie in den Ansprüchen 1 bis 6 definiert sind, daß einer der Reste $X_1$ bis $X_5$ eine $H_2N$-CO-T-B-C-Gruppe enthält, wobei B und C wie in den Ansprüchen 1 bis 6 definiert sind und
T eine Bindung oder eine Alkylengruppe mit 1 bis 5 Kohlenstoffatomen darstellt, mit einer Phenyljod(III)verbindung der allgemeinen Formel

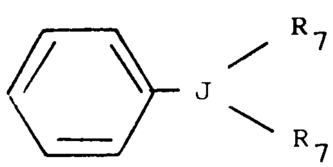
,(XXII)

in der
$R_7$ jeweils den Acylrest einer organischen Carbonsäure wie die Acetoxy- oder Trifluoracetoxygruppe darstellt, umgesetzt wird oder
o) zur Herstellung von Verbindungen der allgemeinen Formel I, in der A eine Aminoalkylgruppe, in der die Aminogruppe nicht an ein quartäres Kohlenstoffatom gebunden ist, oder eine Aminogruppe darstellt, die an eine CH- oder $CH_2$-Gruppe des Restes B oder C gebunden ist, darstellt, eine Verbindung der allgemeinen Formel

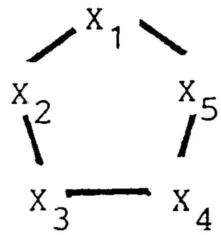

,(XXIII)

in der
$X_1$ bis $X_5$ mit der Maßgabe wie in den Ansprüchen 1 bis 6 definiert sind, daß einer der Reste $X_1$ bis $X_5$ eine Gruppe der Formel

A''' - B - C - enthält, in der

B und C wie in den Ansprüchen 1 bis 6 definiert sind und A''' eine N-Hydroxy-iminogruppe enthält, reduziert wird oder
p) zur Herstellung von Verbindungen der allgemeinen Formel I, in der mindestens einer der Reste $X_1$ bis $X_5$ eine durch eine oder zwei Alkyl- oder Aralkylgruppen substituierte Aminogruppe oder eine durch eine Alkylgruppe substituierte Iminogruppe enthält, eine Verbindung der allgemeinen Formel

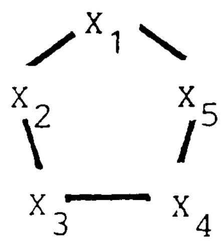

,(XXIV)

in der
$X_1$ bis $X_5$ mit der Maßgabe wie in den Ansprüchen 1 bis 6 definiert sind, daß einer der Reste $X_1$ bis $X_5$ eine Amino-, Alkylamino- oder Iminogruppe enthält, mit einer Verbindung der allgemeinen Formel

$Z_8$ - ($R_8$-C-$R_9$) - $Z_9$ ,(XXV)

in der
$R_8$ und $R_9$, die gleich oder verschieden sein können, Wasserstoffatome, Alkyl-, Aralkyl- oder Arylgruppen, eine der Gruppen $Z_8$ oder $Z_9$ eine nukleophile Austrittsgruppe und
die andere der Gruppen $Z_8$ oder $Z_9$ ein Wasserstoffatom oder eine Alkylgruppe oder
$Z_8$ und $Z_9$ zusammen ein Sauerstoffatom bedeuten, umgesetzt wird oder
q) zur Herstellung von Verbindungen der allgemeinen Formel I, in der A eine durch eine Dialkylphosphorylgruppe mit jeweils 1 bis 3 Kohlenstoffatomen im Alkylteil substituierte Amino-, Aminoalkyl-, Amidino- oder Guanidinogruppe darstellt, eine Verbindung der allgemeinen Formel

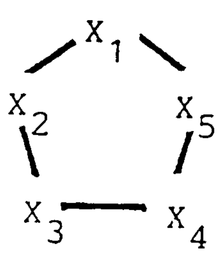

,(XXVI)

in der
$X_1$ bis $X_5$ mit der Maßgabe wie in den Ansprüchen 1 bis 6 definiert sind, daß einer der Reste $X_1$ bis $X_5$ eine Gruppe der Formeln

A' - B - C - N< ,
A' - B - C - CH< oder

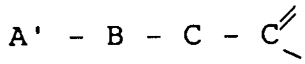 darstellt,

in denen

B und C wie in den Ansprüchen 1 bis 6 definiert sind und A' eine Amino-, Aminoalkyl-, Amidino- oder Guanidinogruppe darstellt, mit einer Verbindung der allgemeinen Formel

$Z_{10}$ - $PO(OR_{10})_2$ ,(XXVII)

in der
$R_{10}$ eine Alkylgruppe mit 1 bis 3 Kohlenstoffatomen und $Z_{10}$ eine nukleophile Austrittsgruppe darstellen, umgesetzt wird und

gewünschtenfalls anschließend eine so erhaltene Verbindung der allgemeinen Formel I, welche eine Carboxygruppe enthält, in ihren Ester übergeführt wird und/oder

erforderlichenfalls ein während den Umsetzungen zum Schutze von reaktiven Gruppen verwendeter Schutzrest abgespalten wird und/oder

gewünschtenfalls eine so erhaltene Verbindung der allgemeinen Formel I in ihre Stereoisomere aufgetrennt wird und/oder

eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.